# EUROPEAN PATENT APPLICATION

(11) **EP 4 159 860 A1**
(43) Date of publication of application: **05.04.2023**
(21) Application number: 21817253.4
(22) Date of filing: 01.06.2021
(51) Int. Cl.: C12N 15/13, A61K 31/7088, A61K 39/395, A61K 48/00, A61P 21/00, A61P 35/00, C07K 16/28, C07K 16/46, C12N 1/15, C12N 1/19, C12N 1/21, C12N 5/10, C12N 15/63, C12P 21/08

(54) **ANTI-IGF-1 RECEPTOR HUMANIZED ANTIBODY**

(30) Priority: 02.06.2020 JP 2020096344
(71) Applicant: Teijin Pharma Limited, Tokyo 100-0013 (JP)
(72) Inventor: MATSUKAWA, Hiroaki, Tokyo 100-0013 (JP); EGUCHI, Hiroshi, Tokyo 100-0013 (JP); NAMIKI, Naoko, Tokyo 100-0013 (JP); TANOKURA, Akira, Tokyo 100-0013 (JP)
(74) Representative: Lavoix
(86) International application number: PCT/JP2021/020890
(87) International publication number: WO 2021/246413

(57) **Abstract**

Provided are: an anti-IGF-1 receptor humanized antibody which includes CDRs of a light chain and a heavy chain derived from mice parent antibody IGF 11-16, and respective FRs of a light chain and a heavy chain derived from a human antibody, and in which at least one of the CDRs includes at least one amino acid residue substitution with respect to a corresponding CDR of the mice parent antibody; a fragment of the anti-IGF-1 receptor humanized antibody; or a derivative thereof.

## Description

### TECHNICAL FIELD

The present invention relates to an anti-IGF-1 receptor humanized antibody and, more specifically, to an anti-IGF-1 receptor humanized antibody which specifically binds to an IGF-1 receptor.

### BACKGROUND ART

### 1. IGF-1

IGF-1 is an insulin-like growth factor secreted mainly from the liver through activation of a growth hormone (GH) receptor by the growth hormone secreted from the pituitary gland, and affects an IGF-1 receptor to thereby express a variety of physiological functions in various organs. Because of this, IGF-1 is expected to be used for the treatment of a variety of diseases. Since the amino acid sequence of IGF-1 has a high similarity of about 40% to that of proinsulin, IGF-1 can bind to an insulin receptor and thereby express insulin-like effects. In addition, since the amino acid sequence of the IGF-1 receptor has a high similarity of about 60% to that of an insulin receptor, these receptors can form a heterodimer and thereby exhibit physiological effects. Insulin can act on the insulin receptor to thereby express a strong effect of lowering the level of blood glucose, and is thus used as a hypoglycemic drug.

### 2. IGF-1 receptor

An IGF-1 receptor is a transmembrane protein consisting of an alpha chain and a beta chain, and has six extracellular domains (L1, CR, L2, Fn1, Fn2, and Fn3), a transmembrane domain, and an intracellular domain. The intracellular domain of the IGF-1 receptor incorporates a tyrosine kinase. The extracellular domain participates in activation of the intracellular tyrosine kinase associated with conformational change of the IGF-1 receptor, which occurs when IGF-1 binds to the IGF-1 receptor. The IGF-1 receptor forms a homodimeric complex (homo-type). IGF-1 binding to the IGF-1 receptor (homo-type) triggers signaling via activation of the receptor kinase. The IGF-1 receptor also forms a heterodimeric complex (hetero-type) with the insulin receptor. Insulin or IGF-1 binding to the IGF-1 receptor (hetero-type) triggers signaling via activation of the receptor kinase.

### 3. Physiological effects of IGF-1

IGF-1 has been shown to exhibit growth promoting effects, such as increasing the body length and the body mass, and insulin-like metabolic effects, such as glucose metabolism acceleration and hypoglycemic effects. It has been revealed that mecasermin, a human recombinant IGF-1, improves symptoms related to insulin receptor abnormality, such as hyperglycemia, hyperinsulinemia, acanthosis nigricans and hirsutism. IGF-1 has also been shown to improve growth disorder of dwarfism resistant to growth hormone (Non-Patent Literature 1).

### 4. Growth promoting effects of IGF-1

IGF-1 is a major growth-promoting factor (Non-Patent Literature 2, Non-Patent Literature 3). In fact, mecasermin, a human recombinant IGF-1, has been used clinically as a drug for treating dwarfism. IGF-1 is also known to enhance the DNA synthesis capacity of human chondrocytes. Administration of IGF-1 also increases the body mass and lengthens the femur bone length in pituitaryectomized rats.

### 5. Effect of IGF-1 on increasing muscle mass

Enhancement of cell proliferation activity with IGF-1 requires continuous activation of the IGF-1 receptor. An animal engineered to overexpress the IGF-1 receptor exhibits increased muscle mass. Sustained administration of IGF-1/IGFBP3 to a patient with proximal femur fracture enhances her/his grip strength and improves her/his ability of standing from a seated position without assistance. The muscle IGF-1 levels of the elderly humans and mice are known to be lower than those of the young. Over expression of IGF-1 specifically in muscle tissues of elderly mice improved their muscle masses compared to wild-type mice (Non-Patent Literature 4).

### 6. Precedent products for increasing muscle mass

Anamorelin, a ghrelin receptor agonist, increased lean body mass in a clinical trial for cachexia, which is a disuse muscle atrophy. However, it involves adverse effects such as inducing nausea and hyperglycemia. Myostatin, a negative control factor of skeletal myogenesis, affects activin receptor II (ActRII) to thereby inhibit Akt/mTOR. LY2495655, an anti-myostatin antibody, increases the muscle masses of patients who received total hip replacement arthroplasty and those of elderly subjects. Bimagrumab, an anti-ActRII antibody, increases the muscle mass of neuromuscular disease patients. However, there is no drug so far which promotes formation of skeletal muscles and can thereby be used for the treatment of a subject in need thereof.

### 7. Hypoglycemic effect of IGF-1

IGF-1 is known to have hypoglycemic effect as an insulin-like effect. IGF-1 enhances glucose uptake effect of rat muscle-derived cells. Administration of IGF-1 also reduces the blood glucose level of rats. It has been reported that the glucose lowering effect of IGF-1 cause hypoglycemia as a clinical adverse effect. In addition, administration of IGF-1 to a human subject causes hypoglycemia. Therefore, at the onset of IGF-1 treatment, it is necessary to keep controlling the dosage starting from a low dosage with observing various clinical findings including the blood glucose level after administration.

IGF-1 expresses hypoglycemic effect via, e.g., promotion of Akt phosphorylation. An active variant of Akt enhances glucose uptake by 3T3-L1 cells. On the other hand, an Akt2-deficient mouse exhibited elevated blood glucose level. An Akt inhibitor inhibits insulin-induced glucose uptake by rat muscle-derived cells. In addition, IGF-1 is also known to activate an insulin receptor which plays a role in hypoglycemic effect. These findings suggest that the hypoglycemic effect of IGF-1 involves overactivation of Akt and activation of the insulin receptor.

### 8. Short half-life of IGF-1 in blood

IGF-1 has a short half-life in blood, and therefore requires frequent administrations when used in treatment. In fact, mecasermin, a human recombinant IGF-1, has a blood half-life of about 11 hours to about 16 hours, and therefore needs to be administered once to twice daily in the treatment of dwarfism. About 70 to 80% of IGF-1 is bound to IGFBP3 in blood, while a free form of IGF-1 exhibits physiological effect. Binding of IGF-1 to IGFBP3 maintains its half-life in blood to a time period of from about 10 hours to about 16 hours. IPLEX, a combination drug of IGF-1 with IGFBP3, exhibited a blood half-life extended from that of IGF-1 to a time period of about 21 hours to about 26 hours, and thereby allowed for reduction of administration frequency to once daily. However, IPLEX was already withdrawn from the market. There has been also an attempt to develop a PEGylated IGF-1 with improved IGF-1 kinetics, but no drug has successfully been developed so far and is currently available.

### 9. Therapeutic effects expected to be achieved via IGF-1's effects

IGF-1 is known to affect various organs and exerts a wide variety of physiological functions. IGF-1 has been reported to have neuroprotective effect on the central nervous system by protecting mitochondria and antioxidant effect via activation of the IGF-1 receptor. IGF-1 promotes regeneration of injured neurites. IGF-1 is deemed to be effective in the treatment of hepatic cirrhosis, which evolves from liver damage or chronic liver disease and involves hepatic fibrosis. Administration of IGF-1 improved hepatic fibrosis in a model animal of hepatic cirrhosis. IGF-1 is also known to play a role in the development and functions of kidney. IGF-1 has protective effect against oxidative stress and apoptosis due to glucotoxicity in mesangial cells of kidney. IGF-1 is expected as a drug for the treatment of nephropathy.

Examples of conditions expected to be improved via IGF-1 administration include: sarcopenia, disuse muscle atrophy, cachexia, dwarfism, Laron syndrome, hepatic cirrhosis, hepatic fibrosis, aging, intrauterine growth restriction (ILTGR), neurological disease, cerebral stroke, spinal cord injury, cardiovascular protection, diabetes, insulin resistant, metabolic syndrome, nephropathy, osteoporosis, cystic fibrosis, wound healing, myotonic dystrophy, AIDS-associated sarcopenia, HIV-associated fat redistribution syndrome, burn, Crohn's disease, Werner's syndrome, X-linked combined immunodeficiency disease, hearing loss, anorexia nervosa, and retinopathy of prematurity (Non-Patent Literature 19). Thus, IGF-1 is expected as a drug for the treatment of a variety of diseases because of its wide spectrum of physiological effects. However, problems such as its adverse hypoglycemic effect and its short half-life requiring multiple administrations have prevented its clinical applications.

### 10. Anti-IGF-1 receptor agonist antibody

In general, antibody formulations have long half-life, and prove effective if administered once to twice a month. Some IGF-1 receptor agonist antibodies have been reported to be effective in activating the receptor in vitro. Specifically, antibodies 3B7 and 2D1 enhance cellular DNA synthesis of recombinant IGF-1 receptor expression cells cultured for five hours in vitro (Non-Patent Literature 5). Anti-IGF-1 receptor antagonist antibodies 11A1, 11A4, 11A11, and 24-57, which has an activity to inhibit the proliferation of a cancer cell line, enhance, although very slightly, tyrosine phosphorylation of IGF-1 receptor in vitro (Non-Patent Literature 6). Antibodies 16-13, 17-69, 24-57, 24-60, and 24-31 are shown to be effective in promoting cellular DNA synthesis and glucose uptake in vitro in a short time, and have the potential to exhibit hypoglycemic effect (Non-Patent Literature 7).

However, IGF-1 receptor tyrosine phosphorylation has been observed even with anti-IGF-1 receptor antagonist antibodies which have an inhibitory effect on cancer cell proliferation, such as αIR-3, and is not an indicator of agonist action (Non-Patent Literatures 5, 6, 8). It cannot be an indicator of agonist antibodies with cell proliferation activity also, since in cell proliferation assays using DNA synthesis as an indicator, such as thymidine or BrdU uptake, thymidine uptake has also been observed for anti-IGF-1 receptor antagonist antibodies with cancer cell growth inhibitory activity (Non-Patent Literatures 5 to 8). Furthermore, all of these were short-term evaluations within 24 hours, and there have been no reports of IGF-1 receptor agonist antibodies for promoting cell proliferation in culture for several days (Non-Patent Literatures 5 to 8), let alone antibodies that showed agonist activity against the IGF-1 receptor in vivo. In addition, since IGF-1 exerts both hypoglycemic and cell proliferative effects, it is necessary to avoid hypoglycemic effects in order to administer anti-IGF-1 receptor agonist antibodies to humans as therapeutic agents, although there have been no reports of such anti-IGF-1 receptor agonist antibodies. In addition, antibodies have a large molecular mass and are known to exhibit low tissue distribution, with a brain distribution of about 0.1% and a muscle tissue distribution of about 2%. Therefore, antibodies that show sufficient pharmacological activity at extremely low concentrations (on the order of pM) are required in order to exert their effects in tissues where antibody migration is low. However, there have been no reports of anti-IGF-1 receptor agonist antibodies that can act at such extremely low concentrations.

Against this background, the present inventors have succeeded in producing an anti-IGF-1 receptor monoclonal mouse antibody, IGF 11-16, which exerts myoblast proliferative activity at very low concentrations in vitro and does not induce glucose uptake by differentiated skeletal muscle cells at such concentrations. In addition, the obtained monoclonal antibody IGF11-16 can be used to induce glucose uptake by skeletal muscle cells. Furthermore, they have confirmed that the obtained monoclonal mouse antibody induces muscle mass gain and growth plate elongation in vivo without causing hypoglycemic symptoms (Patent Literature 1).

### 11. Anti-IGF-1 receptor antagonist antibody

There are attempts to use an antibody which binds to the IGF-1 receptor for the treatment of malignancies, based on its antagonist effect of inhibiting binding of IGF-1 to the IGF-1 receptor. However, existing IGF-1 receptor antagonist antibodies have various adverse effects such as hyperglycemia in monotherapy (Non-Patent Literature 9), and exhibit increased incidence of hyperglycemia when used in combination with other anticancer agents (Non-Patent Literature 10). Accordingly, their therapeutic applications are expected to be limited. Recently, teprotumumab was approved for the treatment of ophthalmopathy in hyperthyroidism (Non-Patent Literature 11).

### LIST OF CITATIONS

### PATENT LITERATURE

[Patent Literature 1] WO2018/221521A

### NON-PATENT LITERATURE

[Non-Patent Literature 1] Human somatomedin C "Somazon ®Formulation for Injection 10mg," Drug interview form, revised May 2015, 5th ed.
[Non-Patent Literature 2] Abuzzahab, M.J., et al., IGF-1 receptor mutations resulting in intrauterine and postnatal growth retardation. N Engl J Med, 2003. 349(23): p. 2211-22.
[Non-Patent Literature 3] Woods, K.A., et al., Intrauterine growth retardation and postnatal growth failure associated with deletion of the insulin-like growth factor I gene. N Engl J Med, 1996. 335(18): p. 1363-7.
[Non-Patent Literature 4] Musaro, A., et al., Localized Igf-1 transgene expression sustains hypertrophy and regeneration in senescent skeletal muscle, Nature Genetics, 2001, Vol.27, No.2, pp.195-200
[Non-Patent Literature 5] Xiong, L., et al., Growth-stimulatory monoclonal antibodies against human insulin-like growth factor I receptor. Proc Natl Acad Sci USA, 1992. 89(12): p. 5356-60.
[Non-Patent Literature 6] Runnels, H.A., et al., Human monoclonal antibodies to the insulin-like growth factor 1 receptor inhibit receptor activation and tumor growth in preclinical studies. Adv Ther, 2010. 27(7): p. 458-75.
[Non-Patent Literature 7] Soos, M.A., et al., A panel of monoclonal antibodies for the type I insulin-like growth factor receptor. Epitope mapping, effects on ligand binding, and biological activity. J Biol Chem, 1992. 267(18): p. 12955-63.
[Non-Patent Literature 8] Kato, H., et al., Role of tyrosine kinase activity in signal transduction by the insulin-like growth factor-I (IGF-1) receptor. Characterization of kinase-deficient IGF-1 receptors and the action of an IGF-1-mimetic antibody (alpha IR-3). J Biol Chem, 1993. 268(4): p. 2655-61.
[Non-Patent Literature 9] Atzori, F., et al., A Phase I Pharmacokinetic and Pharmacodynamic Study of Dalotuzumab (MK-0646), an Anti-Insulin-like Growth Factor-1 Receptor Monoclonal Antibody, in Patients with Advanced Solid Tumors. Clin Cancer Res., 2011.17(19):p.6304-12.
[Non-Patent Literature 10] de Bono J.S., et al., Phase II randomized study of figitumumab plus docetaxel and docetaxel alone with crossover for metastaticcastrationresistant prostate cancer. Clin Cancer Res., 2014.20(7):p.1925-34.
[Non-Patent Literature 11] Markham. A, Teprotumumab: First Approval. Drugs, 2020. 80(5): p.509-512.
[Non-Patent Literature 12] Riechman, L., Clark, M., Waldmann, H., Winter, G.: Reshaping human antibodies for therapy. Nature, 1988. 332:p.323-327.
[Non-Patent Literature 13] Kabat et al., The Journal of Immunology, 1991, Vol.147, No.5, pp.1709-1719
[Non-Patent Literature 14] Al-Lazikani et al., Journal of Molecular Biology, 1997, Vol.273, No.4, pp.927-948
[Non-Patent Literature 15] Abhinandan, K.R. et al., Molecular Immunology, 2008, Vol.45, pp.3832-3839
[Non-Patent Literature 16] Jian, Y et al., Nucleic Acids Research, 2013, Vol.41, W34-W40
[Non-Patent Literature 17] Yamada, T. et al., Therapeutic monoclonal antibodies. Keio Journal of Medicine, 2011, Vol.60, No.2, pp37-46
[Non-Patent Literature 18] Burks, E. A., et al., Proc. Natl. Acad. Sci. USA, 1997, Vol.94, No.2, pp.412-417
[Non-Patent Literature 19] Dumet, C., et al., MAbs, 2019, Vol.11, No.8, pp1341-1350
[Non-Patent Literature 20] Saunders, K. O., Frontiers in Immunology, 2019, Vol.10, Article 1296
[Non-Patent Literature 21] Walle et al., Expert Opin. Biol. Ther., 2007, Vol.7, No.3, pp.405-418
[Non-Patent Literature 22] Silva, J-P., et al., The Journal of Biological Chemistry, 2015, Vol.290, No.9, pp.5462-5469

### SUMMARY OF INVENTION

### PROBLEM TO BE SOLVED BY THE INVENTION

An objective of the present invention is to provide an anti-IGF-1 receptor humanized antibody or its fragment or a derivative thereof having a specificity and a binding affinity or activity equivalent to or higher than those of the previously reported anti-IGF-1 receptor mouse antibody IGF 11-16 (Patent Literature 1), as well as a method of producing the same.

Specific objectives of the present invention include, but are not limited to, with an aim to obtain a humanized antibody having a specificity and a binding affinity or activity equivalent to or higher than those of the previously reported anti-IGF-1 receptor mouse antibody IGF11-16 (Patent Literature 1): (1) provision of amino acid residues essential for the design of a human framework; (2) provision of amino acid positions essential for maintaining activity in the CDR sequences, which are antigen binding sites (identified by the Kabat method in the present invention); (3) provision of amino acid substitutions to reduce immunogenicity; and (4) provision of amino acid substitutions to avoid the risk of deamidation.

Utilization and application of the present invention allows for provision of an anti-IGF-1 receptor humanized antibody that can increase muscle mas via, e.g., the human IGF-1 receptor, without inducing hypoglycemic symptoms. This makes it possible to obtain an anti-IGF-1 receptor humanized antibody that can be administered to humans for the purpose of ameliorating or treating conditions or diseases related to IGF-1 receptor signaling such as, for example, sarcopenia, disuse muscular atrophy, or cachexia. It also makes it possible to provide a humanized antibody with low immunogenicity and physical stability that can be administered to humans.

### MEANS TO SOLVE THE PROBLEM

Thus, the present invention relates to, e.g., the following Aspects:
[Aspect 1] An anti-IGF-1 receptor humanized antibody or its fragment or a derivative thereof comprising:
   heavy-chain and light-chain complementarity determining regions (CDRs) each derived from mouse parent antibody IGF11-16; and
   heavy-chain and light-chain framework regions (FRs) each derived from a human antibody,
   wherein at least one of the CDRs contains a substitution of at least one amino acid residue relative to the corresponding CDR of the mouse parent antibody IGF11-16.
[Aspect 2] The anti-IGF-1 receptor humanized antibody or its fragment or a derivative thereof according to claim 1, wherein the amino acid residue at the 25th position in Framework Region 1 of the heavy chain variable region (FR-H1) is a proline residue.
[Aspect 3] The anti-IGF-1 receptor humanized antibody or its fragment or a derivative thereof according to claim 1 or 2, comprising:
   as a sequence of CDR-1 of the heavy chain variable region (CDR-H1), amino acid sequence defined in SEQ ID NO: 1, or an amino acid sequence derived from SEQ ID NO: 1 via substitution of any one amino acid residue thereof,
   as a sequence of CDR-2 of the heavy chain variable region (CDR-H2), SEQ ID NO:3or amino acid sequence defined in SEQ ID NO:5, or amino acid sequence derived from SEQ ID NO:3 or SEQ ID NO:5 via substitution of any one, two, or three amino acid residues thereof,
   as a sequence of CDR-3 of the heavy chain variable region (CDR-H3), amino acid sequence defined in SEQ ID NO:7, or an amino acid sequence derived from SEQ ID NO:7 via substitution of any one or two amino acid residues thereof,
   as a sequence of CDR-1 of the light chain variable region (CDR-L1), amino acid sequence defined in SEQ ID NO:9, or an amino acid sequence derived from SEQ ID NO:9 via substitution of any one or two amino acid residues thereof,
   as a sequence of CDR-2 of the light chain variable region (CDR-L2), amino acid sequence defined in SEQ ID NO: 11, or an amino acid sequence derived from SEQ ID NO: 11 via substitution of any one amino acid residue thereof,
   as a sequence of CDR-3 of the light chain variable region (CDR-L3), amino acid sequence defined in SEQ ID NO: 13, or an amino acid sequence derived from SEQ ID NO: 13 via substitution of any one or two amino acid residues thereof.
[Aspect 4] The anti-IGF-1 receptor humanized antibody or its fragment or a derivative thereof according to claim 1 or 2, comprising:
   as a sequence of CDR-1 of the heavy chain variable region (CDR-H1), an amino acid sequence having a homology of 80% or more to SEQ ID NO: 1,
   as a sequence of CDR-2 of the heavy chain variable region (CDR-H2), an amino acid sequence having a homology of 82% or more to SEQ ID NO:3 or SEQ ID NO:5,
   as a sequence of CDR-3 of the heavy chain variable region (CDR-H3), an amino acid sequence having a homology of 75% or more to SEQ ID NO:7,
   as a sequence of CDR-1 of the light chain variable region (CDR-L1), an amino acid sequence having a homology of 81% or more to SEQ ID NO:9,
   as a sequence of CDR-2 of the light chain variable region (CDR-L2), an amino acid sequence having a homology of 85% or more to SEQ ID NO: 11, and
   as a sequence of CDR-3 of the light chain variable region (CDR-L3), an amino acid sequence having a homology of 77% or more to SEQ ID NO: 13.
[Aspect 5] The anti-IGF-1 receptor humanized antibody or its fragment or a derivative thereof according to claim 1 or 2, comprising:
   as a sequence of CDR-1 of the heavy chain variable region (CDR-H1), an amino acid sequence derived from SEQ ID NO:1 in which Trp at the 3rd position of SEQ ID NO:1 is retained or substituted with a similar amino acid residue, the amino acid sequence further including substitution of any one amino acid residue other than the amino acid residue at the 3rd position or having a homology of 80% or more to SEQ ID NO: 1,
   as a sequence of CDR-2 of the heavy chain variable region (CDR-H2),
   an amino acid sequence derived from SEQ ID NO:3 in which Glu at the 1st position and Asn at the 3rd position of SEQ ID NO:3 are each retained or substituted with a similar amino acid residue and Asn at the 6th position is retained or substituted with Ser or Gln, the amino acid sequence further including substitution of any one, two, or three amino acid residues other than the amino acid residues at the 1st position, the 3rd position, and the 6th position or having a homology of 82% or more to SEQ ID NO:3, or
   an amino acid sequence derived from SEQ ID NO:5 in which Glu at the 1st position and Asn at the 3rd position of SEQ ID NO:5 are each retained or substituted with a similar amino acid residue and Ser at the 6th position of SEQ ID NO:5 is retained or substituted with Asn or Gln, the amino acid sequence further including substitution of any one, two, or three amino acid residues other than the amino acid residues at the 1st position, the 3rd position, and the 6th position or having a homology of 82% or more to SEQ ID NO:5,
   as a sequence of CDR-3 of the heavy chain variable region (CDR-H3), an amino acid sequence derived from SEQ ID NO:7 in which Arg at the 4th position of SEQ ID NO:7 is retained or substituted with a similar amino acid residue, the amino acid sequence further including substitution of any one or two amino acid residues other than the amino acid residue at the 4th position of SEQ ID NO:7 or having a homology of 75% or more to SEQ ID NO:7,
   as a sequence of CDR-1 of the light chain variable region (CDR-L1), an amino acid sequence derived from SEQ ID NO:9 in which Trp at the 9th position of SEQ ID NO:9 is retained or substituted with a similar amino acid residue, the amino acid sequence further including substitution of any one or two amino acid residues other than the amino acid residue at the 9th position of SEQ ID NO:9 or having a homology of 81% or more to SEQ ID NO:9,
   as a sequence of CDR-2 of the light chain variable region (CDR-L2), an amino acid sequence derived from SEQ ID NO: 11 substitution of any one amino acid residue or having a homology of 85% or more to SEQ ID NO: 11,
   as a sequence of CDR-3 of the light chain variable region (CDR-L3), an amino acid sequence derived from SEQ ID NO: 13 substitution of any one or two amino acid residues or having a homology of 77% or more to SEQ ID NO: 13.
[Aspect 6] The anti-IGF-1 receptor humanized antibody or its fragment or a derivative thereof according to any one of claims 1 to 5, which binds specifically to an extracellular domain of human IGF-1 receptor having the amino acid sequence defined in SEQ ID NO:71.
[Aspect 7] The anti-IGF-1 receptor humanized antibody or its fragment or a derivative thereof according to any one of claims 1 to 6, comprising:
   as a heavy chain variable region, an amino acid sequence defined in SEQ ID NO:43, 47, 49, 53, 55, or 59, an amino acid sequence derived from SEQ ID NO:43, 47, 49, 53, 55, or 59 via substitution, deletion, or addition of one or several amino acid residues, or an amino acid sequence having a homology of 90% or more to SEQ ID NO:43, 47, 49, 53, 55, or 59, and
   as a light chain variable region, an amino acid sequence defined in SEQ ID NO:65, 67, or 69, an amino acid sequence derived from SEQ ID NO:65, 67, or 69 via substitution, deletion, or addition of one or several amino acid residues, or an amino acid sequence having a homology of 90% or more to SEQ ID NO:65, 67, or 69.
[Aspect 8] The anti-IGF-1 receptor humanized antibody or its fragment or a derivative thereof according to any one of claims 1 to 7, comprising as a constant region of heavy and/or light chains, a constant region of heavy and/or light chains of any class of human immunoglobulin.
[Aspect 9] The anti-IGF-1 receptor humanized antibody or its fragment or a derivative thereof according to any one of claims 1 to 8, wherein the heavy chain constant region is the heavy chain constant region of human IgG4 or a region derived therefrom via substitution of 1 to 10 amino acids.
[Aspect 10] The anti-IGF-1 receptor humanized antibody or its fragment or a derivative thereof according to any one of claims 1 to 8, wherein the heavy chain constant region is the heavy chain constant region of human IgG1 or a region derived therefrom via substitution of 1 to 10 amino acids.
[Aspect 11] The anti-IGF-1 receptor humanized antibody or its fragment or a derivative thereof according to any one of claims 1 to 10, which binds to an IGF-1 receptor with an affinity represented by an equilibrium dissociation constant (KD) of 1 × 10⁻⁷ M or less.
[Aspect 12] The anti-IGF-1 receptor humanized antibody or its fragment or a derivative thereof according to any one of claims 1 to 11, which has an ability to activate IGF-1 receptor signaling.
[Aspect 13] The anti-IGF-1 receptor humanized antibody or its fragment or a derivative thereof according to any one of claims 1 to 12, which exhibits a proliferative activity in a myoblast proliferation assay.
[Aspect 14] The anti-IGF-1 receptor humanized antibody or its fragment or a derivative thereof according to any one of claims 1 to 13, which exhibits a binding affinity comparable to that of the mouse parent antibody IGF 11-16 in a BIACORE binding assay to recombinant soluble IGF-1 receptor.
[Aspect 15] The anti-IGF-1 receptor humanized antibody or its fragment or a derivative thereof according to any one of claims 1 to 14, which has an ability to induce muscle mass gain effect without inducing hypoglycemic symptoms in a normal mammal.
[Aspect 16] The anti-IGF-1 receptor humanized antibody or its fragment or a derivative thereof according to any one of claims 1 to 15, which has an ability to induce growth plate cartilage elongation effect without inducing hypoglycemic symptoms in a hypophysectomized model animal.
[Aspect 17] The anti-IGF-1 receptor humanized antibody or its fragment or a derivative thereof according to any one of claims 1 to 16, which, when administered to a vertebrate animal at a dose which induces an increase in muscle mass and/or body length, does not reduce the blood glucose level of the vertebrate animal.
[Aspect 18] The anti-IGF-1 receptor humanized antibody or its fragment or a derivative thereof according to any one of claims 1 to 16, which, even at a blood exposure level 10 times higher than an effective dose sufficient to induce an increase in muscle mass and/or body length, does not reduce the blood glucose level of a vertebrate animal.
[Aspect 19] The anti-IGF-1 receptor humanized antibody or its fragment or a derivative thereof according to any one of claims 1 to 18, which has an ability to inhibit the activation of IGF-1 receptor signaling.
[Aspect 20] The anti-IGF-1 receptor humanized antibody or its fragment or a derivative thereof according to any one of claims 1 to 18, which inhibits the proliferative activity of at least one ligand of IGF-1, IGF-2 and insulin, which ligand can activate the IGF-1 receptor in a myoblast proliferation assay.
[Aspect 21] The anti-IGF-1 receptor humanized antibody or its fragment or a derivative thereof according to any one of claims 1 to 18, which has an activity to inhibit cell proliferation in a cancer cell proliferation assay.
[Aspect 22] The anti-IGF-1 receptor humanized antibody or its fragment or a derivative thereof according to any one of claims 1 to 18, which has at least one characteristic selected from:
   (1) inhibiting the proliferation of vertebrate-derived cells induced by an IGF-1 receptor activating ligand;
   (2) inhibiting the proliferation of cells in a vertebrate animal induced by an IGF-1 receptor activating ligand in a cell proliferative disorder;
   (3) not affecting glucose uptake by differentiated muscle cells at a dose sufficient to inhibit the proliferation of vertebrate-derived cells induced by an IGF-1 receptor activating ligand; and
   (4) not affecting the blood glucose level in a vertebrate animal even at a dose sufficient to inhibit cell proliferation in a vertebrate cell proliferative disorder caused by IGF-1 receptor activating ligand.
[Aspect 23] The anti-IGF-1 receptor humanized antibody or its fragment or a derivative thereof according to any one of claims 1 to 22, which has an ability to induce an inhibitory effect on cancer cell proliferation without affecting the blood glucose level in a cancer-bearing model animal.
[Aspect 24] The anti-IGF-1 receptor humanized antibody or its fragment or a derivative thereof according to claim 23, which, even at a blood exposure level 10 times higher than an effective dose sufficient to induce an inhibitory effect on cancer cell proliferation in a cancer-bearing model animal, does not affect the blood glucose level of the model animal.
[Aspect 25] A nucleic acid molecule comprising a polynucleotide sequence encoding an anti-IGF-1 receptor humanized antibody or its fragment or a derivative thereof according to any one of claims 1 to 24.
[Aspect 26] A cloning vector or expression vector comprising at least one nucleic acid molecule according to claim 25.
[Aspect 27] A recombinant cell derived from a host cell via introduction of a vector according to claim 26.
[Aspect 28] A process of producing an anti-IGF-1 receptor humanized antibody or its fragment or a derivative thereof according to any one of claims 1 to 24, comprising:
   culturing a recombinant cell according to claim 27; and
   purifying the anti-IGF-1 receptor humanized antibody or its fragment or a derivative thereof produced by the recombinant cell.
[Aspect 29] A pharmaceutical composition comprising, as an active ingredient, an anti-IGF-1 receptor humanized antibody or its fragment or a derivative thereof according to any one of claims 1 to 24, a nucleic acid molecule according to claim 25, a vector according to claim 26, or a recombinant cell according to claim 27.
[Aspect 30] The pharmaceutical composition according to claim 29, for use in the treatment of muscle atrophic disease or dwarfism.
[Aspect 31] The pharmaceutical composition according to claim 30, wherein the muscle atrophic disease is disuse muscle atrophy, sarcopenia, or cachexia.
[Aspect 32] The pharmaceutical composition according to claim 30, wherein the dwarfism is Laron-type dwarfism or growth-hormone resistant dwarfism.
[Aspect 33] The pharmaceutical composition according to claim 29, for use in the treatment of an IGF-1 receptor associated disease.
[Aspect 34] The pharmaceutical composition according to claim 33, wherein the IGF-1 receptor associated disease is selected from the group consisting of: liver cancer, neuroblastoma, rhabdomyosarcoma, bone cancer, pediatric cancer, acromegalia, ovary cancer, pancreas cancer, benignant prostatic hypertrophy, breast cancer, prostate cancer, bone cancer, lung cancer, colorectal cancer, neck cancer, synoviosarcoma, urinary bladder cancer, stomach cancer, Wilms' tumor, diarrhea associated with metastatic carcinoid and vasoactive intestinal peptide secreting tumor, vipoma, Verner-Morrison syndrome, Beckwith-Wiedemann syndrome, kidney cancer, renal-cell cancer, transitional cell cancer, Ewing's sarcoma, leukemia, acute lymphoblastic leukemia, brain tumor, glioblastoma, non-glioblastomatous brain tumor, meningioma, pituitary adenoma, vestibular schwannoma, undifferentiated neuroectodermal tumor, medulloblastoma, astrocytoma, oligodendroglioma, brain room top swell, choroid plexus papilloma, gigantism, psoriasis, atherosclerosis, vascular smooth muscle restenosis, inappropriate microvascular growth, diabetic retinopathy, Graves' disease, multiple sclerosis, systemic erythematodes, myasthenia gravis, autoimmune thyroiditis, Hashimoto's thyroiditis, thyroid ophthalmopathy, hyperthyroidism and Behcet's disease.

### ADVANTAGEOUS EFFECTS OF INVENTION

The present invention allows for provision of an anti-IGF-1 receptor humanized antibody that binds to the human IGF-1 receptor, and that can be used for the treatment or prevention of diseases that act through the human IGF-1 receptor. The present invention also allows for provision of a humanized antibody with low immunogenicity and physical stability that can be administered to humans.

### BRIEF DESCRIPTION OF DRAWINGS

[Figure 1A] Figures 1A to 1F show the human myoblast proliferative activity of various humanized antibodies of the present invention in comparison with the mouse parent antibody IGF11-16.
[Figure 1B] Same as above.
[Figure 1C] Same as above.
[Figure 1D] Same as above.
[Figure 1E] Same as above.
[Figure 1F] Same as above.
[Figure 2] Figure 2 is a graph showing the reactivity of the humanized antibodies hIGF13_PS and hIGF25_PS of the present invention against IGF-1R of each animal species as measured by ELISA using HEK293T cells expressing IGF-1Rs of different animal species in comparison with that of the human mouse chimeric antibody IGF11-16.
[Figure 3A] Figure 3A shows the transition of the blood glucose level over time in guinea pigs treated with the humanized antibody hIGF13_PS of the present invention.
[Figure 3B] Figure 3B shows the transition of the blood glucose level over time in guinea pigs administered with the humanized antibody hIGF25_PS of the present invention.
[Figure 4] Fig. 4 shows the transition of the blood concentration over time in guinea pigs treated with the humanized antibody hIGF13_PS or hIGF25_PS of the present invention in guinea pigs in comparison with those treated with the mouse parent antibody IGF11-16.
[Figure 5] Figure 5 shows the changes in the mass of extensor digitorum longus mass after 2 weeks of a single intravenous administration of the humanized antibody hIGF13_PS to normal guinea pigs, in comparison with continuous subcutaneous administration of IGF-1 and a single intravenous administration of the mouse parent antibody IGF11-16.
[Figure 6] Figure 6 shows the change in epiphyseal thickness of the proximal tibia after 2 weeks of a single intravenous administration of the humanized antibody hIGF13_PS to pituitary-ectomized guinea pigs in comparison with continuous subcutaneous administration of IGF-1 and continuous subcutaneous administration of a GH preparation.
[Figure 7] Fig. 7 shows the change in the blood glucose level in crab-eating macaques treated with administration of the humanized antibody hIGF13_PS in comparison with those treated with IGF-1 administration.
[Figure 8] Figure 8 shows the change in the blood concentration in crab-eating monkeys treated with administration of the humanized antibody hIGF13_PS.
[Figure 9] Figure 9 shows the concentration-dependent effect of the mouse parent antibody IGF11-16 on HepG2 cell proliferation.

### DESCRIPTION OF EMBODIMENTS

The present invention will now be described below with reference to specific embodiments, although the present invention shout not be limited in any way to these embodiments. All references cited herein, including patent publications, patent application publications, and non-patent documents, are hereby incorporated by reference in their entirety for all purposes.

The unit "M," which refers to concentration, is used herein synonymously with the unit "mol/L," which refers to molar concentration.

The present invention relates to an anti-IGF-1 receptor humanized antibody that specifically binds to the IGF-1 receptor. The antibody of the present invention has a function to increase muscle mass acts via the human IGF-1 receptor without inducing hypoglycemic symptoms. This makes it possible to ameliorate or treat conditions or diseases involving IGF-1 receptor signaling, such as sarcopenia, disuse muscular atrophy, and keratoconus. In addition, the antibody of the present invention is a humanized antibody that ensures low immunogenicity and physical stability.

### [IGF]

In the present disclosure, IGF refers to as an insulin-like growth factor, which may be either IGF-1 or IGF-2. Both IGF-1 and IGF-2 are biological ligands having agonist activities which bind to an IGF-1 receptor (insulin-like growth factor-I receptor) and transduce signals such as cell division and metabolism into the cell. IGF-1 and IGF-2 are also known to have cross-avidity to an insulin receptor (INSR), which is structurally similar to the IGF-1 receptor. The present specification will mainly discuss IGF-1, since its properties such as physiological functions are known more than those of IGF-2. However, in the context of discussion about various effects and diseases mediated via binding of a ligand to the IGF-1 receptor, both IGF-1 and IGF-2 may collectively be mentioned.

IGF-1, also referred to as somatomedin C, is a single polypeptide hormone consisting of 70 amino acids. The sequence of human IGF-1 is available, e.g., with NCBI Reference Sequence number: NP_000609, or, on the EMBL-EBI, with UniProtKB accession number P05019. The amino acid sequence of mature human IGF-1 is shown in SEQ ID NO:83, and an example of the corresponding nucleotide sequence is shown in SEQ ID NO:84. This sequence consisting of 70 amino acids is conserved in many species. In the present invention, the term "IGF-1" without any limitation means an IGF-1 protein having such hormone activity, unless specified otherwise.

IGF-1 is produced by a variety of cells in the living body, including liver cells, and exists in blood and other body fluids. Therefore, wild-type IGF-1 can be obtained via purification from body fluid of an animal or from a primary cultured cell or a cultured cell line derived from an animal. Since a growth hormone induces IGF-1 production by cells, IGF-1 can also be purified from body fluid of an animal to which a growth hormone has been administered, or from a primary cultured animal cell or an animal cell line incubated in the presence of a growth hormone. As a different method, IGF-1 can also be obtained from a recombinant cell prepared by transfection of an expression vector carrying a nucleic acid molecule encoding an amino acid sequence of IGF-1 into a host such as a prokaryotic organism (e.g., E. coli) or a eukaryotic cell including a yeast, an insect cell, or a cultured mammal-derived cell, or from a transgenic animal or a transgenic plant into which an IGF-1 gene has been transfected. Human IGF-1 is also available as a research reagent (Enzo Life Sciences, catalog: ADI-908-059-0100, Abnova, catalog: P3452, etc.) or as a pharmaceutical product (Somazon^{®} mecasermin, INCRELEX^{®}, etc.). The *in vivo* and *in vitro* activities of IGF-1 for use can be evaluated as specific activities relative to an IGF-1 standard substance under NIBSC code: 91/554, whose activity corresponds to one international unit/microgram. The standard substance is available from World Health Organization's National Institute for Biological Standards and Control (NIBSC). In the context of the present invention, IGF-1 is considered as having a specific activity equivalent to the IGF-1 of NIBSC code: 91/554.

### [IGF-1 receptor]

In the present disclosure, the term "IGF-1 receptor" or "IGF-1R" refers to as an insulin-like growth factor -I receptor. The term "IGF-1 receptor" used herein means an IGF-1 receptor protein, unless specified otherwise. The IGF-1 receptor is a protein formed with two subunits, each consisting of an alpha chain and a beta chain. The amino acid sequence of a human IGF-1 receptor is indicated in SEQ ID NO:71, of which a subsequence consisting of the amino acid residues at positions 31 to 735 represents the alpha chain, while a subsequence starting from the amino acid residue at position 740 represents the beta chain. The alpha chain of the IGF-1 receptor has a portion to which IGF-1 binds, while the beta chain has a transmembrane structure and exhibits a function to transmit signals into the cell. The alpha chain of the IGF-1 receptor can be divided into L1, CR, L2, FnIII-1, and FnIII-2a/ID/FnIII-2b domains. According to the amino acid sequence of the human IGF-1 receptor defined in SEQ ID NO:71, the residues at position 31 to position 179 correspond to the L1 domain, the residues at position 180 to position 328 correspond to the CR domain, the residues at position 329 to position 491 correspond to the L2 domain, the residues at position 492 to position 607 correspond to the FnIII-1 domain, and the residues at position 608 to position 735 correspond to the FnIII-2a/ID/FnIII-2b domain. The amino acid sequence of human IGF-1 receptor is available, e.g., on EMBL-EBI with UniProtKB-accession number P08069, and is also indicated in the sequence listing as SEQ ID NO:71.

The IGF-1 receptor is known to be expressed in a wide range of tissues and cells in the living body, and receives various stimuli via IGF-1, such as induction of cell proliferation and activation of intracellular signals. In particular, effects of IGF-1 on myoblasts via the IGF-1 receptor can be evaluated using cell proliferation activities as indicators. For this reason, myoblasts are useful in analyzing the effects of antibodies binding to the IGF-1 receptor. Cells expressing an IGF-1 receptor derived from human or any other vertebrate can be prepared artificially, by transfection of an expression vector carrying a nucleic acid molecule encoding the amino acid sequence of an IGF-1 receptor derived from human or any other vertebrate into a eukaryotic host cell, such as a cultured insect cell or a mammal-derived cell, to prepare a recombinant cell expressing the IGF-1 receptor encoded by the transfected nucleic acid on its cell membrane. The resultant cell expressing the IGF-1 receptor can be used for analysis of the binding ability and intracellular signal transmissibility of antibodies.

### [Mouse parent antibody IGF11-16]

The amino acid sequence of CDR-H1 of IGF11-16 is shown in SEQ ID NO:85, the amino acid sequence of CDR-H2 in SEQ ID NO:86, the amino acid sequence of CDR-H3 in SEQ ID NO:87, the amino acid sequence of CDR-L1 in SEQ ID NO:88, the amino acid sequence of CDR-L2 in SEQ ID NO:89, and the amino acid sequence of CDR-L3 in SEQ ID NO:90. The amino acid sequence of the heavy chain variable region is shown in SEQ ID NO:39 (an example of the corresponding nucleotide sequence is shown in SEQ ID NO:40), and the amino acid sequence of the light chain variable region is shown in SEQ ID NO:41 (an example of the corresponding nucleotide sequence is shown in SEQ ID NO:42). The full-length amino acid sequence of the light chain of IGF 11-16 is shown in SEQ ID NO:91 (an example of the corresponding nucleotide sequence is shown in SEQ ID NO:92), and the full-length amino acid sequence of the heavy chain is shown in SEQ ID NO:93 (an example of the corresponding nucleotide sequence is shown in SEQ ID NO:94). All antibodies having names including the expression IGF 11-16 refer to this mouse parent antibody IGF11-16.

### [Anti-IGF-1 receptor humanized antibody]

One aspect of the present invention provides a novel anti-IGF-1 receptor humanized antibody (hereinafter referred to as "the antibody of the present invention" as appropriate).

In the present disclosure, the term "an antibody" indicates a glycoprotein containing at least two heavy (H) chains and two light (L) chains coupled together via disulfide bindings. Each heavy chain has a heavy chain variable region (abbreviated as VH) and a heavy chain constant region. The heavy chain constant region contains three domains, i.e., CH1, CH2, and CH3. Each light chain contains a light chain variable region (abbreviated as VL) and a light chain constant region. Alight chain constant region has one domain, i.e., CL. There are two types of light chain constant regions, i.e., λ (lambda) chain and κ (kappa) chain. Heavy chain constant regions are classified into γ (gamma) chain, µ (mu) chain, α (alpha) chain, δ (delta) chain and ε (epsilon) chain, and different types of heavy chain constant regions result in different isotypes of antibodies, i.e., IgG, IgM, IgA, IgD, and IgE, respectively. Each of the VH and VL is also divided into four relatively conserved regions (FR-1 (FR1), FR-2 (FR2), FR-3 (FR3), and FR-4 (FR4)), collectively referred to as framework regions (FR), and three highly variable regions (CDR-1 (CDR1), CDR-2 (CDR2), and CDR-3 (CDR3)), collectively referred to as complementarity determining regions (CDR). The VH region includes the three CDRs and the four FRs arranged in the order of FR-1 (FR-H1), CDR-1 (CDR-H1), FR-2 (FR-H2), CDR-2 (CDR-H2), FR-3 (FR-H3), CDR-3 (CDR-H3), and FR-4 (FR-H4) from the amino terminal to the carboxyl terminal. The VL includes the three CDRs and the four FRs arranged in the order of FR-1 (FR-L1), CDR-1 (CDR-L1), FR-2 (FR-L2), CDR-2 (CDR-L2), FR-3 (FR-L3), CDR-3 (CDR-L3), and FR-4 (FR-L4) from the amino terminal to the carboxyl terminal. The variable region of each of the heavy chain and the light chain includes a binding domain, which interacts with an antigen.

The antibody of the present invention may be a fragment and/or derivative of an antibody. Examples of antibody fragments include F(ab')2, Fab, and Fv. Examples of antibody derivatives include: antibodies to which an amino acid mutation has been introduced in its constant region; antibodies in which the domain arrangement of the constant regions has been modified; antibodies having two or more Fc's per molecule; antibodies consisting only of a heavy chain or only of a light chain; antibodies with modified glycosylation; bispecific antibodies; conjugates of antibodies or antibody fragments with compounds or proteins other than antibodies; antibody enzymes; nanobodies; tandem scFv's; bispecific tandem scFv's; diabodies; and VHHs. The term "antibody" used herein encompasses such fragments and/or derivatives of antibodies, unless otherwise specified.

The term monoclonal antibody classically refers to an antibody molecule obtained from a clone derived from a single antibody-producing cell, but in the present disclosure, refers to a single type of antibody molecule containing a combination of VH and VL consisting of a specific amino acid sequence. It is also possible to obtain from a monoclonal antibody a nucleic acid molecule having a gene sequence encoding the amino acid sequence of the antibody protein, which nucleic acid molecule can be used to produce a genetically engineered antibody. It is also well known to those skilled in the art to use genetic information of the sequences of an H chain and an L chain, or their variable regions or CDR sequences, for modifying an antibody to improve its binding ability and specificity, or for modifying an antibody derived from an animal such as mouse to a human-type antibody having a structure suitable for use as a therapeutic agent. It is also possible to obtain a human monoclonal antibody by preparing a non-human transgenic animal into which a human antibody gene has been introduced and sensitizing the animal with an antigen. In addition, as a method that does not require sensitization of animals, a person skilled in the art can also employ a technique including using a phage library expressing antigen-binding regions of human antibodies or parts thereof (human antibody phage display) can be used to obtain phage clones presenting antibodies that specifically bind to the corresponding antigen or specific amino acid sequences, and producing a human antibody using the information from the obtained phase clones (see, e.g., Non-Patent Literature 17). A person skilled in the art can also design an antibody to be administered to a non-human animal by using the amino acid sequence information of CDRs and variable regions as appropriate, in a similar manner to humanization techniques.

According to one aspect, the antibody of the present invention is an anti-IGF-1 receptor humanized antibody that contains complementarity determining regions (CDRs) in each of the heavy and light chains derived from the mouse parent antibody IGF11-16, and framework regions (FRs) in each of the heavy and light chains derived from a human antibody, wherein at least one of the CDRs contains a substitution of at least one amino acid residue relative to the corresponding CDR of the mouse parent antibody IGF11-16.

Specifically, according to the present aspect, each of the complementarity determining regions (CDRs) of the heavy and light chains is derived from the corresponding CDR of the mouse parent antibody IGF11-16. The mouse parent antibody "IGF11-16" herein refers to an anti-IGF-1 receptor monoclonal mouse antibody previously produced by the inventors, as explained above (Patent Literature 1). The term "derived" from the CDR of the mouse parent antibody herein means that the amino acid sequence of each CDR of the antibody of this aspect is homologous (preferably, identical) to the amino acid sequence of the corresponding CDR of the mouse parent antibody IGF11-16 with a homology (preferably, an identity) of typically 75% or more, particularly 80% or more, or 85% or more, or even particularly 90% or more, and/or with the exception of a difference of typically four amino acid residues or less, particularly three amino acid residues or less, and even particularly two amino acid residues or less (Non-Patent Literature 18). However, the antibody of the present aspect requires that at least one of its CDRs contains a substitution of at least one amino acid residue relative to the corresponding CDR of the mouse parent antibody IGF 11-16. In addition, the amino acid sequence of the heavy chain CDR-2 (CDR-H2) should only be homologous (preferably identical) to either the amino acid sequence of the CDR-H2 of the mouse parent antibody IGF 11-16 or the CDR-H2 of the humanized antibody hIGF 13 _PS derived from the mouse parent antibody IGF11-16 as described below, with a homology (preferably, an identity) of typically 75% or more, particularly 80% or more, or 85% or more, or even particularly 90% or more, and/or with the exception of a difference of typically four amino acid residues or less, particularly three amino acid residues or less, and even particularly two amino acid residues or less.

In addition, each framework region (FR) of the heavy and light chains is derived from the corresponding FR of each class of human immunoglobulin, respectively. The term "derived from" the FR of a human immunoglobulin herein means that the amino acid sequence of each FR of the antibody of this form is homologous (preferably identical) to the amino acid sequence of the corresponding FR of the human immunoglobulin, with a homology (preferably, an identity) of typically 80% or more, particularly 85% or more, or even particularly 90% or more, and/or with the exception of a difference of typically four amino acid residues or less, particularly three amino acid residues or less, and even particularly two amino acid residues or less. Human immunoglobulin frameworks are available from public databases, and can be used for selecting frameworks with high homology to mouse immunoglobulin frameworks. Amino acid sequences having high homology can be identified using, e.g., IgBLAST (Non-Patent Literature 16).

The amino acid residue at position 25 of the heavy chain FR1 herein may preferably be proline. Although there are several different amino acid residues between the heavy chain FR1 of the mouse parent antibody IGF 11-16 and the heavy chain FR1 of the humanized antibody, the inventors' investigation revealed that the amino acid residue at position 25 of the heavy chain FR1, which is serine in the humanized antibody, may preferably be replaced with proline, as in the mouse parent antibody IGF11-16, since as described later in Example 3, the humanized antibody can exhibit activity equivalent to or higher than that of the mouse parent antibody IGF11-16 (the "equivalent" activity herein means that the ratio of the activity is within the range of ±20%).

The heavy and light chains having the above homology can be obtained via, e.g., evolutionary engineering of antibodies, using the sequences of the heavy and light chains derived from the humanized antibodies of the present invention as templates. Specific examples include site-directed mutagenesis, random mutagenesis of CDRs, chain shuffling, CDR walking, etc.

"Random mutagenesis" is a method of generating mutants by introducing random mutations into specific genetic DNA. According to PCR mutagenesis, mutations are introduced by DNA amplification under specific conditions with low replication stringency (error-prone PCR), whereby mutations are introduced at arbitrary sites throughout the DNA amplified by PCR. According to DNA shuffling, the target gene is first fragmented, and mutations are introduced to the resulting fragments in the same manner as the PCR mutagenesis. Random mutations can also be introduced in an intended region or in a sitespecific manner by mixing several bases in a specific synthetic step during DNA synthesis

"Chain shuffling" is a method in which one of the VH or VL genes of the antibody variable regions is immobilized, and the other is combined with a V gene library to construct a library. The constructed library is expressed on phages, and then screened for combinations of antibody variable regions having high specificity for the original antigen. This method is the first choice for in vitro affinity maturation of antibodies obtained from naive/non-immune libraries.

"CDR walking" is a method in which random mutations are introduced into each CDR of the VH and VL genes, and the resulting population of mutants is subjected to screening using specific conditions to select antibodies having strong binding activity. The selected CDRs are then combined to obtain a clone having even stronger binding activity. In general, random mutations may be introduced only in CDR3 for further investigation.

Once a humanized parent antibody having specific activity is thus obtained, the parent antibody can then be used as a template and modified into a new humanized antibody with maintaining its activity using a methodology which has almost completely been established, and such a process can be outsourced to, e.g., CRO.

According to one aspect, the antibody of the present invention may preferably have a specific amino acid sequence as each CDR sequence. Specific examples are described below. The "identity" between amino acid sequences herein refers to the percentage of identical amino acid residues between the sequences, and the "similarity" between amino acid sequences herein refers to the percentage of identical or similar amino acid residues between the sequences. The homology and identity between amino acid sequences can be determined by, e.g., the BLAST method (the default conditions of NCBI's PBLAST). The term "similar amino acid residues" herein refers to amino acid residues that have side chains with similar chemical properties (e.g., charge or hydrophobicity). Examples of groups of similar amino acid residues are shown below. The groups below mean that in the case of replacing, e.g., an alanine residue with a similar amino acid residue, it should be replaced with a valine, leucine, isoleucine, or methionine residue.
(1) Amino acid residues with aliphatic side chains: alanine (Ala or A), valine (Val or V), leucine (Leu or L), isoleucine (Ile or 1), and methionine (Met or M) residues.
(2) Amino acid residues with aliphatic hydroxyl side chains: serine (Ser or S) and threonine (Thr or T) residues.
(3) Amino acid residues with amide-containing side chains: asparagine (Asn or N) and glutamine (Gln or Q) residues.
(4) Amino acid residues with aromatic side chains: phenylalanine (Phe or F), tyrosine (Tyr or Y), tryptophan (Trp or W), and histidine (His or H) residues.
(5) Amino acid residues with basic side chains: lysine (Lys or K), arginine (Arg or R), and histidine (His or H) residues.
(6) Amino acid residues with acidic side chains: aspartic acid (Asp or D) and glutamic acid (Glu or E) residues.
(7) Amino acid residues with sulfur-containing side chains: cysteine (Cys or C) and methionine (Met or M) residues.

The CDR-1 sequence of the heavy chain variable region (CDR-H1) may preferably be the amino acid sequence of SEQ ID NO:1, or an amino acid sequence derived from SEQ ID NO:1 via substitution of one amino acid residue. Alternatively, the CDR-H1 sequence may preferably have 80% or more homology (preferably, identity) with SEQ ID NO:1. Particularly preferable among them as the CDR-H1 sequence are amino acid sequences having the Trp residue at position 3 of SEQ ID NO: 1 maintained or replaced with a similar amino acid residue, and also having any one amino acid residue other than the residue at position 3 maintained or replaced with a similar amino acid residue, or also having 80% or more homology (preferably, identity) with SEQ ID NO:1. An example of the nucleic acid sequence corresponding to SEQ ID NO:1 is shown in SEQ ID NO:2.

The CDR-2 sequence of the heavy chain variable region (CDR-H2) may preferably be the amino acid sequence of SEQ ID NO:3 or SEQ ID NO:5, or an amino acid sequence derived from SEQ ID NO:3 or SEQ ID NO:5 via substitution of one, two, or three amino acid residues. Alternatively, the CDR-H2 sequence may preferably have 82% or more homology (preferably, identity) with SEQ ID NO:3 or SEQ ID NO:5. Particularly preferable among them as the CDR-H2 sequence are amino acid sequences having the Glu residue at position 1 and the Asn residue at position 3 of SEQ ID NO:3 each maintained or replaced with a similar amino acid residue, and the Asn residue at position 6 of SEQ ID NO:3 maintained or replaced with Ser or Gln, and also having any one, two, or three amino acid residues other than the residues at positions 1, 3, and 6 each maintained or replaced with a similar amino acid residue, or also having 82% or more homology (preferably, identity) with SEQ ID NO:3. Alternatively, particularly preferable as the CDR-H2 sequence are amino acid sequences having the Glu residue at position 1 and the Asn residue at position 3 of SEQ ID NO:5 each maintained or replaced with a similar amino acid residue, and the Ser residue at position 6 of SEQ ID NO:5 maintained or replaced with Asn or Gln, and also having any one, two, or three amino acid residues other than the residues at positions 1, 3, and 6 each maintained or replaced with a similar amino acid residue, or also having 82% or more homology (preferably, identity) with SEQ ID NO:5. Examples of the nucleic acid sequences corresponding to SEQ ID NO:3 and SEQ ID NO:5 are shown in SEQ ID NO:4 and SEQ ID NO:6, respectively.

The CDR-3 sequence of the heavy chain variable region (CDR-H3) may preferably be the amino acid sequence of SEQ ID NO:7, or an amino acid sequence derived from SEQ ID NO:7 via substitution of one or two amino acid residues. Alternatively, the CDR-H3 sequence may preferably have 75% or more homology (preferably, identity) with SEQ ID NO:7. Particularly preferable among them as the CDR-H3 sequence are amino acid sequences having the Arg residue at position 4 of SEQ ID NO:7 maintained or replaced with a similar amino acid residue, and also having any one or two amino acid residues other than the residue at position 4 each maintained or replaced with a similar amino acid residue, or also having 75% or more homology (preferably, identity) with SEQ ID NO:7. An example of the nucleic acid sequence corresponding to SEQ ID NO:7 is shown in SEQ ID NO:8.

The CDR-1 sequence of the light chain variable region (CDR-L1) may preferably be the amino acid sequence of SEQ ID NO:9, or an amino acid sequence derived from SEQ ID NO:9 via substitution of one or two amino acid residues. Alternatively, the CDR-L1 sequence may preferably have 81% or more homology (preferably, identity) with SEQ ID NO:9. Particularly preferable among them as the CDR-L1 sequence are amino acid sequences having the Trp residue at position 9 of SEQ ID NO:9 maintained or replaced with a similar amino acid residue, and also having any one or two amino acid residues other than the residue at position 9 each maintained or replaced with a similar amino acid residue, or also having 81% or more homology (preferably, identity) with SEQ ID NO:9. An example of the nucleic acid sequence corresponding to SEQ ID NO:9 is shown in SEQ ID NO:10.

The CDR-2 sequence of the light chain variable region (CDR-L2) may preferably be the amino acid sequence of SEQ ID NO: 11, or an amino acid sequence derived from SEQ ID NO: 11 via substitution of one amino acid residue. Alternatively, the CDR-L2 sequence may preferably have 85% or more homology (preferably, identity) with SEQ ID NO: 11. An example of the nucleic acid sequence corresponding to SEQ ID NO: 11 is shown in SEQ ID NO:10.

The CDR-3 sequence of the light chain variable region (CDR-L3) may preferably be the amino acid sequence of SEQ ID NO: 13, or an amino acid sequence derived from SEQ ID NO: 13 via substitution of one amino acid residue. Alternatively, the CDR-L3 sequence may preferably have 77% or more homology (preferably, identity) with SEQ ID NO: 13. An example of the nucleic acid sequence corresponding to SEQ ID NO: 13 is shown in SEQ ID NO:14.

The antibody of the present invention may particularly preferably have specific combinations of CDR sequences indicated below. Specifically, the antibody of the present invention may preferably have the combination of the amino acid sequence of SEQ ID NO:1 as the CDR-H1 sequence, the amino acid sequence of SEQ ID NO:3 or SEQ ID NO:5 as the CDR-H2 sequence, the amino acid sequence of SEQ ID NO:7 as the CDR-H3 sequence, the amino acid sequence of SEQ ID NO:9 as the CDR -L1 sequence, the amino acid sequence of SEQ ID NO: 11 as the CDR-L2 sequence, and the amino acid sequences of SEQ ID NO: 13 as the CDR-L3 sequence.

Examples of methods for identifying the CDR-H1, CDR-H2, CDR-H3, CDR-L1, CDR-L2, and CDR-L3 sequences in antibody sequences include the Kabat method (Non-Patent Literature 13) and the Chothia method (Non-Patent Literature 14), as well as methods improved from these methods (Non-Patent Literature 15). These methods are well-known to those skilled in the art, and can be learnt from, e.g., from the Internet homepage of Dr. Andrew C.R. Martin's Group (http://www.bioinf.org.uk/abs/).

In addition, as shown in Example 4, an alanine scan can be performed to identify the sites in the amino acid sequence of the CDR that are important for binding activity. From the results, it is clear that the amino acid residues shown in Table 7 and Table 8 below are extremely important. Substitution of at least these amino acid residues in this site with amino acids which do not have similar properties is expected to lead to decreased binding ability. In contrast, substitution with amino acids having similar properties may lead to an increase in binding affinity. On the other hand, among the 54 alanine substituted CDR sites, 44 sites maintained more than 80% of the binding activity even after alanine substitution. This suggests that the amino acid substitutions at these sites do not significantly affect the binding activity. Thus, scanning through the amino acid sequences of the CDR regions to identify the sites playing a role in binding to the antigen may serve to reduce immunogenicity, improve physical properties, and enhance binding while maintaining the binding property.

The antibody of the present invention should preferably have specific amino acid sequences as the sequences of heavy chain and light chain variable regions. Specific examples of the sequences are shown below. The phrase "one or several positions" herein refers to one, two, three, four, five, six, seven, eight, nine, or ten positions, unless otherwise noted.

The antibody of the present invention may preferably have, as the heavy chain variable region, the amino acid sequence of SEQ ID NO:47, or an amino acid sequence derived from SEQ ID NO:47 via substitution, deletion, or addition of one or more amino acid residues. Alternatively, the antibody of the present invention may preferably have, as the heavy chain variable region, an amino acid sequence having 90% or more homology (preferably, identity) with SEQ ID NO:47. Particularly preferred among them as the heavy chain variable region are the amino acid sequence of VH13_PN (SEQ ID NO:43), VH13 _PS (SEQ ID NO:47), VH23_PN (SEQ ID NO:49), VH23_PS (SEQ ID NO:53), VH25_PN (SEQ ID NO:55), or VH25_PS (SEQ ID NO:59). Examples of nucleic acid sequences corresponding to the amino acid sequences of SEQ ID NOs:43, 47, 49, 53, 55, and 59 are shown in SEQ ID NOs:44, 48, 50, 54, 56, and 60, respectively.

The antibody of the present invention may preferably have, as the light chain variable region, the amino acid sequence of SEQ ID NO:67, or an amino acid sequence derived from SEQ ID NO:67 via substitution, deletion, or addition of one or more amino acid residues. Alternatively, the antibody of the present invention may preferably have, as the light chain variable region, an amino acid sequence having 90% or more homology (preferably, identity) with SEQ ID NO:67. Particularly preferred among them as the light chain variable region are the amino acid sequence of VL13 (SEQ ID NO:61), VL14 (SEQ ID NO:63), VL22 (SEQ ID NO:65), VL23 (SEQ ID NO:67), or VL24 (SEQ ID NO:69) as the light chain variable region. Even more preferred are the amino acid sequence of VL22 (SEQ ID NO:65), VL23 (SEQ ID NO:67), or VL24 (SEQ ID NO:69). Examples of nucleic acid sequences corresponding to the amino acid sequences of SEQ ID NOs:61, 63, 65, 67, and 69 are shown in SEQ ID NOs:62, 64, 66, 68, and 70, respectively.

The antibody of the present invention may more preferably have any of the amino acid sequences described above as the heavy chain variable region and the light chain variable region. Particularly preferred as the antibody of the present invention are: the antibody having the amino acid sequence of VH13_PS (SEQ ID NO:47) as the heavy chain variable region and the amino acid sequence of VL23 (SEQ ID NO:67) as the light chain variable region (hereinafter referred to as "hIGF13_PS"); and the antibody having the amino acid sequence of VH25_PS (SEQ ID NO:59) as the heavy chain variable region and the amino acid sequence of VL23 (SEQ ID NO:67) as the light chain variable region (hereinafter referred to as "hIGF25_PS").

The amino acid sequence of each of the constant regions of the heavy and light chains of the antibody of the invention can be selected from, e.g., the amino acid sequences of the human IgG, IgA, IgM, IgE, and IgD classes as well as their variants. According to one aspect, the amino acid sequence of the heavy chain constant region of the antibody of the present invention may preferably have the amino acid sequence of the heavy chain constant region of the human IgG4 class, or an amino acid sequence derived therefrom via one to ten amino acid residues thereof (Non-Patent Literatures 19 and 20). According to another aspect, the amino acid sequence of the heavy chain constant region of the antibody of the present invention may preferably have the amino acid sequence of the heavy chain constant region of the human IgG1 class, or an amino acid sequence derived therefrom via one to ten amino acid residues thereof (Non-Patent Literatures 19 and 20).

The antibody of the present invention causes an antigen-antibody reaction with the human IGF-1 receptor. The term "antigen-antibody reaction" herein refers to the binding of an antibody to the IGF-1 receptor with an affinity of an equilibrium dissociation constant (KD) of 1 × 10⁻⁷ M or less. The antibodies of the present invention usually bind to the IGF-1 receptor with a KD of 1×10⁻⁷ M or less, preferably 1×10⁻⁸ M or less, and even 1×10⁻⁹ M or less. Most preferably, it is 1×10⁻¹⁰ M or less.

The antibody of the present invention may preferably have the ability to specifically bind to the extracellular domain of the human IGF-1 receptor having the amino acid sequence of SEQ ID NO:71. The term "specificity" of an antibody to an antigen herein means that a high antigen-antibody reaction occurs between the antibody and the antigen. The term "IGF-1 receptor-specific antibody" herein refers to an antibody whose antigen-antibody reactivity to INSR, which has high similarity to the higher-order structure of IGF-1 receptor, is less than 1/100 at a concentration that causes significant antigen-antibody reaction with cells expressing IGF-1 receptor.

A person skilled in the art can measure the antigen-antibody reaction employing a binding assay in a solid-phase or liquid-phase system selected as appropriate. Examples of such methods include, although not limited to: enzyme-linked immunosorbent assay (ELISA), enzyme immunoassay (EIA), surface plasmon resonance (SPR), fluorescence resonance energy transfer (FRET), luminescence resonance energy transfer (LRET), etc. Antigen-antibody reaction can also be detected by labelling the antibody and/or the antigen with an appropriate label substance such as enzymes, fluorescent substances, luminescent substances, radioisotopes, etc., and detecting the reaction employing a measurement method suitable for the physical and/or chemical properties of the label substance.

According to one aspect, the antibody of the present invention may preferably have an IGF-1 receptor signaling activity equivalent to or greater than that of the mouse parent antibody IGF11-16. The term "equivalent" in terms of IGF-1 receptor signaling activity herein means that the
value is within 2-fold and/or the Eₘₐₓ value is within ±20%.

According to one aspect, the antibody of the present invention may preferably have a proliferative activity equivalent to or greater than that of the mouse parent antibody IGF11-16. The term "equivalent" in terms of proliferative activity herein means that the EC₅₀ value is within 2-fold and/or the Eₘₐₓ value is within ±20% in a myoblast proliferation assay.

According to one aspect, the antibody of the present invention may preferably have a binding affinity to a recombinant soluble IGF-1 receptor that is equivalent to or higher than the mouse parent antibody IGF 11-16. The term "equivalent" in terms of proliferative activity in terms of binding affinity to the recombinant soluble IGF-1 receptor herein means that the KD value is within the range of from 1/3 to 3 times that of the mouse parent antibody IGF11-16.

According to one aspect, the antibody of the present invention may preferably have a long half-life in blood, and exhibit muscle mass increasing effect via a single administration to an animal. Actually, according to the inventors' study, the anti-IGF-1 receptor humanized antibody of the present invention, when administered as a single dose to a guinea pig or a crab-eating macaque, exhibited a muscle mass-increasing effect equivalent to that achieved via continuous administration of IGF-1, as explained in the Examples below.

According to one aspect, the antibody of the present invention may preferably induce muscle mass increasing effect without inducing hypoglycemic symptoms in a normal mammal. According to one aspect, the antibody of the present invention may preferably induce growth plate cartilage elongation effect without inducing hypoglycemic symptoms in a hypophysectomized model animal. The term "hypoglycemic symptoms" herein refers to, in the case of the human, symptoms such as cold sweat, palpitations, disturbance of consciousness, convulsions, and tremors of limbs that occur with hypoglycemia. In the case of vertebrates such as monkeys, spontaneous movements decrease as an initial symptom, movements almost completely disappear as symptoms grow stronger, and consciousness is impaired as the blood glucose level drops further, leading to death.

When administered to a vertebrate at a dose that causes an increase in muscle mass, IGF-1 exhibits a marked hypoglycemic effect and usually induces hypoglycemic symptoms. However, according to one aspect, the antibody of the present invention may not induce such a hypoglycemic effect on a vertebrate, even when administered at a dose that induces an increase in muscle mass and/or body length, or more preferably at a dose 10 times higher than that dose. According to one aspect, the antibody of the present invention may not have the effect of lowering the blood glucose level in a vertebrate even when administered at a blood exposure level that is 10 times higher than the effective dose that induces an increase in muscle mass and/or body length of the vertebrate. In fact, according to the inventors' examination, the antibody of the present invention did not induce hypoglycemic symptoms in a guinea pigs or a crab-eating macaque even when administered at a dose of 10 mg/kg, as shown in the Examples below.

In summary, the anti-IGF-1 receptor humanized antibody of the present invention has the potential to be a therapeutic or prophylactic agent for various diseases related to the IGF-1 receptor, such as disuse muscle atrophy and dwarfism, while overcoming the problematic hypoglycemic effect expected to be caused by IGF-1, and thereby allowing for prolonged half-life in blood.

The antibody of the present invention is deemed to activate both the homo-type receptor, in which IGF-1 receptor molecules form a dimer, and the hetero-type receptor, in which an IGF-1 receptor molecule and an INSR molecule form a dimer, by binding to the extracellular domain of the IGF-1 receptor molecule(s).

### [Evaluation of immunogenicity]

Since Anti-Drug Antibodies (ADAs) may affect the efficacy and pharmacokinetics of therapeutic antibodies and sometimes result in serious side effects, thus the utility and efficacy of therapeutic antibodies in clinical practice can be limited by their ADA production. Although many factors influence the immunogenicity of therapeutic antibodies, the importance of effector T-cell epitopes present in therapeutic proteins has been widely reported. Various in silico tools for predicting T cell epitopes have been developed, such as Epibase (Lonza), iTope/TCED (Antitope), and EpiMatrix (EpiVax). Employing these in silico tools allows for prediction of the presence of T-cell epitopes in each amino acid sequence (Non-Patent Literature 21), whereby potential immunogenicity can be evaluated. For the anti-IGF-1 receptor humanized antibody of the present invention, potential immunogenicity was assessed using Epibase (Lonza).

### [Deamidation risk]

Among possible sequences of amino acids that make up proteins, NG, NT, NS, and NN are known to be prone to deamidation. The presence of any of these sequences may cause deamidation of the asparagine residue therein to an aspartic acid residue, resulting in a possible decrease in the activity of the antibody as well as a loss of uniformity in quality. In order to maintain quality during the manufacture and storage process, amino acids at risk of deamidation may be replaced with other amino acids to thereby prevent any loss of its activity and maintain its uniform quality. With regard to the antibody of the present invention, since the heavy chain CDR-2 (CDR-H2) region of the mouse parent antibody IGF11-16 includes an NS sequence (positions 55 and 56 of the heavy chain), the asparagine (N) residue at position 55 of the heavy chain was replaced with serine (S), in order to avoid the risk of this asparagine (N) being deamidated and converted to aspartic acid (D).

### [Evaluation of stability of physical properties]

In general, in order to ensure that the activity of the antibody is stably maintained for a long period of time, the stability of physical properties is examined by increasing the temperature or changing the pH. In the case of the anti-IGF-1 receptor humanized antibody of the present invention, the stability of physical properties was confirmed using a PBS solution of this antibody as a sample, by incubating it for one month at 37°C, and confirming that the purity was 95% or more and that no aggregates were produced.

### [Epitope of the anti-IGF-1 receptor humanized antibody]

According to one aspect, the antibody of the present invention recognizes the CR domain of the IGF-1 receptor as an epitope. It is preferable that the antibody of the present invention may bind to an epitome that contains a peptide having the amino acid sequence corresponding to the amino acid residues from position 308 to position 319 (ProSerGlyPheIleArgAsnGlySerGlnSerMet) in the amino acid sequence of the human IGF-1 receptor (SEQ ID No:71), or to a sequence in the vicinity thereof. The antibody of the present invention is deemed to activate both the homo-type receptor, in which IGF-1 receptor molecules form a dimer, and the hetero-type receptor, in which an IGF-1 receptor molecule and an INSR molecule form a dimer, by binding to the CR domain of the IGF-1 receptor molecule(s).

### [Anti-IGF-1 receptor humanized agonist antibody]

The agonist antibody of the present invention may preferably be in the form of human IgG class or variants thereof, human IgG4 subclass or variants thereof, or human IgG1 subclass or variants thereof. In one example, the stabilized IgG4 constant region contains a proline at position 241 of the hinge region by Kabat's system (Non-Patent Literature 22). This position corresponds to position 228 in the hinge region according to the EU numbering scheme (Non-Patent Literature 13). In human IgG4, this residue is generally serine, while stabilization can be induced by replacing this serine with proline. In one example, the N297A mutation can be incorporated into the constant region of IgG1 to suppress as much as possible its abilities to bind to the Fc receptor and/or to anchor a complement. According to another aspect, an amino acid substitution can be introduced in the constant region in order to modulate its ability to bind to FcRn and thereby increase its half-life in blood. However, possible amino acid substitutions that can be introduced in the constant region are not limited to these examples.

The agonist antibody of the present invention may bind specifically and potently to the IGF-1 receptor and have the effect of increasing myoblast proliferation from very low concentrations in vitro.

The agonist antibody of the present invention may exhibit, when administered as a single dose to an animal, an effect of increasing muscle mass which is comparable to that of continuous administration of IGF-1. The agonist antibody of the present invention may also have a long half-life in the blood, and exhibit an effect of increasing muscle mass after a single administration to an animal. In fact, when administered as a single dose to a guinea pig or crab-eating macaque, the agonist antibody of the present invention exhibited the same level of muscle mass-increasing effect as that caused by continuous administration of IGF-1.

The agonist antibody of the present invention may also be characterized by not inducing a hypoglycemic effect at doses that induce muscle mass gain. IGF-1 has a marked hypoglycemic effect when administered at doses that induce muscle mass gain. However, the agonist antibody of the present invention may not have a hypoglycemic effect in a vertebrate at doses that induce an increase in muscle mass and/or body length in the vertebrate. It is preferred that the agonist antibody of the present invention may not have the effect of lowering the blood glucose level in a vertebrate, even when administered at a blood exposure level that is 10 times higher than the effective dose that induces an increase in muscle mass and/or body length in the vertebrate. In fact, even when the agonist antibody of the present invention was administered to a guinea pig or crab-eating macaque at a blood exposure level that is10 times higher than the effective dose to induce an increase in muscle mass, no symptoms associated with an decrease in the blood glucose level or hypoglycemia were observed.

Based on the findings above, the agonist antibody of the present invention has the potential to be a therapeutic or prophylactic agent for various diseases related to the IGF-1 receptor, such as sarcopenia, disuse muscular atrophy, cathexis, and dwarfism, while overcoming the problematic hypoglycemic effect expected to be caused by IGF-1, and thereby allowing for prolonged half-life in blood.

### [Anti-IGF-1 receptor humanized antagonist antibody]

The anti-IGF-1 receptor humanized antibody of the present invention can be made into an anti-IGF-1 receptor antagonist antibody with excellent activity and specificity, by taking advantage of the extremely high binding ability and specificity of its variable regions. In this aspect, the antibody of the present invention may be used not only as IgG but also in any other form such as, although not limited to, Fab, Fv, scFv, or VHH.

The anti-IGF-1 receptor antagonist antibodies produced in this manner can be evaluated based on, e.g., its ability to inhibit IGF-1-dependent cell proliferation activity in a cancer cell line. The anti-IGF-1 receptor antagonist antibody selected in this manner is expected to be used as an anti-cancer agent and as a drug for improving and treating diseases and conditions associated with abnormal cell proliferation.

This antibody can also be used for constructing bispecific or multispecific antibodies by fusing it directly or via a linker with various antibodies that recognize other antigens or epitopes. In this case, the antibody may be used not only as IgG but also in any other forms such as, although not limited to, Fab, Fv, scFv, or VHH.

The bispecific or multispecific antibody containing the anti-IGF-1 receptor antagonist antibody produced in this manner can be evaluated based on, e.g., its ability to inhibit IGF-1-dependent cell proliferation activity in a cancer cell line. The bispecific or multispecific antibody containing the anti-IGF-1 receptor antagonist antibody produced in this manner is expected to be used as an anti-cancer agent and as a drug for improving and treating diseases and conditions associated with abnormal cell proliferation.

The anti-IGF-1 receptor antagonist antibody of the present invention is expected to be a therapeutic agent for the treatment of diseases whose pathological conditions can be induced by the activation of IGF-I receptor signaling. Ligands that can activate IGF-I receptors include IGF-1, IGF-2, and Insulin, as well as ligands for RTKs (receptor-type tyrosine kinases) that form heterodimers with IGF-1 receptors (e.g., EGF) and ligands for other receptors that cross-talk (e.g., TSH). The antibody of the present invention may have the activity to suppress IGF-1 receptor signaling activated by these ligands (allosteric antagonist action). In other words, the antibody of the present invention may suppress the excessively induced IGF-1 receptor signaling activity by binding to the IGF-1 receptor, and may be used for the treatment or prevention of diseases induced by the abnormal activation of the IGF-1 receptor. The antibody of the present invention may preferably have the ability to suppress signal activation in a level that is equivalent to or greater than that of the mouse parent antibody IGF11-16. The phrase "equivalent to mouse antibody IGF11-16" herein refers to an activity to inhibit by 10% or more, preferably 25% or more, particularly preferably 35% or more of the maximum cell proliferative activity that can be induced by ligands that can activate IGF-I receptors such as IGF-1, IGF-2, or Insulin in a myoblast proliferation assay.

Specific examples of diseases induced by abnormal activation of IGF-1 receptors include: liver cancer, neuroblastoma, rhabdomyosarcoma, bone cancer, pediatric cancer, acromegalia, ovary cancer, pancreas cancer, benignant prostatic hypertrophy, breast cancer, prostate cancer, bone cancer, lung cancer, colorectal cancer, neck cancer, synoviosarcoma, urinary bladder cancer, stomach cancer, Wilms' tumor, diarrhea associated with metastatic carcinoid and vasoactive intestinal peptide secreting tumor, vipoma, Verner-Morrison syndrome, Beckwith-Wiedemann syndrome, kidney cancer, renal-cell cancer, transitional cell cancer, Ewing's sarcoma, leukemia, acute lymphoblastic leukemia, brain tumor, glioblastoma, non-glioblastomatous brain tumor, meningioma, pituitary adenoma, vestibular schwannoma, undifferentiated neuroectodermal tumor, medulloblastoma, astrocytoma, oligodendroglioma, brain room top swell, choroid plexus papilloma, gigantism, psoriasis, atherosclerosis, vascular smooth muscle restenosis, inappropriate microvascular growth, diabetic retinopathy, Graves' disease, multiple sclerosis, systemic erythematodes, myasthenia gravis, autoimmune thyroiditis, Hashimoto's thyroiditis, thyroid ophthalmopathy, hyperthyroidism and Behcet's disease. These effects can be confirmed by using a cancer-bearing model animal.

### [Anti-IGF-1 receptor humanized antibody as a local delivery tool]

The anti-IGF-1 receptor antibody can be used for local delivery of drugs and antibodies to IGF-1 receptor-expressing cells and tissues by utilizing its extremely high binding ability and specificity. In this case, the antibody may be used not only as IgG but also in any other forms such as, although not limited to, Fab, Fv, scFv, or VHH. An antibody-drug conjugate containing the anti-IGF-1 receptor antibody of the present invention conjugated to a drug can deliver the drug to a local target, whereby the drug can specifically exert its efficacy at a lower dose, leading to a reduction in side effects.

The thus-produced conjugate of a drug or antibody with the anti-IGF-1 receptor humanized antibody of the present invention as a delivery tool can be evaluated based on, if the drug is an apoptosis-inducing agent, its ability to induce apoptosis in IGF-1 receptor-expressing cancer cell line. The thus-selected conjugate of a drug or antibody with the anti-IGF-1 receptor humanized antibody of the present invention is expected to be used as an anti-cancer agent and as a drug for improving and treating diseases and conditions associated with abnormal cell proliferation.

Alternatively, the anti-IGF-1 receptor antibody of the present invention can be labeled with a radioactive or fluorescent compound for use in detecting cancer cells expressing IGF-1 receptors. Use of such a diagnostic technique allows for efficient treatment using an anti-IGF-1 receptor antagonist antibody.

### [Competitive Binding]

Antibodies that bind to the IGF-1 receptor in a competitive manner with the anti-IGF-1 receptor antibody of the present invention are also included in the scope of the present invention. The term "competitive binding" herein refers to a phenomenon in which when two or more monoclonal antibodies co-exist with an antigen, the binding of one of the antibodies to the antigen is inhibited by the binding of another of the antibodies to the antigen. In general, it can be measured by adding to a fixed amount (concentration) of a monoclonal antibody a different monoclonal antibody with increasing the amount (concentration) of the latter antibody, and measuring the amount (concentration) of the latter antibody at which the binding of the former antibody to the antigen decreases. The degree of its inhibition can be expressed in terms of IC₅₀ or Ki.

The phrase "a monoclonal antibody that binds to the antigen in a competitive manner with the anti-IGF-1 receptor antibody of the present invention" means an antibody that has, when antigen-antibody binding is detected using the anti-IGF-1 receptor antibody of the present invention at 10 nM, an IC₅₀ of usually 1000 nM or less, particularly 100 nM or less, or even particularly 10 nM or less. When measuring competitive binding, one or more of the antibodies used can be labelled with an appropriate label substance such as enzymes, fluorescent substances, luminescent substances, radioisotopes, etc., and the reaction can be detected by employing a measurement method suitable for the physical and/or chemical properties of the label substance.

### [Cross-reactivity]

The antibody of the present invention may have cross-reactivity with the IGF-1 receptors of other vertebrates. The term "cross-reactivity" of an antibody herein refers to the binding ability of the antibody to the IGF-1 receptor of another animal species different from the target animal species (e.g., human) to which the antibody is designed to cause antigen-antibody reaction. The anti-IGF-1 receptor humanized antibody of the present invention may exhibits cross-reactivity with, in addition to the human IGF-1 receptor, IGF-1 receptors of other animals such as guinea pigs, monkeys, rabbits, etc. On the other hand, it does not cause cross-reaction with mouse and rat IGF-1 receptors.

It is also possible to use an animal species that do not cross-react with the antibody of the present invention, and genetically engineer a cell or animal of that species to produce a cell or animal expressing IGF-1 receptors with which the antibody of the present invention can cross-react.

### [Evaluation of binding affinity]

The anti-IGF-1 receptor humanized antibody of the present invention may have an extremely strong binding affinity at a level equivalent to or higher than that of the mouse parent antibody IGF11-16 (Patent Literature 1). The binding affinity can be evaluated by, e.g., SPR (surface plasmon resonance) analysis using the extracellular region of the recombinant IGF-1 receptor as an antigen. In the Examples below, the binding affinity of monovalent is analyzed by using BIACORE, raising the reaction temperature to 40°C and keeping the amount of fixed antigens low, although the methods for analyzing the binding affinity are not limited to this specific method, but may be any analytical methods that can quantitatively evaluate strong binding affinity.

### [Evaluation of IGF-1 receptor signaling]

The anti-IGF-1 receptor humanized antibody of the present invention has been obtained by selecting humanized antibodies with levels equivalent to or higher than that of the mouse parent antibody IGF 11-16 (Patent Literature 1) by using their ability to activate IGF-1 receptor signal as a primary evaluation system.

For the evaluation of IGF-1 receptor signal activation, we used the commercially available PathHunter^{®} IGFIR Functional Assay (manufactured by DiscoverX). This system allows for evaluation of phosphorylation directly under the IGF-1 receptor signal in terms of enzymatic activity, by using a chemiluminescent substance as substrate, and measuring the signal intensity based on the luminescence intensity.

Specifically, a cell line used was HEK293 cells engineered to forcedly express an adapter protein SHC1-Enzyme Acceptor (EA) fusion protein with an SH2 domain that binds to the IGF-1 receptor and the intracellular tyrosine kinase of the IGF-1 receptor. In this cell line, ligand binding to the IGF-1 receptor leads to receptor dimerization, followed by receptor phosphorylation, which recruits an adapter protein with an SH2 domain to form a receptor signaling complex, whereby the binding of EA to the spatially adjacent tyrosine kinase is promoted, and an active β-galactosidase is reconstituted. The level of chemiluminescence signal of the substrate hydrolyzed by this β-galactosidase activity can be measured for identifying the action of the drug on the receptor-type tyrosine kinase.

Humanized antibody variants with signal-inducing activity equivalent to or higher than that of the mouse parent antibody IGF11-16 were selected and then subjected to a secondary evaluation based on human myoblast proliferation. The means to evaluate IGF-1 receptor signaling is not limited to this specific method, but may be any system that can detect IGF-1 receptor tyrosine phosphorylation directly or indirectly and quantitatively.

### [Proliferation-inducing activity of vertebrate-derived cells and muscle mass-increasing activity]

Human myoblast cell proliferation assay was carried out as a secondary evaluation system for the humanized antibody of the present invention, whereby humanized antibodies with agonist activity equivalent to or higher than that of the mouse parent antibody IGF 11-16 (Patent Document 1) in the same concentration range were narrowed down. The humanized antibodies selected in this manner were confirmed not to exhibit hypoglycemic effects in vivo, but to have the effect of increasing muscle mass. In other words, the anti-IGF-1 receptor humanized antibody of the present invention in one form has the ability to induce proliferation of vertebrate-derived cells.

The term "vertebrate-derived cells" in the context of the present disclosure should preferably be cells derived from mammals, birds, reptiles, amphibia, or fish, more preferably cells derived from mammals or birds, further more preferably cells derived from human, monkey, rabbit, guinea pig, cow, pig, sheep, horse, dog, rat, or mouse. Cells derived from these species which express an IGF-1 receptor with which the antibody of the present invention cross-reacts can be induced to proliferate by the antibody of the present invention. The "vertebrate-derived cells" according to the present disclosure also encompass: cells and animals engineered to express an IGF-1 receptor of a species with which the antibody of the present invention cross-reacts; and modified animal cells derived from such engineered cells and animals.

An antibody's proliferation-inducing activity of vertebrate-derived cells can be analyzed *in vitro* using primary cultured cells, established cell lines, or transformants derived from such cells.

In the present disclosure, the term "primary cultured cells" means cells which were isolated from an organ or a tissue of a living organism, and can typically be subcultured for some passages. Primary cultured cells derived from a vertebrate can be obtained from an organ or a tissue of the vertebrate via enzyme treatment, dispersion with physical means, or explant method. An organ or a tissue or its fragment obtained from the vertebrate can also be used for analyzing the antibody's activity above. Preferable examples of organs and tissues from which primary cells are prepared include: endocrine tissues such as thyroid, parathyroid, and adrenal gland; immune tissues such as appendix, tonsil, lymph nodes, and spleen; respiratory organs such as trachea and lung; digestive organs such as stomach, duodenum, small intestine, and large intestine; urinary organs such as kidney and urinary bladder; male genital organs such as vas deferens, testicle, and prostate; female genital organs such as breast and fallopian tube; and muscle tissues such as heart muscle and skeletal muscles. More preferred examples include liver, kidney, or digestive organs or muscle tissues, among which muscle tissues are still more preferred. Primary cultured cells which can be used for analyzing the proliferation-inducing activity of an antibody of the present invention are cells which express an IGF-1 receptor and can be induced to proliferate by IGF-1 binding to the IGF-1 receptor. Typical examples thereof are skeletal muscle myoblasts, which are primary cultured cells isolated from muscle tissue. Human- or animal-derived primary cultured cells available by assignment or commercially on the market can also be obtained and used. Human primary cultured cells are available from various institutions and companies, e.g., ATCC^{®}, ECACC, Lonza, Gibco^{®}, Cell Applications, ScienCell research laboratories, and PromoCell.

Methods for determining the cell proliferation-inducing activity by the antibody of the present invention in vertebrate-derived cells include: cell counting, measurement of DNA synthesis, and measurement of change in the metabolic enzyme activity. Methods for cell counting include methods using blood cell counting plates or cell counting devices such as Coulter counters. Methods for measuring DNA synthesis include methods based on uptake of [3H]-thymidine or 5-bromo-2'-deoxyulysine (BrdU). Method for measuring the change in metabolic enzyme activity include colorimetric quantitative methods such as MTT method, XTT method, and WST method. A person skilled in the art could also employ other methods as appropriate.

The cell proliferation-inducing activity can be determined by that the proliferation of cultured cells reacted with the antibody of the present invention increases compared to that of cultured cells not reacted with the antibody. In this case, the inducing activity can favorably be normalized through the measurement using IGF-1, an original ligand of the IGF-1 receptor, that is reacted under the same conditions as a control. An EC₅₀ value indicates a concentration at which 50% of the maximum proliferation-inducing activity is given in the case that the antibody of the present invention and IGF-1 are reacted with various concentrations to the cultured cells. In the case that the proliferation-inducing activity is evaluated with human skeletal muscle myoblast cells, the antibody of the present invention may preferably have an EC₅₀ value in the cell proliferation-inducing activity equivalent to or lower than that of IGF-1, more preferably an EC₅₀ value of 1/10 or less, further more preferably 1/20 or less, most preferably 1/50 or less that of IGF-1. In addition, in the case that the proliferation-inducing activity is evaluated with human skeletal muscle myoblast cells, the antibody of the present invention may preferably have an EC₅₀ value of preferably 0.5 nM or less, more preferably 0.3 nM or less, most preferably 0.1 nM or less.

Methods for measuring the activity to induce cell growth *in vivo* include: a method involving administering the antibody of the present invention to a vertebrate and measuring changes in the mass, size, cell count, etc., for the entire body of the individual which received the administration or for an organ or a tissue isolated from the individual; and a method involving using an animal with a graft of vertebrate cells and measuring changes in the mass, size, cell count, etc., of the graft including vertebrate cells. Measurements for the entire body of an individual include: measurements of the body mass, the body length, and the circumferences of four limbs; measurement of the body composition, using impedance method; and measurement of the creatinine height coefficient. Measurements for an organ, a tissue, or a graft from an individual include: in the case of a non-human animal, a method involving directly recovering the target organ, tissue or graft and measuring its mass, size, or the number of cells included therein. Non-invasive measurements for an organ, a tissue, or a graft from an individual include: image analysis using X-ray photography represented by Dual-energy X-ray absorptiometry (DXA), CT, and MRI; and contrast methods using tracers with isotopes or fluorescent substances. If the target tissue is skeletal muscle, then a change in the muscle force can also be used as an indicator. A person skilled in the art could also employ any other methods as appropriate for analyzing the activity of the antibody of the present invention to induce growth of vertebrate-derived cells *in vivo.* Methods for measuring the activity of the antibody of the present invention to induce growth of vertebrate-derived cells *in vivo* include: carrying out measurements using, e.g., the methods mentioned above for individuals who received administration of the antibody of the present invention and individuals who received administration of a different antibody other than the antibody of the present invention or any other control substance, and comparing the resultant measurements between these individuals.

With regard to the hemodynamics of antibodies, Example 14 below indicates comparison of the guinea pig hemodynamics of the hIGF13_PS and hIGF25_PS antibodies, which are the antibodies of the present invention, with those of the mouse IGF11-16 antibody (Patent Literature 1), which was the basis for designing the antibodies of the present invention. This example shows that the antibodies of the present invention have improved hemodynamics compared to IGF11-16.

One of the in vivo effects achieved by the antibody of the present invention is the effect of increasing the muscle mass and/or the body length. Specifically, IGF-1 has an effect of inducing the growth and differentiation of myoblasts in skeletal muscles as mentioned above, as well as an effect of broadening muscle fibers. It is expected that these effects collectively lead to the effect of increasing the muscle mass. Like IGF-1, when the antibody of the present invention is administered to an animal, it also exhibits an effect of increasing the muscle mass of the animal.

Methods for measuring the activity of the antibody of the present invention to increase the muscle mass include: for the entire body of the individual which received the administration, measurement of the body mass, the body length, and the circumferences of four limbs; measurement of the body composition, using impedance method; and measurement of the creatinine, and height coefficient. Other methods include: image analysis using X-ray photography represented by Dual-energy X-ray absorptiometry (DXA), CT, and MRI; contrast methods using tracers with isotopes or fluorescent substances; and measurement of a change in the muscle force. In the case of a non-human animal, a method involving directly recovering the target organ, tissue or graft and measuring its mass and/or size can also be used.

The effect of increasing the muscle mass can be evaluated by: comparing the muscle mass increases between an individual to which the antibody of the present invention was administered and an individual to which the antibody was not administered; or comparing the muscle masses of an individual before and after administration of the antibody of the present invention. The effect of increasing the muscle mass can be determined if there is any increase in the muscle mass of an individual before and after the administration of the antibody of the present invention. IGF-1 also plays a role in the bone growth, and has an effect of increasing the body length (the body height in the case of the human). Therefore, the antibody of the present invention also exhibits an effect of increasing the body length when administered to an animal. The effect of the antibody of the present invention in increasing the body length of an individual can be determined by measuring the body weight, the body length, and the circumferences of four limbs of the individual.

### [Effects on blood glucose levels in animals]

According to one aspect, the antibody of the present invention may have the feature of not affecting the blood glucose level in a vertebrate. IGF-1 is known to have the activity to lower the blood glucose level as a part of its agonist actions on the IGF-1 receptor. However, the agonist antibody of the present invention, which functions as an anti-IGF-1 receptor agonist antibody, exhibits the feature of not altering the blood glucose level even at a blood exposure that is 10 times higher than the effective dose that induces an increase in muscle mass when administered parenterally to an animal.

The feature of not inducing hypoglycemia in a vertebrate, which feature is characteristic of the antibody of the present invention, can also be evaluated in vitro. The antibody of the present invention does not affect glucose uptake by a vertebrate-derived cell in vitro. Primary cultured cells, strain cells, or transformed cells of these cells can be used as cells for evaluating this feature of the antibodies of the present invention.

Examples of methods for determining the effect of the antibody of the present invention on the glucose uptake by vertebrate-derived cells include: measurement of the intracellular glucose concentration; measurement of the intracellular uptake of a glucose analog tracer substance; and measurement of a change in the amount of a glucose transporter. Methods for measuring the glucose concentration include absorbance measurement methods such as enzyme method. Methods for measuring the intracellular uptake amount of a glucose analog tracer substance include measurement of the uptake amount of, e.g., [3H]-2'-deoxyglucose. Methods for measuring a change in the amount of a glucose transporter include immunocytostaining and western blotting. A person skilled in the art could also employ other methods as appropriate. The fact that there is no effect on the intracellular glucose uptake can be confirmed if the intracellular glucose uptake of the cultured cells reacted with the antibody of the present invention is almost the same of the intracellular glucose uptake of the cultured cells in the absence of the antibody. In this case, it is convenient to also carry out the measurement under the same conditions using IGF-1, which is an original ligand for the IGF-1 receptor, as a control.

Methods for determining the glucose uptake by vertebrate-derived cells *in vivo* include: methods involving parenterally administering the antibody of the present invention to a vertebrate and determining a change in the glucose content of an organ or a tissue of the individual. Methods of measurement for the entire body of the individual which received the administration include: measurement of the blood glucose level; and hemoglobin A1C using glycosylated proteins as indicators. Methods of measuring the glucose uptake for an organ or a tissue of an individual include: in the case of a non-human animal, directly recovering the target organ or tissue, and calculating the concentration of glucose or a tracer. Non-invasive methods for measuring the glucose uptake individual for an organ or a tissue of an individual include: image analysis using X-ray photography, CT, and MRI; and contrast methods using tracers with isotopes or fluorescent substances. If the target tissue is a skeletal muscle, then the glucose clamp can also be used as an indicator. A person skilled in the art could also employ any other methods as appropriate for analyzing the effect of the antibody of the present invention on the glucose uptake by vertebrate-derived cells *in vivo.*

The antibody of the present invention is also characterized in that when administered to a vertebrate even at an effective dosage sufficient to increase the muscle mass of the vertebrate, preferably at a dosage of 10 times or more the effective dosage, it does not change the blood glucose level of the vertebrate. When evaluating the effect of the antibody of the present invention in changing the blood glucose level of a vertebrate, it is preferred to use an animal belonging to mammals, birds, reptiles, amphibia or fish, more preferably an animal belonging to mammals or birds, still more preferably human, monkey, rabbit, guinea pig, cow, pig, sheep, horse, dog, rat, or mouse. An animal engineered to express an IGF-1 receptor of a species which has cross-reactivity with the antibody of the present invention can also be used as an animal for evaluating the effect of the antibody of the present invention in changing the blood glucose level. Invasive methods for measuring the blood glucose level include colorimetric method and electrode method. Examples of enzyme methods used for detection include glucose oxidase method (GOD method) and glucose dehydrogenase method (GDH method). Non-invasive methods include optical measurement methods. A person skilled in the art can also select any other method as appropriate. In the case of human, the normal range of fasting blood glucose level is from 100mg/dL to 109mg/dL. With regard to adverse events in the blood glucose level resulting from a drug administration (Common Terminology Criteria for Adverse Events v4.0), the blood glucose level of lower than the range of from 77mg/dL to 55mg/dL is defined as an indicative of low blood glucose, while a blood glucose level of higher than the range of from 109mg/dL to 160mg/dL is defined as an indicative of high blood glucose. A drug administration is considered as not affecting the blood glucose level when the blood glucose level after the drug administration is higher than 55mg/dL and lower than 160mg/dL, more preferably higher than 77mg/dL and lower than 109mg/dL. However, the normal value of blood glucose level and its range of fluctuation vary depending on the animal to which a drug is administered, and even a human subject may not always have a blood glucose level within a normal range at the time of the drug administration. Accordingly, in the context of the present invention, the antibody of the present invention should preferably be considered as not changing the blood glucose level of a vertebrate to which the antibody is administered when the change in the blood glucose level of the vertebrate is preferably 30% or less, more preferably 20% or less, still more preferably 10% or less, compared to the solvent-administered control group.

### [Process for producing anti-IGF-1 receptor humanized antibody]

The antibody of the present invention can be obtained by humanizing the mouse monoclonal antibody against the IGF-1 receptor, IGF11-16 (Patent Literature 1). Humanization is a process of using a monoclonal antibody derived from non-human animal species and grafting its CDR regions into human frameworks by CDR grafting (Non-Patent Literature12). Subsequently, based on three-dimensional structural analysis, the resulting antibody is subjected to introduction of amino acid substitutions intended to reduce immunogenicity to humans (T-cell antigenicity) and/or amino acid substitutions intended to avoid the risk of post-translational modifications such as deamidation and oxidation, while maintaining its three-dimensional structure. Thus, a humanized antibody can be produced that maintains its activity while ensuring manufacturability and clinical safety.

It is very important for the humanization process to obtain information on (1) what kind of human framework design is needed in order to maintain its activity and (2) which amino acids in the CDR sequences are essential. Examples of methods for obtaining such humanized antibodies are described in Examples 1 to 9 below. The humanized antibodies thereby obtained include: humanized antibodies having VH13_PN (SEQ ID NO:43), VH13_PS (SEQ ID NO:47), VH23_PN (SEQ ID NO:49), VH23_PS (SEQ ID NO:53), VH25_PN (SEQ ID NO:55), or VH25_PS (SEQ ID NO:59) as the heavy chain variable region, and VL13 (SEQ ID NO:61), VL14 (SEQ ID NO:63), VL22 (SEQ ID NO:65), VL23 (SEQ ID NO:67), or VL24 (SEQ ID NO:69) as the light chain variable region, and more preferably, VL22 (SEQ ID NO:65), VL23 (SEQ ID NO:67), or VL24 (SEQ ID NO:69) as the light chain variable region. However, the antibodies of the present invention are not limited to these specific antibodies.

A nucleic acid molecule having a base sequence encoding the amino acid of the protein in the resultant anti-IGF-1 receptor humanized antibody can be produced, and such a nucleic acid molecule is also genetically engineered to produce an antibody. The H chain, L chain, or their variable regions in gene information of the antibody can be modified to improve the avidity and specificity of the antibody with reference to information of, for example, CDR sequences.

In a method of producing the antibody of the present invention, for example, mammalian cells, insect cells, and Escherichia coli into which genes encoding the amino acids of proteins in target antibodies are introduced are cultured, and thereby the antibody can be produced through purification of the resultant culture supernatant by a conventional process. A specific method is illustrated below.

A nucleic acid molecule encoding an H chain variable region is bound to a nucleic acid molecule encoding an H chain signal peptide and a nucleic acid molecule encoding an H chain constant region to produce the antibody of the present invention. A nucleic acid molecule encoding an L chain variable region is bound to a nucleic acid molecule encoding an L chain signal peptide and a nucleic acid molecule encoding an L chain constant region to produce the antibody of the present invention.

These H chain gene and L chain gene are incorporated into a vector, for example, a cloning vector or an expression vector, suitable for expression in a selected host cell. In this case, the H chain gene and the L chain gene may be incorporated into one vector or separate vectors such that both genes can be expressed.

The vector into which the H chain gene and the L chain gene are incorporated is then introduced into the host cell. Examples of host cells include eukaryotic cells, such as mammalian cells, insect cells, yeast cells or plant cells, and bacterial cells. A method of introducing the genes into the host cell may be appropriately selected from a chemical method such as calcium phosphate process or a lipofection process, a physical method such as an electroporation process or a particle gun process, and a method based on infection with a virus or a phage. The host cell into which the H chain gene and L chain gene are introduced can be used in culturing without any selection, selectively condensing of recombinant cells into which the genes are introduced using properties of, for example drug resistance and auxotrophy, or culturing of recombinant clone cells constructed from a single host cell into which the genes are introduced.

The host cell into which the H chain gene and L chain gene are introduced is cultured under an optimum medium and culturing condition. In this process, the products of the H chain gene and the L chain gene expressed in the host cell are usually secreted into the medium as antibody proteins, and the produced antibody proteins can be recovered by collecting the medium. However, through combining of the genes and the host cell, the antibody proteins accumulated in the cell can be recovered by destruction of the host cell as needed, or the antibody proteins can be recovered from a periplasm fraction in the case of a prokaryotic cell. Examples of methods generally used for purifying an antibody from a sample such as a medium containing the recovered antibody proteins include salt precipitation; enrichment or solvent exchange by dialysis and ultrafiltration; and affinity chromatography using a carrier that contains, for example, immobilized protein A, protein G, or antigen. Also available are ion exchange chromatography, hydrophobic chromatography, mixed mode chromatography, and size exclusion chromatography. A variety of techniques used in these methods is well known to those skilled in the art.

In this connection, a person skilled in the art can produce various antibodies such as antibody chimeric proteins, low molecule antibodies, and scaffold antibodies using known techniques, e.g., by making a genetic modification to a gene encoding a heavy chain and/or a light chain of an immunoglobulin for introducing a desired trait, or by using structure information of variable regions or CDR regions of a heavy chain and/or a light chain of an immunoglobulin. In addition, in order to improve the performance of the antibody or avoiding side effects, it is possible to introduce a modification into the structure of a constant region of an antibody or to introduce glycosylation sites of an antibody, using techniques well-known to persons skilled in the art as appropriate.

### [Drug containing the anti-IGF-1 receptor humanized antibody]

The antibody of the present invention can be used as a therapeutic agent or a prophylactic agent or a diagnostic agents for conditions associated with IGF-1 or diseases caused by effects on IGF-1 receptors. The therapeutic agents, prophylactic agents, or diagnostic agents will be collectively referred to as "drugs" or "agents."

Specifically, conditions associated with IGF-1 or diseases that can be the target of therapy or prevention using the anti-IGF-1 receptor agonist antibody include, although not limited to: muscular atrophy disease (e.g., disuse muscle atrophy, sarcopenia and cachexia), dwarfism (e.g., Laron type dwarfism and growth hormone resistant dwarfism), hepatic cirrhosis, hepatic fibrosis, diabetic nephropathy, chronic renal failure, aging, intrauterine growth restriction (IUGR), neurological diseases, stroke, spinal cord injury, cardiovascular protection, diabetes, insulin resistant, metabolic syndrome, nephropathy, osteoporosis, cystic fibrosis, wound healing, myotonic dystrophy, AIDS-associated sarcopenia, HIV-associated fat redistribution syndrome, burns, Crohn's disease, Werner's syndrome, X-linked combined immunodeficiency disease, hearing loss, anorexia nervosa and retinopathy of prematurity, Turner's syndrome, Prader-Willi syndrome, Silver-Russell syndrome, idiopathic dwarfism, obesity, multiple sclerosis, ulcerous colitis, low muscle mass, myocardial ischemia, and decreased bone density.

The antibody of the invention may preferably be for use as a therapeutic or prophylactic agent for muscle atrophic disease (e.g., disuse muscular atrophy, sarcopenia, cathexis, etc.) and/or dwarfism (e.g., Laron-type short stature, growth hormone-resistant short stature, etc.). The antibody of the present invention may also be superior in that it does not cause fluctuations in the blood glucose level upon administration. An antibody drug, antibody-drug conjugate, or diagnostic agent in which a part or all of the anti-IGF-1 receptor antibody as a component can be used for treating or preventing or diagnosing diseases including: neuroblastoma, rhabdomyosarcoma, bone cancer, pediatric cancer, acromegalia, ovary cancer, pancreas cancer, benignant prostatic hypertrophy, breast cancer, prostate cancer, bone cancer, lung cancer, colorectal cancer, neck cancer, synoviosarcoma, urinary bladder cancer, stomach cancer, Wilms' tumor, diarrhea associated with metastatic carcinoid and vasoactive intestinal peptide secreting tumor, vipoma, Verner-Morrison syndrome, Beckwith-Wiedemann syndrome, kidney cancer, renal-cell cancer, transitional cell cancer, Ewing's sarcoma, leukemia, acute lymphoblastic leukemia, brain tumor, glioblastoma, non-glioblastomatous brain tumor, meningioma, pituitary adenoma, vestibular schwannoma, undifferentiated neuroectodermal tumor, medulloblastoma, astrocytoma, oligodendroglioma, brain room top swell, choroid plexus papilloma, gigantism, psoriasis, atherosclerosis, vascular smooth muscle restenosis, inappropriate microvascular growth, diabetic retinopathy, Graves' disease, systemic erythematodes, myasthenia gravis, autoimmune thyroiditis, Hashimoto's thyroiditis, thyroid ophthalmopathy, hyperthyroidism, and Behcet's disease.

A drug containing the antibody of the present invention may be formulated in the form of a pharmaceutical composition which contains, in addition to the antibody of the present invention, a pharmaceutically acceptable carrier and/or any other excipient. Drug formulation using a pharmaceutically acceptable carrier and/or any other excipient can be carried out in accordance with, e.g., a method described in the University of the Sciences in Philadelphia, "Remington: The Science and Practice of Pharmacy, 20th EDITION", Lippincott Williams & Wilkins, 2000.

Such an agent may be provided as a liquid formulation prepared by dissolving, suspending, or emulsifying the ingredients into sterile aqueous medium or oily medium, or as a lyophilized formulation thereof. A medium or solvent for preparing such a formulation may be an aqueous medium, examples of which include distilled water for injection and physiological saline solution, which may optionally be used with addition of an osmoregulating agent (e.g., D-glucose, D-sorbitol, D-mannitol, and sodium chloride), and/or in combination with a suitable dissolving aid such as an alcohol (e.g., ethanol), a polyalcohol (e.g., propylene glycol or polyethylene glycol), or a nonionic surfactant (e.g., polysorbate 80 or polyoxyethylene hydrogenated castor oil 50). Such a formulation can also be prepared with an oily medium or solvent, examples of which include sesame oil and soybean oil, which can optionally be used in combination with a dissolving aid such as benzyl benzoate and benzyl alcohol. Such liquid drugs may often be prepared using appropriate additives such as buffering agents (e.g., phosphate buffering agents and acetate buffering agents), soothing agents (e.g., benzalkonium chloride and procaine hydrochloride), stabilizers (e.g., human serum albumin and polyethylene glycol), preservatives (e.g., ascorbic acid, erythorbic acid, and their salts), coloring agents (e.g., copper chlorophyll β-carotene, Red #2 and Blue #1), antiseptic agents (e.g., paraoxybenzoic acid esters, phenol, benzethonium chloride and benzalkonium chloride), thickeners (e.g., hydroxypropyl cellulose, carboxymethyl cellulose, and their salts), stabilizers (e.g., human serum albumin mannitol and sorbitol), and odor correctives (e.g., menthol and citrus aromas).

Other alternative forms include agents for application onto mucous membranes, such formulations often containing additives such as pressure-sensitive adhesives, pressure-sensitive enhancers, viscosity regulators, thickening agents and the like (e.g., mucin, agar, gelatin, pectin, carrageenan, sodium alginate, locust bean gum, xanthan gum, tragacanth gum, gum arabic, chitosan, pullulan, waxy starch, sucralfate, cellulose and its derivatives (such as hydroxypropyl methyl cellulose), polyglycerol fatty acid esters, acrylic acid-alkyl (meth)acrylate copolymers, or their salts and polyglycerol fatty acid esters), primarily for the purpose of imparting mucosal adsorption or retention properties. However, the form, solvent and additives for the therapeutic agent or prophylactic agent to be administered to the body are not limited to these, and appropriately selection may be made by a person skilled in the art.

A drug containing the antibody of the present invention may further contain, in addition to the antibody of the present invention, another known agent (active ingredient). It can also be fused or linked to other drugs such as antibody-drug conjugates or bispecific or multispecific antibodies. A drug containing the anti-IGF-1 receptor antibody of the present invention may be combined with another known agent in the form of a kit. Examples of active ingredients to be combined with the anti-IGF-1 receptor agonist antibody include: growth hormone or an analog thereof, insulin or an analog thereof, IGF-2 or an analog thereof, an anti-myostatin antibody, myostatin antagonist, anti-activin type IIB receptor antibody, activin type IIB receptor antagonist, soluble activin type IIB receptor or an analog thereof, ghrelin or an analog thereof, follistatin or an analog thereof, a beta-2 agonist, and a selective androgen receptor modulator.

In the preparation of an antibody drug or antibody-drug conjugate containing the anti-IGF-1 receptor antibody of the present invention as a component, examples of active ingredients to be combined with the anti-IGF-1 receptor antibody or to be included with the anti-IGF-1 receptor antibody include: corticosteroid, antiemetic, ondansetron hydrochloride, granisetron hydrochloride, metoclopramide, domperidone, haloperidol, cyclizine, lorazepam, prochlorperazine, dexamethasone, levomepromazine, tropisetron, cancer vaccine, GM-CSF inhibitor, GM-CSF DNA vaccine, cell-based vaccine, dendritic cell vaccine, recombinant virus vaccine, heat shock protein (HSP) vaccine, homologous tumor vaccine, autologous tumor vaccine, analgesic, ibuprofen, naproxen, choline magnesium trisalicylate, oxycodone hydrochloride, anti-angiogenic, antithrombotic, anti-PD-1 antibody, nivolumab, pembrolizumab, anti-PD-L1 antibody, atezolizumab, anti-CTLA4 antibody, ipilimumab, anti-CD20 antibody, rituximab, anti-HER2 antibody, trastuzumab, anti-CCR4 antibody, mogamulizumab, anti-VEGFantibody, bevacizumab, anti-VEGF receptor antibody, soluble VEGF receptor fragment, anti-TWEAK antibody, anti-TWEAK receptor antibody, soluble TWEAK receptor fragment, AMG 706, AMG 386, antiproliferative, farnesyl protein transferase inhibitor, alpha v beta 3 inhibitor, alpha v beta 5 inhibitor, p53 inhibitor, Kit receptor inhibitor, ret receptor inhibitor, PDGFR inhibitor, growth hormone secretion inhibitor, angiopoietin inhibitor, tumor-infiltrating macrophage inhibitor, c-fms inhibitor, anti-c-fms antibody, CSF-1 inhibitor, anti-CSF-1 antibody, soluble c-fms fragment, pegvisomant, gemcitabine, panitumumab, irinotecan, and SN-38. The dosage of the other agent used in combination with the antibody may be within a dosage used for normal therapy, but can be increased or decreased depending on the situation.

The agents according to the present invention can be parenterally administered for the purpose of improving symptoms. For parenteral administration, a transnasal agent may be prepared, and a liquid drug, suspension or solid formulation may be selected. An injection may be prepared as a different form of parenteral administration, the injection being selected as subcutaneous injection, intravenous injection, infusion, intramuscular injection, intracerebroventricular injection or intraperitoneal injection. Other formulations used for parenteral administration include suppositories, sublingual agents, percutaneous agents and transmucosal administration agents other than transnasal agents. In addition, intravascular local administration is possible by a mode of addition or coating onto a stent or intravascular obturator.

The dose for an agent for treatment or prevention according to the invention will differ depending on the patient age, gender, body weight and symptoms, the therapeutic effect, the method of administration, the treatment time, or the types of active ingredients in the medical composition, but normally it may be administered in the range of 0.1 mg to 1 g and preferably in the range of 0.5 mg to 100 mg of active compound per administration for adults, once every one to four weeks, or once every one to two months. However, since the administration dose and frequency will vary depending on a variety of conditions, lower administration dose and fewer administration frequency than those mentioned above may be sufficient, or administration dose and frequency exceeding these ranges may be necessary.

### EXAMPLES

The present invention will now be described in more detail by way of the following Examples. However, the present invention should not be construed to be limited to these Examples, but can be implemented in any form without departing from the spirit of the present invention.

### [Example 11 Design of humanized antibodies based on the mouse antibody IGF11-16:

### ^{∗}Selection of human frameworks

A mouse monoclonal antibody against the IGF-1 receptor, IGF 11-16, was generated by the hybridoma method of Kohler et al (Nature, (1975), Vol. 256, pp. 495-497) (Patent Literature 1). From this antibody, the complementarity determining region (CDR) amino acids in the heavy chain variable region (VH) and light chain variable region (VL) were transferred into template human antibodies. As the template human antibodies, two different humanized antibody frameworks were prepared based on germlines of human antibodies having amino acid sequences highly homologous to the VH and VL amino acid sequences (SEQ ID NO: 39 and 41, respectively) of mouse antibody IGF11-16 (mouse parent antibody), by selecting VH-1-46 (SEQ ID NO: 95) and VH-1-e (SEQ ID NO: 96) as heavy chain sequences, JH4 (SEQ ID NO: 97) as a heavy chain J-segment, VK1-L5 (SEQ ID NO: 98) and VK1-A20 (SEQ ID NO: 99) as light chain sequences, and JK2 (SEQ ID NO: 100) as a light chain J-segment, and combining these sequences as shown in Table 1 below.

### [Table 1]

**Table 1**

| Humanized antibody framework | Heavy chain | Heavy chain J-segment | Light chain | Light chain J-segment |
|---|---|---|---|---|
| FW1 | VH-1-46 | JH4 | VK1-L5 | JK2 |
| FW2 | VH-1-e | JH4 | VK1-A20 | JK2 |

### *Grafting of CDR regions and substitutions of FR amino acids

The essential amino acid sequences from the VH and VL of the mouse antibody IGF11-16 were transferred to the FRs of the template human antibodies above to thereby prepare humanized antibodies.

Specifically, the amino acid sequence of the VH sequence of the mouse antibody IGF11-16 was humanized by replacing the CDR amino acid sequences and several FR amino acids of the VH of the template human antibodies mentioned above with the corresponding amino acid sequences in the VH of the mouse antibody IGF 11-16, and a DNA sequence encoding these amino acids was also designed.

The amino acid sequence of the VL sequence of the mouse antibody IGF11-16 was humanized by replacing the CDR amino acid sequence and several FR amino acids of the VL of the template human antibody mentioned above with the corresponding amino acid sequence in the VL of the mouse antibody IGF 11-16, and a DNA sequence encoding these amino acids was also designed.

The constitutions of the designed heavy and light chains for humanized antibodies are shown in Table 2 below.

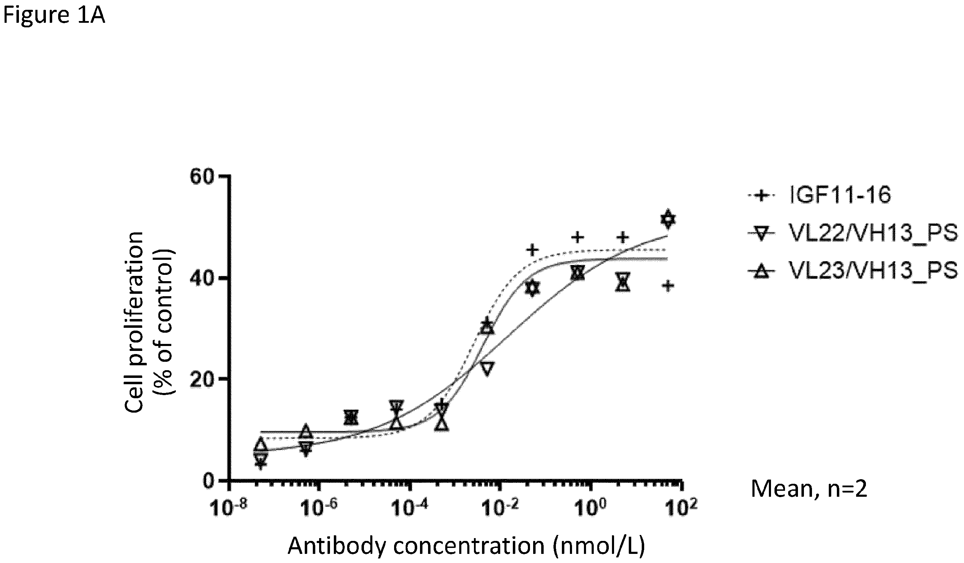

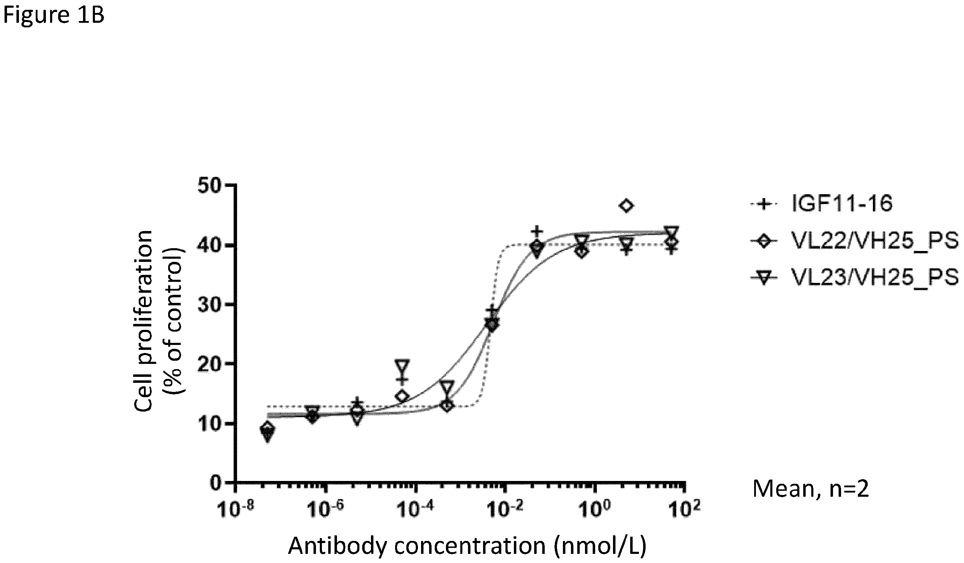

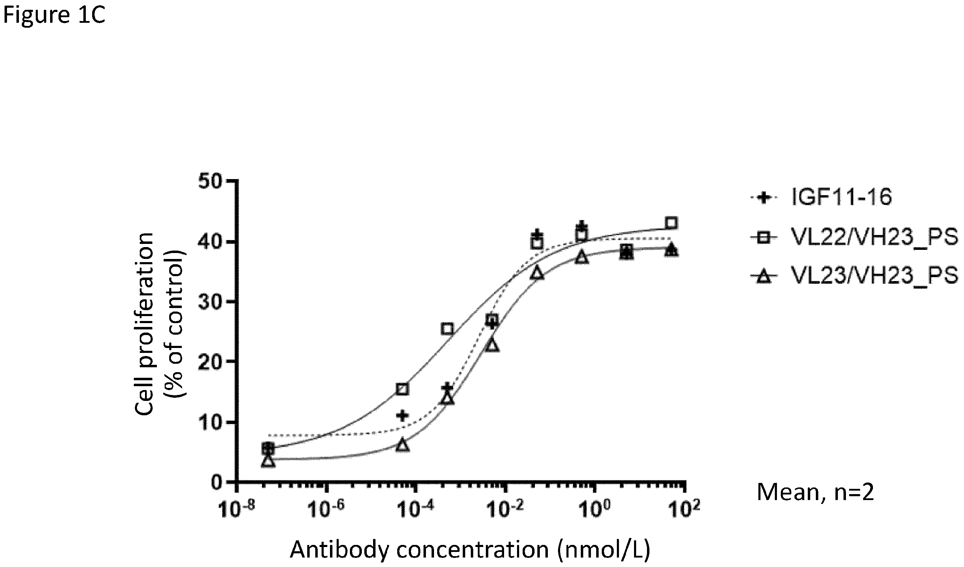

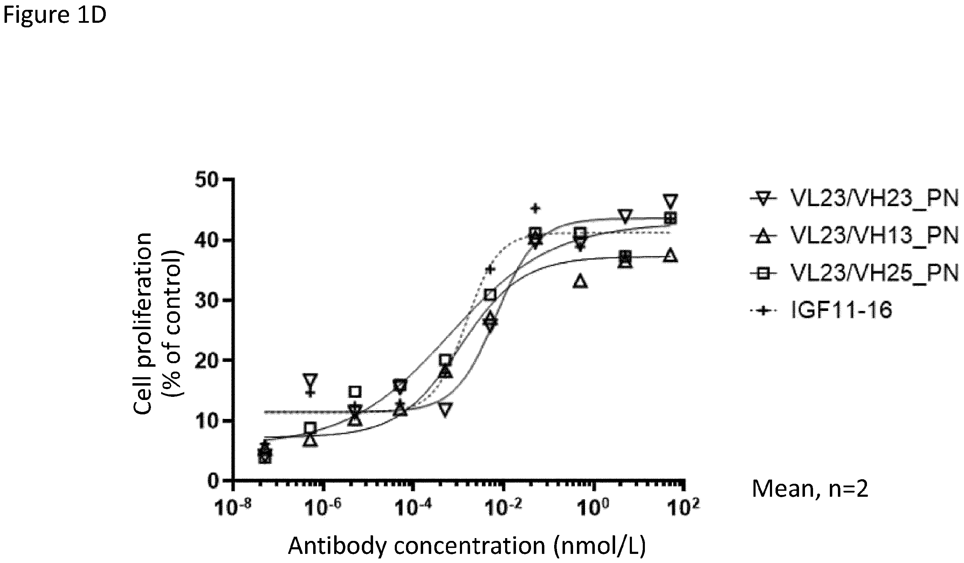

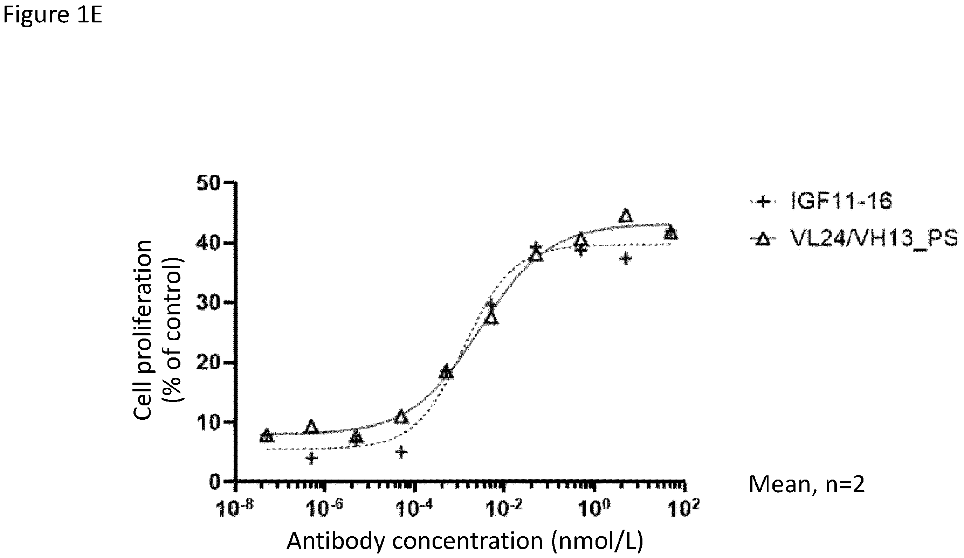

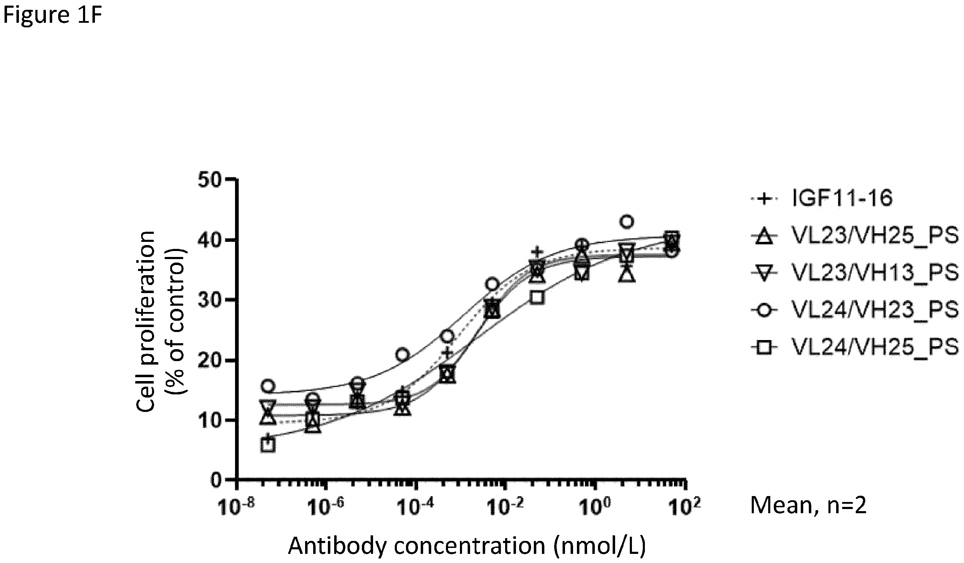

Incidentally, in the descriptions of the examples below and in the related figures, the name(s) of a humanized heavy chain variable region and/or a humanized light chain variable region designed herein may be used for referring to a humanized heavy chain composed of the designed humanized heavy chain variable region linked to a heavy chain constant region and/or a humanized light chain composed of the designed light chain variable region linked to a light chain constant region, as well as a complete humanized antibody by combining the humanized heavy chain and the humanized light chain. For example, "VL22/VH13_PS" in Figure 1A refers to a humanized antibody designed by combining a light chain composed of VL22 as the light chain variable region and a human kappa chain constant region linked thereto, and a heavy chain composed of VH13_PS as the heavy chain variable region and an IgG4S228P heavy chain constant region linked thereto. Examples of nucleotide sequences corresponding to the amino acid sequences of SEQ ID NOs: 15, 17, 19, 21, 23, 25, and 27 are shown in SEQ ID NOs: 16, 18, 20, 22, 24, 26, and 28, respectively.

### [Table 2]

**Table 2: Constitutions of the humanized antibodies as designed**

| | Light chain variable region | | | | Heavy chain variable region | | | | | |
|---|---|---|---|---|---|---|---|---|---|---|
| FW1 antibody name | FW1 light chain^{∗1} | Amino acid substitution | | | FW1 heavy chain^{∗2} | Amino acid substitution | | | | |
| | | FR2 | | | | CDR2 | | | | FR3 |
| | | Y36 | A43 | K45 | | N61 | E62 | K65 | S66 | V93 |
| FW1_var1 | FW1_VL1 (SEQ ID NO: 21) | C | I | K | FW1_VH1 (SEQ ID NO: 15) | A | Q | Q | G | V |
| FW1_var9 | FW1_VL3 (SEQ ID NO: 23) | Y | A | K | FW1_VH1 | A | Q | Q | G | V |
| FW1_var10 | FW1_VL3 | Y | A | K | FW1_VH2 (SEQ ID NO: 17) | A | Q | Q | G | T |
| FW1_var14 | FW1_VL4 (SEQ ID NO: 25) | Y | A | R | FW1_VH2 | A | Q | Q | G | T |
| FW2 antibody name | FW2 light chain^{∗3} | Amino acid substitution | | | FW2 heavy chain^{∗4} | Amino acid substitution | | | | |
| | | FR2 | | | | CDR2 | | | | FR3 |
| | | Y36 | V43 | K45 | | N61 | E62 | K65 | S66 | V93 |
| FW2_var2 | FW2_VL2 (SEQ ID NO: 27) | Y | I | K | FW2_VH1 (SEQ ID NO: 19) | A | Q | Q | G | V |

| | | | | | | | | | | |
|---|---|---|---|---|---|---|---|---|---|---|
| ^{∗}1: Amino acid substitutions introduced to FW1 light chains - Amino acid substitution to restore amino acids in the mouse parent antibody: Y36C, A43I - Amino acid substitutions to simulate the human germline sequence for reducing immunogenicity: K45R ^{∗}2: Amino acid substitutions introduced to FW1 heavy chains - Amino acid substitutions to simulate the human germline sequence for reducing immunogenicity: N61A, E62Q, K65Q, and S66G Amino acid substitutions to reduce immunogenicity: V93T ^{∗}3: Amino acid substitutions introduced to FW2 light chain - Amino acid substitution to restore amino acids in the mouse parent antibody: V43I ^{∗}4: Amino acid substitutions introduced to FW2 heavy chain - Amino acid substitutions to simulate the human germline sequence for reducing immunogenicity: N61A, E62Q, K65Q, and S66G | | | | | | | | | | |

### [Example 21 Preparation of humanized antibodies:

DNAs were synthesized which encode each of the designed heavy chain variable regions for humanized antibodies linked to a heavy chain constant region of the human IgG4S228P mutant, which is a stabilized mutant of the human IgG4 subclass. The synthesized DNAs were integrated and linked into a pcDNA3.4 expression vector to prepare plasmids expressing the humanized antibody heavy chains.

DNAs were also synthesized which encode each of the designed light chain variable regions for humanized antibodies linked to a x-chain constant region, and the synthesized DNAs were incorporated into a pcDNA3.4 expression vector to prepare plasmids expressing the humanized antibody light chains.

These plasmids expressing the humanized antibody heavy chains and the humanized antibody light chains were mixed and introduced into cells using the ExpiCHO^{®} Expression System (Thermo Fisher Scientific) for causing them to express various antibodies. In this connection, the humanized antibody expressed by a combination of the heavy chain expressing plasmid carrying FW1_VH1 and the light chain expressing plasmid carrying FW1_VL1 is referred to as the FW1_var1 antibody, and the humanized antibody expressed by combining the heavy chain expression plasmid carrying FW2_VH1 and the light chain expression plasmid carrying FW2_VL2 is referred to as the FW2_var2 antibody. The same procedure and nomenclature were used for FW1_var9, FW1_var10, and FW1_var14. Humanized antibodies were obtained from culture supernatant of cells transfected with the plasmids expressing the humanized antibody heavy chain and the humanized antibody light chain, via affinity purification using a Protein A column.

Subsequent preparation of humanized antibodies was also carried out according to the method described above.

### [Example 3] IGF-1 receptor activation effect using PathHunter^{®}:

In order to detect the effect of activating the IGF-1 receptor by the designed humanized antibodies on, the PathHunter^{®} IGFIR Functional Assay (DiscoverX) was used to detect the activation of IGF-1 receptor signaling by the following procedure.

Cells expressing the IGF-1 receptor were seeded in a poly-D-lysine-coated or collagen-I-coated 96-well plate (Black/clear or White/clear) at 90 µL/well (2×10⁴ cells/well or 5×10³ cells/well) and incubated at 37°C with 5% CO₂. The next day, 10 µL/well of each concentration of the drug was added and incubated at 37°C with 5% CO₂. On the following day, 30 µL of the culture supernatant was taken, 15 µL of substrate solution was added, and the reaction was allowed to continue for 60 minutes. The luminescence signal (RLU) was measured with a luminometer (Tristar, Berthold). The fluorescence intensity when 12.5 nM of the antibody was added was determined, from which the value with 0.1 nM of the antibody was subtracted as background, and the resulting value was used as the activity level. The activity level of the mouse parent antibody IGF11-16 was assumed as 1, and the relative value of the activity level of each humanized antibody was calculated

The results are shown in Table 3. These results indicate that the IGF-1 receptor activation ability of the humanized antibodies (FW1_var1, var9, var10, var14, and FW2_var2) was attenuated by more than 20% compared to the mouse parent antibody IGF11-16.

### [Table 3]

**Table 3: Measurement of IGF-1 receptor activation by humanized antibodies using PathHunter^{®} system**

| | Fluorescence intensity (12.5nM) (RLU) | Fluorescence intensity (0.1nM) (RLU) | Activation intensity (RLU) | Ratio |
|---|---|---|---|---|
| IGF11-16 | 2734 | 557 | 2177 | 1.00 |
| FW1_var1 | 2159 | 555 | 1604 | 0.74 |
| FW1_var9 | 2044 | 591 | 1454 | 0.67 |
| FW1_var10 | 1864 | 516 | 1348 | 0.62 |
| FW1_var14 | 1903 | 558 | 1345 | 0.62 |
| FW2_var2 | 1893 | 572 | 1321 | 0.61 |

Next, the humanized antibodies were modified at their CDR regions (antigen-binding regions), by replacing A61, Q62, Q65, and G66 in the heavy-chain CDR2 region, which are different from the corresponding residues of the mouse parent antibody, with N61, E62, K65, and S66, respectively, to make them identical to those of the mouse parent antibody. The resulting humanized antibodies with amino acid substitutions (FW1_var10_NEKS, FW1_var14_NEKS) were compared for their ability to activate the IGF-1 receptor by the same procedure as described above, using the mouse parent antibodies IGF11-16 and FW1_var1 as standard for comparison.

The results are shown in Table 4. These results indicate that no recovery of activity level was observed.

### [Table 4]

**Table 4: Measurement of IGF-1 receptor activation by mouse CDR-substituted humanized antibodies by PathHunter^{®} system**

| | Fluorescence intensity (12.5nM) (RLU) | Fluorescence intensity (0.1nM) (RLU) | Activation intensity (RLU) | Ratio |
|---|---|---|---|---|
| IGF11-16 | 3801 | 230 | 3572 | 1.00 |
| FW1_var1 | 2477 | 209 | 2269 | 0.64 |
| FW1_var10_NEKS | 2186 | 223 | 1963 | 0.55 |
| FW1_var14_NEKS | 1965 | 221 | 1744 | 0.49 |

These results indicated that even when the amino acid sequences of the CDRs were changed back to the same as the CDRs of the mouse parent antibody IGF11-16, its activity level was not restored to the same level as that of the mouse parent antibody IGF11-16 (activity level ratio within ±20%). Therefore, it was inferred that the FRs (framework regions), not the CDRs, were responsible for the decrease in the activity level.

Therefore, the humanized antibodies were modified by replacing their FR1, FR2, and FR3 with the corresponding FRs of the mouse antibody. The humanized antibodies modified via mouse FR substitutions are shown in Table 5. Activation of the IGF-1 receptor signaling by these modified antibodies was evaluated by the PathHunter^{®} system as described above. A human chimeric IGF11-16 antibody (Chimera), which is a chimera of the variable regions of the mouse parent antibody IGF 11-16 and the constant regions of human IgG4 (S228P), was used as a positive control, and the signal intensity at an antibody concentration of 16.7 nM was compared as described above. The results are shown in Table 5. Examples of nucleotide sequences corresponding to the amino acid sequences of SEQ ID NOs: 29, 31, 33, 35, and 37 are shown in SEQ ID NOs: 30, 32, 34, 36, and 38, respectively.

### [Table 5]

**Table 5: Humanized antibodies modified via mouse FR substitutions and their signal intensity ratio**

| Antibody name | Heavy chain variable region | Light chain variable region | Contents of substitutions | Signal intensity (ratio to IGF11-16) |
|---|---|---|---|---|
| Human chimeric IGF11-16 | IGF11-16_VH | IGF11-16 _VL | | 1.00 |
| FW1_var9_mFR-H1 | FW1_VH1_mH1 (SEQ ID NO: 29) | FW1_VL3 | Heavy chain FR-H1 was replaced with FR-H1 of IGF11-16 | 1.08 |
| FW1_var9_mFR-H2 | FW1_VH1_mH2 (SEQ ID NO: 31) | FW1_VL3 | Heavy chain FR-H2 was replaced with FR-H2 of IGF11-16 | 0.51 |
| FW1_var9_mFR-H3 | FW1_VH1_mH3 (SEQ ID NO: 33) | FW1_VL3 | Heavy chain FR-H3 was replaced with FR-H3 of IGF11-16 | 0.70 |
| FW1_var9_mFR-L1 | FW1_VH1 | FW1_VL3_mL1 (SEQ ID NO: 35) | Light chain FR-L1 was replaced with FR-L1 of IGF11-16 | 0.51 |
| FW 1_var9_mFR-L2+L3 | FW1_VH1 | FW1_VL3_mL2+L3 (SEQ ID NO: 37) | Light chain FR-L2 & L3 were replaced with FR-L2 & L3 of IGF11-16 | 0.50 |

These results indicate that the modified antibody whose signal intensity is equivalent (within ±20% of the value) to that of the human chimeric IGF11-16 antibody is FW1_var9_mFR-H1, suggesting that the mouse heavy chain FR1 is essential for maintaining the activity of the humanized antibodies.

The next step was to identify the amino acids essential for maintaining the activity in the mouse heavy chain FR1. Since there were seven amino acid differences in the heavy chain FR1 sequence betwen the mouse parent antibody IGF11-16 and the humanized antibodies, each of these amino acids was changed one at a time to the corresponding amino acid of the mouse parent antibody. The humanized antibodies modified via mouse FR1 amino acid substitutions are shown in Table 6. The signal intensity of IGF-1 receptor activation was measured for these humanized antibodies with mouse FR1 amino acid substitutions using the PathHunter^{®} system, and the signal intensity at an antibody concentration of 16.7 nM was compared with that of the mouse parent antibody IGF11-16. As a result, only the humanized antibody in which serine at position 25 was replaced with proline had an equivalent level of activity (activity ratio within ±20%) to that of the mouse parent antibody IGF11-16. The results are shown in Table 6.

### [Table 6]

**Table 6: Signal intensities of humanized antibodies modified via mouse FR1 amino acid substitutions**

| Antibody name | Heavy chain variable region | Heavy chain amino acid substitution relative to FW1_VH1 | Light chain variable region | Signal intensity ratio (compared to IGF11-16) |
|---|---|---|---|---|
| IGF11-16 | IGF11-16_VH | | IGF11-16_VL | 1.00 |
| FW1_var1 | FW1_VH1 | | FW1_VL1 | 0.66 |
| FW1_var9_hH1a | FW1_VH1_hH1a | FR-H1: V2I | FW1_VL3 | 0.70 |
| FW1_var9_hH2a | FW1_VH1_hH2a | FR-H1: V5Q | FW1_VL3 | 0.54 |
| FW1_var9_hH3a | FW1_VH1_hH3a | FR-H1: S7P | FW1_VL3 | 0.60 |
| FW1_var9_hH4a | FW1_VH1_hH4a | FR-H1: V11L | FW1_VL3 | 0.65 |
| FW 1_var9_hH5a | FW1_VH1_hH5a | FR-H1: K12V | FW1_VL3 | 0.56 |
| FW 1_var9_hH6a | FW1_VH1_hH6a | FR-H1: V20L | FW1_VL3 | 0.55 |
| FW 1_var9_hH7a | FW1_VH1_hH7a | FR-H1: S25P | FW1_VL3 | 1.09 |

These results indicate that the proline at position 25 of the heavy chain FR1 region is critical for maintaining the activity. Therefore, all subsequent humanized antibodies used in the examples below included P (proline, Pro) substitution at position 25 of the heavy chain.

### [Example 4] Identification of amino acids in the CDR region that are important for maintaining activity by alanine substitution:

In order to identify the amino acids in the CDR region required to maintain the activity, each amino acid in the mouse parent antibody IGF11-16 was replaced one at a time with alanine, and the resulting substituted antibodies were compared for their signal activation ability in terms of the EC₅₀ and Eₘₐₓ values, and also evaluated for their binding activity by antigen ELISA. The activity of each antibody was evaluated in comparison with that of the mouse parent antibody IGF 11-16, and antibodies having an EC₅₀ value within 2-fold and an Eₘₐₓ value within ±20% of that of the mouse parent antibody IGF 11-16 are determined to have a similar level of activity.

The ability to activate IGF-1 receptor signaling was evaluated in the PathHunter^{®} system described in Example 3. The EC₅₀ and Eₘₐₓ values were calculated using GraphPad Prism analysis software. The binding activity was measured by antigen ELISA using a recombinant IGF-1 receptor extracellular region as an antigen. Specifically, human recombinant IGF-1R (manufactured by R&D SYSTEMS) solution was prepared at 0.5 µg/mL in PBS (phosphate buffered saline). The prepared human recombinant IGF-1R solution was added to the solidified plate at 50 µL/well. The reaction was allowed to occur overnight at 4°C, the medium was replaced with 3% BSA/PBS (containing 0.02% sodium azide), and the solution was stored at 4°C until used for ELISA. The test substance solution (antibody solution at a concentration of 5 nM) was added to the solidified plate at 50 µL/well. The reaction was allowed to run for 1 hour at room temperature, and then washed twice with washing solution (PBST; phosphate buffered saline containing 0.05% Tween 20). An anti-mouse IgG antibody labelled with alkaline phosphatase (diluted 2000-fold in 3% BSA/PBS) was added at 50 µL/well. The reaction was allowed to run for 45 minutes at room temperature, washed three times with washing solution, and then the substrate (pNPP; para-nitrophenyl phosphate) was added to start the reaction. After 1 hour of reaction at room temperature, the absorbance was measured at 405 and 550 nm, and the difference between the absorbances at 405 nm and at 550 nm was calculated. This value was analyzed as the binding activity.

The CDR-substituted IGF11-16 antibodies produced and the results of signal activation and binding activity measurements are shown in Tables 7 and 8. These results indicate that five alanine substitutions made in the CDR region which reduced the binding activity by about 10 to 20%, i.e., tryptophan at position 32 of CDR-L1, tryptophan at position 33 of CDR-H1, glutamate at position 50 of CDR-H2, asparagine at position 52 of CDR-H2, and arginine at position 102 of CDR-H3, are crucial for maintaining the activity. In addition, histidine at position 35 of CDR-H1, serine at position 54 of CDR-H2, asparagine at position 55 of CDR-H2, serine at position 56 of CDR-H2, asparagine at position 59 of CDR-H2, and phenylalanine at position 64 of CDR-H2 are also deemed to contribute to the retention of the activity since their activity was decreased by Ala substitution.

On the other hand, among the 54 amino acid residues in the alanine-substituted CDR region, 44 residues showed a binding activity of 80% or more even after the alanine substitution.

### [Table 7]

**Table 7: Evaluation results of signal activation of alanine substitutions in the light chain CDR region**

| (Only the amino acid residue whose activity was reduced by the alanine substitution are shown.) | | | | | |
|---|---|---|---|---|---|
| | Alanine-substituted position | | Signal activation | | Binding |
| | | | EC₅₀ | Eₘₐₓ | @5nM |
| | Position | Amino acid | vs IGF11-16 | | |
| CDR-L1 | **32^{∗}** | **W** | **9.5** | **1.5** | **14** |

| | | | | | |
|---|---|---|---|---|---|
| *An amino acid residue whose binding activity is reduced to about 10-20% by alanine substitution. | | | | | |

### [Table 8]

**Table 8: Evaluation results of signal activation of alanine substitutions in the heavy chain CDR region**

| (Only the amino acid residues whose activity was reduced by the alanine substitution are shown.) | | | | | |
|---|---|---|---|---|---|
| | Alanine-sub stituted position | | Signal activation | | Binding |
| | | | EC₅₀ | Eₘₐₓ | EC₅₀ |
| | Position | Amino acid | vs IGF11-16 | | |
| CDR-H1 | **33**^{∗} | **W** | **0.5** | **0.0** | **9** |
| | 35 | H | 2.2 | 1.0 | 49 |
| CDR-H2 | **50**^{∗} | **E** | **ND** | **ND** | **9** |
| | **52**^{∗} | **N** | **2.7** | **0.0** | **21** |
| | 54 | S | 2.5 | 1.0 | 97 |
| | 55 | N | 3.0 | 1.0 | 94 |
| | 56 | S | 2.9 | 1.1 | 108 |
| | 59 | N | 2.3 | 0.9 | 112 |
| | 64 | F | 2.4 | 0.9 | 84 |
| CDR-H3 | **102*** | **R** | **54.4** | **4.8** | **9** |

| | | | | | |
|---|---|---|---|---|---|
| *Amino acid residues whose binding activity are reduced to about 10-20% by alanine substitution. | | | | | |

### [Example 5] Design of humanized heavy chain variable regions:

Since the results of Example 3 showed that the proline at position 25 of the heavy chain is important for maintaining the activity, humanized heavy chain variable regions having P at position 25 of the heavy chain were designed using FW1_VH1 and FW2_VH1 as basic frameworks. Since amino acid substitutions were examined using the FR1 of FW1_VH1 as the basic sequence, the FR1 region of FW2_VH1 was modified to be identical to FW1_FR1 by introducing S16A substitution. The introduction of amino acid substitutions for immunogenicity reduction was carried out based on the results of immunogenicity score analysis of Epibase^{®} (Lonza). A list of designed heavy chain variable regions is shown in Table 9 below.

### [Table 9]

**Table 9: Design of humanized heavy chain variable regions**

| Humanized heavy chain variable region | Amino acid substitution | SEQ ID NO: |
|---|---|---|
| FW1_VH1 | Basic framework | 15 |
| VH13_PN | S25P(FR-H1), V93T(FR-H3) | 43 |
| FW2_VH1 | Basic framework | 19 |
| VH23_PN | S16A(FR-H1), S25P(FR-H1) | 49 |
| VH25_PN | S16A(FR-H1), S25P(FR-H1), K74T(FR-H3) | 55 |

| | | |
|---|---|---|
| Note: V93T and K74T are substitutions intended to reduce immunogenicity. | | |

### [Example 6] Design of humanized light chain variable regions:

Humanized light chain variable regions were designed using FW1_VL1 and FW2_VL2 as basic frameworks. The introduction of amino acid substitutions to reduce the immunogenicity score was carried out based on the results of Epibase^{®} (Lonza) analysis. A list of designed light chain variable regions is shown in Table 10 below.

### [Table 10]

**Table 10: Design of humanized light chain variable regions**

| Humanized light chain variable region | Amino acid substitution | SEQ ID NO: |
|---|---|---|
| FW1_VL1 | Basic framework | 21 |
| VL13 | C36Y(FR-L2), I43A(FR-L2) | 61 |
| VL14 | C36Y(FR-L2), I43A(FR-L2), K45R(FR-L2) | 63 |
| FW2_VL2 | Basic framework | 27 |
| VL22 | Same as FW2_VL2 | 65 |
| VL23 | I43V(FR-L2) | 67 |
| VL24 | I43V(FR-L2), L54R(CDR-L2) | 69 |

| | | |
|---|---|---|
| Notes: C36Y, I43A, and I43V are amino acid substitutions back to the human framework sequence intended to reduce immunogenicity; K45R is a human germline sequence amino acid substitution intended to reduce immunogenicity; L54R is an amino acid substitution intended to reduce immunogenicity. | | |

### [Example 7] Design of humanized antibodies via substitution of amino acids at deamidation risk:

If deamidation occurs during the production of humanized antibodies, quality control will be difficult. It is therefore necessary to replace the amino acids at risk of deamidation with other amino acids that do not affect the activity in advance. Common sequences at risk for deamidation include NG, NT, NS, and NN. The NS sequence is present in the CDR-H2 region of the heavy chain of the present humanized antibodies. Hence, amino acid substitutions were made taking into account the risk of asparagine (N) at position 55 being deamidated and converted to aspartic acid (D). A list of substituted heavy chains is shown in Table 11. Examples of nucleotide sequences corresponding to the amino acid sequences of SEQ ID NOs: 45, 51, and 57 are shown in SEQ ID NOs: 46, 52, and 58, respectively.

### [Table 11]

**Table 11: Substitutions of amino acids at deamidation risk in humanized heavy chains**

| Humanized heavy chain variable region | Amino acid substitution | SEQ ID NO: |
|---|---|---|
| VH13_PN | Basic framework | 43 |
| VH13_PQ | N55Q(CDR-H2) | 45 |
| VH13_PS | N55S(CDR-H2) | 47 |
| VH23_PN | Basic framework | 49 |
| VH23_PQ | N55Q(CDR-H2) | 51 |
| VH23_PS | N55S(CDR-H2) | 53 |
| VH25_PN | Basic framework | 55 |
| VH25_PQ | N55Q(CDR-H2) | 57 |
| VH25_PS | N55S(CDR-H2) | 59 |

### [Example 8] Selection of humanized antibodies for activation of IGF-1 receptor signaling:

The humanized antibodies were evaluated based on their ability to activate the IGF-1 receptor, and humanized antibodies having activity equivalent to that of the mouse parent antibody IGF11-16 were selected.

In order to detect the activating effect of the anti-IGF-1 receptor agonist antibodies on the IGF-1 receptor, the activation of IGF-1 receptor signaling was measured using the PathHunter^{®} IGF1R Functional Assay (DiscoverX).

Cells expressing the IGF-1 receptor were seeded in poly-D-lysine-coated or collagen-I-coated 96-well plates (Black/clear or White/clear) at 90 µL/well (2×10⁴ cells/well or 5×10³ cells/well) and incubated at 37°C with 5% CO₂. The next day, 10 µL/well of each concentration of the drug was added and incubated at 37°C with 5% CO₂. The following day, 30 µL of culture supernatant was taken, 15 µL of substrate solution was added, and the reaction was allowed to run for 60 minutes, and the luminescence signal (RLU) was measured with a luminometer (Tristar, Berthold).

As a result of the measurement, the humanized antibodies whose activity was confirmed to be equivalent to that of the mouse parent antibody IGF11-16 (EC₅₀ value: within 2-fold and Eₘₐₓ value: within ±20% compared to the mouse parent antibody IGF11-16) are shown in Table 12.

### [Table 12]

**Table 12: List of humanized antibodies that have been confirmed to be as active as the mouse parent antibody IGF11-16 by the PathHunter^{®} system**

| | | Light chain | | | | |
|---|---|---|---|---|---|---|
| | | VL13 | VL14 | VL22 | VL23 | VL24 |
| Heavy chain | VH13_PS (SEQ ID NO: 47) | VL13/ VH13_PS | VL14/ VH13_PS | VL22/ VH13_PS | VL23/ VH13_PS | |
| | VH23_PS (SEQ ID NO: 53) | VL13/ VH23_PS | VL14/ VH23_PS | VL22/ VH23_PS | VL23/ VH23_PS | |
| | VH25_PS (SEQ ID NO: 59) | VL13/ VH25_PS | VL14/ VH25_PS | VL22/ VH25_PS | VL23/ VH25_PS | |
| | VH13_PN (SEQ ID NO: 43) | VL13/ VH13_PN | VL14/ VH13_PN | VL22/ VH13_PN | VL23/ VH13_PN | VL24/ VH13_PN |
| | VH23_PN (SEQ ID NO: 49) | VL13/ VH23_PN | VL14/ VH23_PN | VL22/ VH23_PN | VL23/ VH23_PN | VL24/ VH23_PN |
| | VH25_PN (SEQ ID NO: 55) | VL13/ VH25_PN | VL14/ VH25_PN | VL22/ VH25_PN | VL23/ VH25_PN | VL24/ VH25_PN |
| | VH13_PQ (SEQ ID NO: 45) | VL13/ VH13_PQ | VL14/ VH13_PQ | VL22/ VH13_PQ | VL23/ VH13_PQ | VL24/ VH13_PQ |
| | VH23_PQ (SEQ ID NO: 51) | VL13/ VH23_PQ | VL14/ VH23_PQ | VL22/ VH23_PQ | VL23/ VH23_PQ | VL24/ VH23_PQ |
| | VH25_PQ (SEQ ID NO: 57) | VL13/ VH25_PQ | VL14/ VH25_PQ | VL22/ VH25_PQ | VL23/ VH25_PQ | VL24/ VH25_PQ |

### [Example 9] Selection of humanized antibodies by their human myoblast proliferative activity:

The humanized antibodies were evaluated based on their human myoblast proliferative activity, whereby humanized antibodies with activity equivalent to that of the mouse parent antibody IGF11-16 were selected.

In order to examine the proliferative activity of the anti-IGF-1 receptor humanized antibodies against human myoblasts, the drug was added to human myoblasts, and the amount of ATP in the cells was measured after 4 days.

Normal human skeletal muscle myoblast cells (HSMM, Lonza) were seeded in 96-well plates (Collagen type I coated) using medium containing 1% BSA in SkBM-2 (Lonza, CC-3246) at 0.1 mL/well (2×10³ cells/well), and incubated at 37°C with 5% CO₂. The day after cell seeding, various drugs were added at 25 µL/well and incubated for 4 days at 37°C with 5% CO₂. As an indicator of cell proliferation, the amount of ATP in the cells was measured using the CellTiter-Glo (registered trademark) Luminescent Cell Viability Assay (Promega). After incubated for 4 days, the supernatant was removed from each well so that the culture medium was 50 µL/well, and the 96-well plate was allowed to stand at room temperature for at least 30 minutes. 50 µL/well of CellTiter-Glo (registered trademark) reagent was added and allowed to react for at least 10 minutes before measuring the luminescence signal with a luminometer (Tristar, Berthold).

As a result, the humanized antibodies whose activity was confirmed to be equivalent to that of the mouse parent antibody IGF11-16 (EC₅₀ value: within 10-fold, Eₘₐₓ: 90% or more compared to the mouse parent antibody IGF11-16) are shown in Table 13. The graphs of the measurement results are shown in Figures 1A to 1F.

### [Table 13]

**Table 13: List of humanized antibodies that have been confirmed to be as active as the mouse parent antibody IGF11-16by myoblast proliferation assay**

| | | Light chain | | |
|---|---|---|---|---|
| | | VL22 | VL23 | VL24 |
| Heavy chain | VH13_PS | VL22/ VH13_PS | VL23/ VH13_PS | VL24/ VH13_PS |
| | VH23_PS | VL22/ VH23_PS | VL23/ VH23_PS | VL24/ VH23_PS |
| | VH25_PS | VL22/ VH25_PS | VL23/ VH25_PS | VL24/ VH25_PS |
| | VH13_PN | | VL23/ VH13_PN | |
| | VH23_PN | | VL23/ VH23_PN | |
| | VH25_PN | | VL23/ VH25_PN | |

### [Example 10] Evaluation of immunogenicity:

To analyze the immunogenicity of the humanized antibodies, Lonza's Epibase^{®} in Silico was used to calculate immunogenicity scores. Lonza's Epibase^{®} in Silico platform is an immunogenicity prediction method that utilizes the structural characteristics of the HLA class II receptor as well as the experimentally determined binding affinity between a 10-mer peptide and an HLA class II receptor to predict the potential peptide/HLA binding, which is a necessary condition for T cell activation, in an amino acid sequence contained in the antibody, and to calculate it as an immunogenicity score. Evaluation in 85 HLA class II allotypes (43 types of DRB1, 8 types of DRB3/4/5, 22 types of DQ, and 12 types of DP) can cover more than 99% of the entire population. Immunogenicity scores were determined by taking into account the frequency of occurrence as well as the binding affinity of the allotypes.

The results are shown in Tables 14 and 15. Compared to the immunogenicity scores of the mouse parent antibody IGF11-16 and the mouse-human chimeric antibody (an antibody with the variable region of the mouse parent antibody IGF11-16 and the constant region of human IgG4 (S228P)), which were evaluated in a similar manner, the immunogenicity of the humanized antibodies was found to be lower.

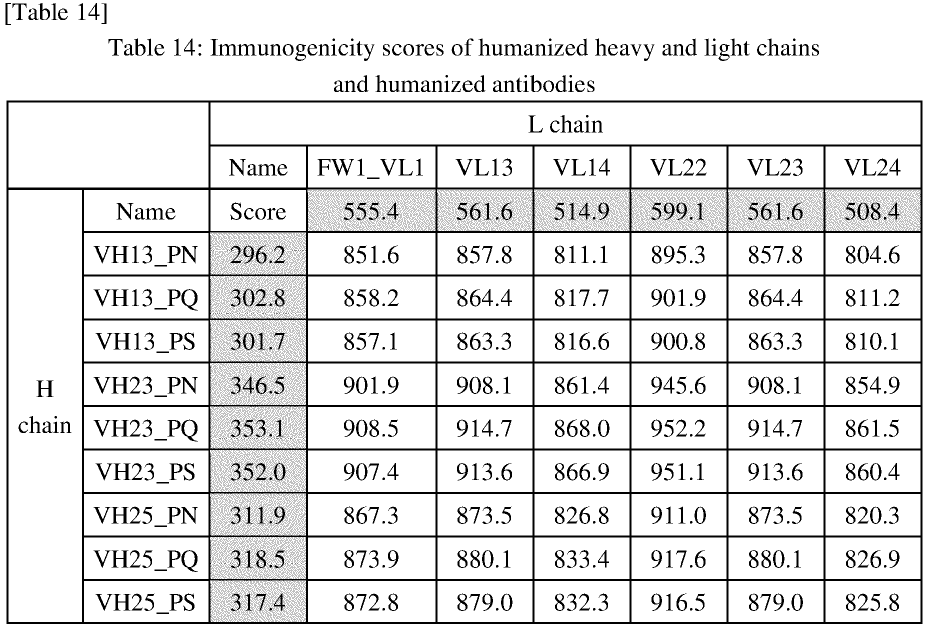

### [Table 15]

**Table 15: Comparison of immunogenicity scores between humanized antibodies and mouse and chimeric antibodies**

| Name | Description | Immunogenicity score | | |
|---|---|---|---|---|
| | | Heavy chain | Light chain | Complete antibody |
| Mouse parent antibody IGF11-16 | Mouse IgG1 antibody | 2319.4 | 1089.1 | 3408.5 |
| Chimeric antibody | Variable region: mouse IGF11-16 Constant region: human IgG4 (S228P) | 791.1 | 665.4 | 1456.5 |
| Humanized antibody hIGF13_PS | Variable region: VL23/VH13_PS Constant region: human IgG4(S228P) | 301.7 | 561.6 | 863.3 |
| Humanized antibody hIGF25 _PS | Variable region: VL23/VH25_PS Constant region: human IgG4 (S228P) | 317.4 | 561.6 | 879.0 |

### [Example 11] Evaluation of binding activity to mammalian IGF-1 receptors:

In order to investigate the binding activity of the anti-IGF-1 receptor agonist antibodies against the IGF-1 receptors of human (SEQ ID NO: 71), crab-eating macaque (SEQ ID NO: 73), rabbit (SEQ ID NO: 75), guinea pig (SEQ ID NO: 77), rat (SEQ ID NO: 79) and mouse (SEQ ID NO: 81), a cell-based ELISA was performed using cells expressing various IGF-1 receptors.

HEK293T cells were transfected by lipofection method with pEF1 expression vectors (Thermo Fisher) incorporated with the IGF-1 receptor genes of rabbit (SEQ ID NO: 76), guinea pig (SEQ ID NO: 78), rat (SEQ ID NO: 80) and mouse SEQ ID NO: 82). The transfected HEK293T cells were allowed to grow overnight or longer after the lipofection, and were then added to a 96-well plate (poly-D-lysine coated) at 4 × 10⁴ cells/well. The cells were then fixed in 10% buffered formalin (Mildform^{®} 10NM, Wako) and blocked with phosphate buffer containing 3% BSA before used for ELISA.

ELISA was carried out as follows. 100 µL of each humanized antibody solution prepared at 5 nM in 1% BSA/1% FBS/PBS was added to each well, and reacted at 37°C for about 1 hour. Anti-human IgG antibody HRP conjugate solution prepared at each concentration in 1% BSA/1% FBS/PBS was added to each well at 100 µL, reacted at 37°C for about 1 hour, and washed three times with washing solution. The reaction was initiated by adding 100 µL of substrate (TMB) to each well. After about 30 minutes, 100 µL of 1M sulfuric acid was added to each well, the absorbances at 450 and 650 nm were measured, and the difference between the absorbances at 450 nm and at 650 nm was calculated. The calculated difference was compared with the difference between the absorbances at 450 nm and at 650 nm for HEK293T cells without IGF-1 receptor gene (Mock) to analyze the binding activity.

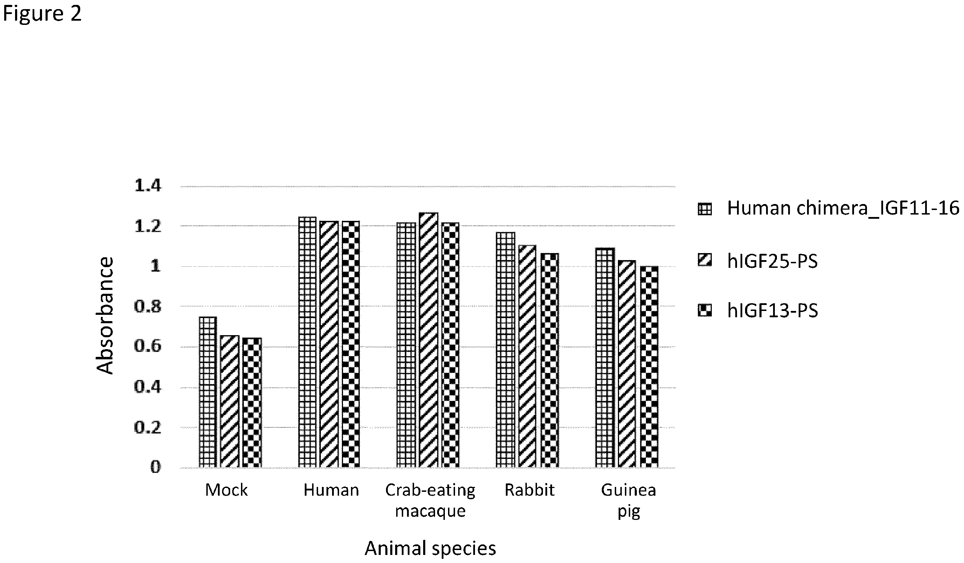

Figure 2 shows the results of reactivity to the IGF-R of each of the human, guinea pig, crab-eating macaque, and rabbit. As a result, the humanized antibodies hIGF13_PS and hIGF25_PS increased the binding activity of human, guinea pig, crab-eating macaque and rabbit IGF-1 receptor-expressing cells by about 2-fold compared to Mock cells, and the reactivity was comparable to that of the human mouse chimeric antibody IGF11 -16. On the other hand, the binding activity to cells expressing rat and mouse IGF-1 receptors was comparable to that of Mock cells. These results indicate that the humanized antibodies hIGF13_PS and hIGF25_PS bind to human, guinea pig, crab-eating macaque, and rabbit IGF-1 receptors, but not to rat and mouse IGF-1 receptors.

### [Example 12] Binding affinity to IGF-1 receptor by surface plasmon resonance:

In order to examine the binding properties (binding and dissociation rates) of the drug to the IGF-1 receptor, the binding was measured by surface plasmon resonance (SPR) method.

The BIACORE T200 system was used as the measurement system. Antihistidine-tagged monoclonal antibodies was fixed in all flow cells of a sensor chip CM3 (BR-1005-36, GE) with Amine Coupling Kit (BR-1000-50, GE) and His Capture Kit (28-9950-56, GE) at approximately 3000 RU before use. HBS-EP+ (BR-1006-69, GE) was used as the running buffer. A recombinant human IGF-1 receptor histidine tag (305-GR-050, R&D SYSTEMS, hereafter IGF-1R-His) was captured in the measurement and used as a ligand. Each concentration of the drug was used as an analyte. A flow cell without IGF-1R-His capture was used as a ligand negative control. PBS (PBS pH 7.4 (1x), #10010049, Gibco) was used as a drug negative control.

The measurement temperature of the measurement system was set at 40°C. The anti-histidine-tagged monoclonal antibodies in the flow cells (2 and 4) were reacted with IGF-1R-His (<2×10-8M) at less than 100 RU. The flow rate was set at 30 µL/min, 10 nM of purified mouse IgG2a, kappa, isotype Ctrl, Clone: MG2a-53 (401502, BioLegend, hereafter ctrl IgG2a) was reacted for 1 min, and HBS-EP+ was passed for at least 10 min. The analyte was diluted in steps (0.5 to 8×10⁻¹⁰M) with HBS-EP+ and reacted in all flow cells.

As measurement conditions, the single cycle kinetics method was used. Each concentration of the analyte was reacted for 600 seconds to obtain a binding curve, and then HBS-EP+ was reacted for 1200 seconds to obtain a dissociation curve. After the reaction, regeneration buffer 1 (0.2% SDS), regeneration buffer 2 (100 mM Tris-HCl (pH 8.5), 1 M NaCl, 15 mM MgCh), and regeneration buffer 3 (10 mM glycine-HCl (pH 1.5)) were reacted for 1 minute each for removing IGF-1R-His from the measurement system and washing the measurement system. The dissociation rate constant (ka, 1/Ms), binding rate constant (kd, 1/s), and dissociation constant (KD, M) were calculated by using Biacore T200 Evaluation software (ver. 2.0) with 1: 1 Binding model. The results are shown in Table 16.

### [Table 16]

**Table 16: Binding affinity of the humanized antibodies and the mouse parent antibody IGF11-16**

| Ligand | Analyte | Ka (1/Ms) | Kd (1/s) | KD (M) |
|---|---|---|---|---|
| IGF-1 receptor | IGF11-16 | 2.67E+06 | 7.72E-05 | 3.14E-11 |
| IGF-1 receptor | hIGF13_PS | 2.85E+06 | 1.58E-04 | 5.78E-11 |
| IGF-1 receptor | hIGF25_PS | 2.87E+06 | 1.35E-04 | 5.10E-11 |

The KD values of hIGF13_PS and hIGF25_PS against the human IGF-1 receptor were found to be less than E-10, meeting the most favorable criterion for anti-IGF-1 receptor agonist humanized antibodies.

### [Example 13] In vivo hypoglycemic effect (hypoglycemic effect in guinea pigs):

In order to confirm whether the anti-IGF-1 receptor agonist antibodies have a hypoglycemic effect in vivo, a single dose of hIGF13_PS or hIGF25_PS was administered to guinea pigs, and the blood glucose levels were measured over time for determining the presence or absence of a hypoglycemic effect. The hypoglycemic effect used herein refers to the effect of lowering the blood glucose level to 50 mg/dL or less or causing hypoglycemic symptoms.

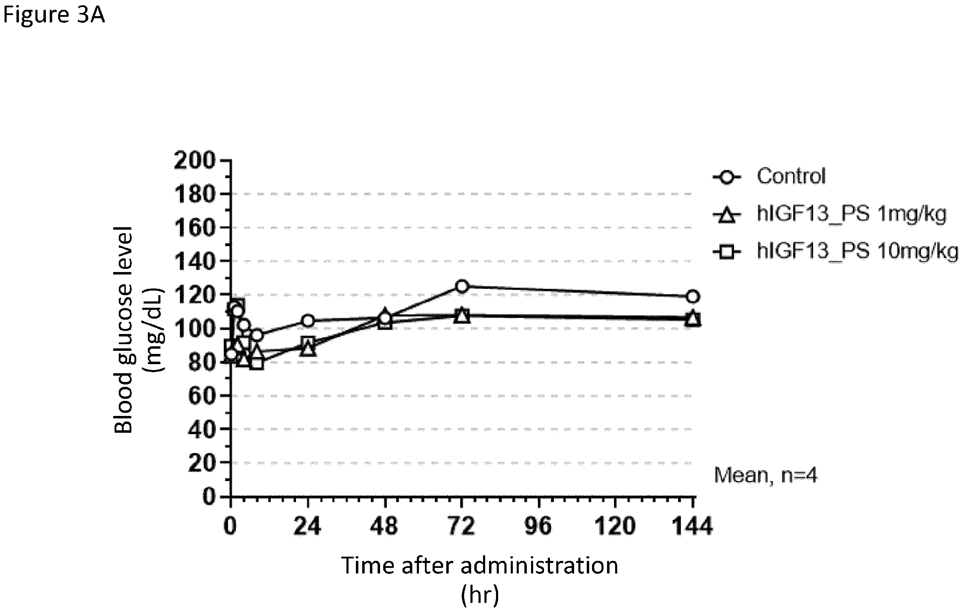

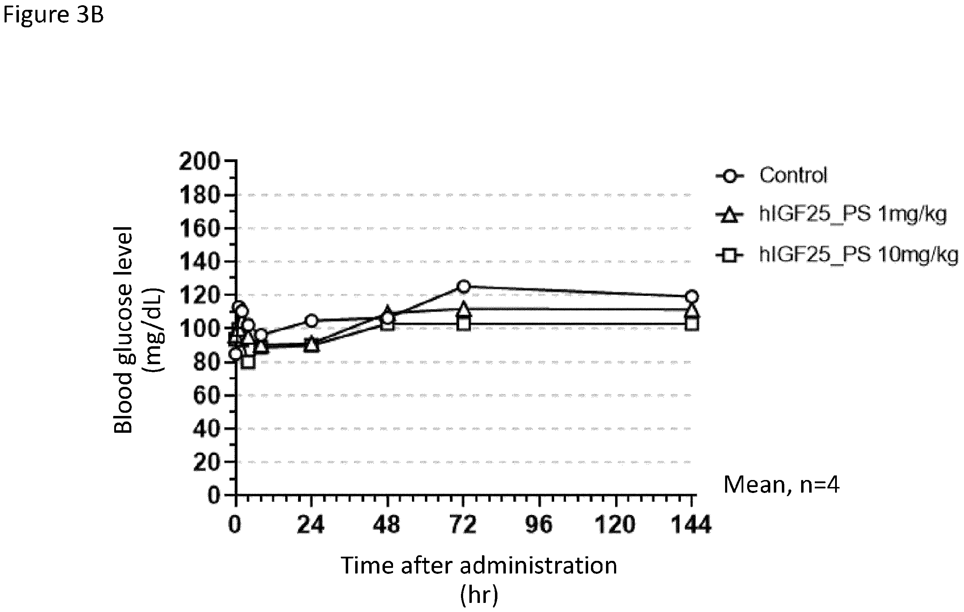

The guinea pigs were fasted for 12 hours, and each of the humanized antibodies hIGF13_PS and hIGF25_PS was administered intravenously as a single dose at 10 mg/kg. Guinea pigs were fasted until 24 hours after administration. Blood samples were taken from the guinea pigs awake before (0 h), 1, 2, 4, 8, 24, 48, 72, and 144 h after the administration, and their blood glucose levels were measured using a Glutest sensor (Sanwa Kagaku Kenkyusho). The results are shown in Figures 3A and B.

Neither of the humanized antibodies showed any significant difference in the blood glucose levels compared to the solvent control group, which received only solvent, and the blood glucose levels after administration were all above 50 mg/dL. This indicates that each humanized antibody does not have a significant hypoglycemic effect like IGF-1, and does not affect blood glucose levels, indicating its potential as a drug to overcome hypoglycemia, which is a side effect of IGF-1.

### [Example 14] Hemodynamics of the humanized antibodies in guinea pigs:

Guinea pigs were fasted for 12 hours, and each of the humanized antibodies hIGF13_PS and hIGF25_PS or IGF11-16 (mouse parent antibody) was administered intravenously in a single doses at 1 or 10 mg/kg. Guinea pigs were fasted until 24 hours after the administration, at which time they were re-fed. Blood samples were taken from the guinea pigs awake before (0 h), 2, 4, 8, 24, 48, 72, 96, 120, and 144 h after the administration, and the concentration of the humanized antibody in plasma was measured by ELISA.

Specifically, recombinant IGF-1R (manufactured by R&D SYSTEMS) was used, and the measurement was made by antigen ELISA. A calibration curve for quantification of each antibody administered to guinea pigs was prepared by diluting the antibody of a known concentration serially with guinea pig plasma to form a series of standards. Both the standards and the plasma samples were diluted 10 to 1000 times to perform the measurement.

A PBS solution of 0.5 µg/mL of the recombinant IGF-1R was added to a 96-well plate (MaxiSorp (NUNC)) and fixed at 4°C overnight. Further blocking with 3% BSA/PBS was performed to prepare a recombinant-IGF-1R fixed plate. On the other hand, plasma from a guinea pig to which no antibody was administered was used for diluting each administered antibody serially to prepare a series of standards. Each of the plasma samples and the standards was diluted 10-fold and added to the recombinant-IGF-1R fixed plate at 50 µL/well. The reaction was carried out for 1 hour and 30 minutes at room temperature, followed by washing operation with PBS-T (PBS, 0.025% Tween 20). Subsequently, a solution of alkaline phosphatase-conjugated anti-human IgG (H+L) polyclonal antibody (Southern Biotechnology Associates, Cat. #2087-04) diluted 2000-fold with 3% BSA/PBS was added at 50 µL/well. The cells were reacted for 1 hour at room temperature, after which washing was performed with PBS-T, and 100 µL/well of pNpp (Wako, Cat# 149-02342) was added as a chromogenic substrate, and incubated for 1 hour at room temperature. After that, the absorbance was measured at 405 nm and at 550 nm using a plate reader, and the difference between the absorbances at 405 nm and at 550 nm was determined. A calibration curve was drawn over the concentration range of the antibody using the series of standards, and the antibody concentration in each plasma sample was calculated.

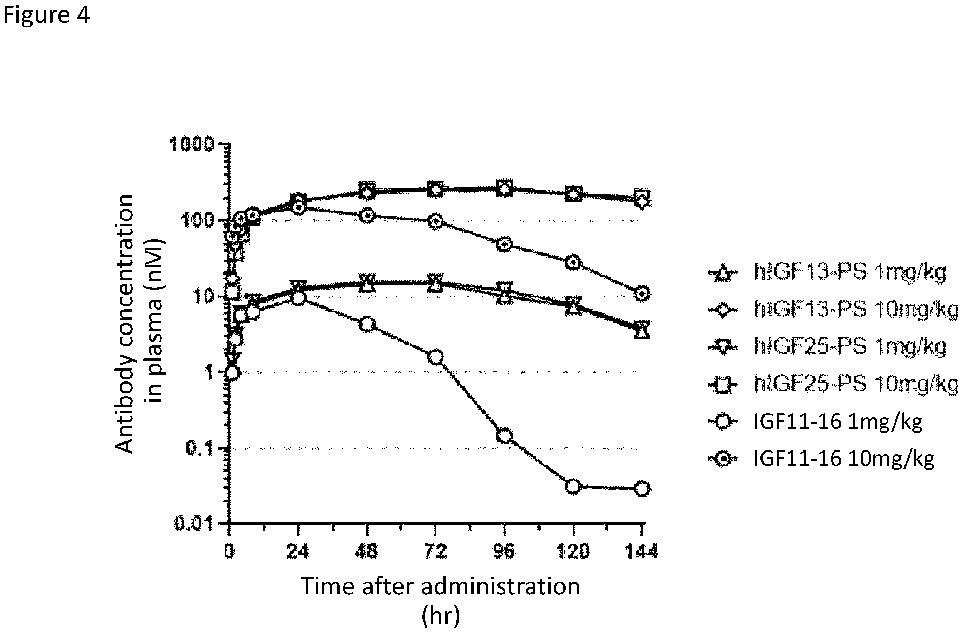

The results are shown in Figure 4. The plasma concentration of each humanized antibody increased in a dose-dependent manner, and even in the low-dose group, the plasma concentration of the humanized antibody was maintained until 144 hours after the administration at more than 50% of that at 24 hours after the administration. These results indicate that the hemodynamics of the humanized antibodies were more persistent than that of the mouse parent antibody IGF11-16.

### [Example 15] Effect of increasing muscle mass in normal guinea pigs by the humanized antibodies:

A single intravenous dose of hIGF13 _PS was administered to normal guinea pigs, and muscle mass was measured after 2 weeks, and compared with the muscle mass-increasing effect of continuous administration of IGF-1 and intravenous administration of the mouse parent antibody IGF11-16.

Either hIGF13_PS or the mouse parent antibody IGF11-16 was administered as a single dose at 0.1 mg/kg intravenously to normal guinea pigs. As a positive control, human IGF-1 (mecasermin) was implanted subcutaneously using an osmotic pump (Alzette) and administered continuously at 1 mg/kg/day. As a control, only solvent was administered intravenously. Two weeks after the drug administration, each guinea pigs was anesthetized and bled to death, the extensor digitorum longus muscle was removed, and the muscle mass was measured.

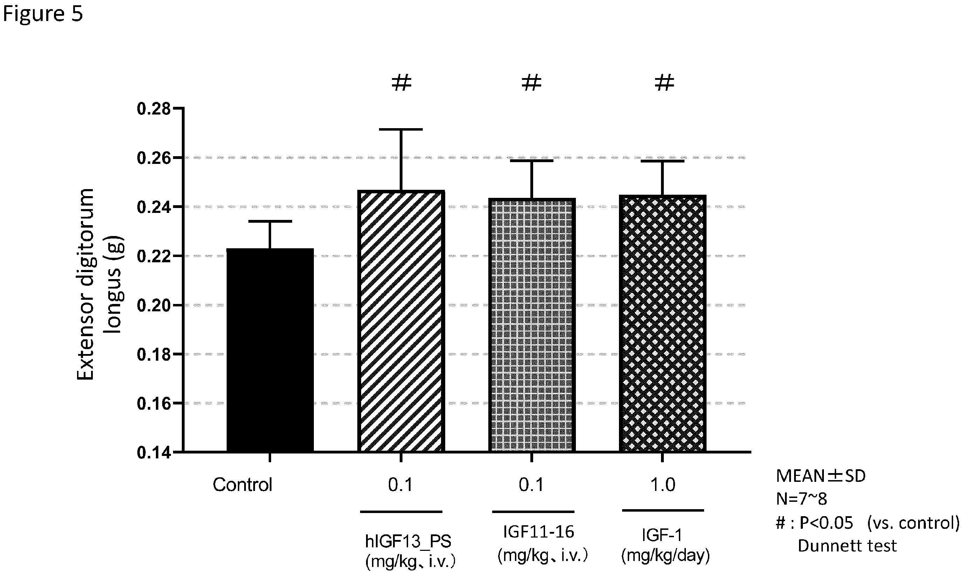

The results are shown in Fig. 5. The group to which hIGF13_PS was administered intravenously at 0.1 mg/kg significantly increased the muscle mass compared to the control group treated only with solvent. The drug effect was comparable in intensity to that of the group treated with continuous administration of human IGF-1 at 1 mg/kg/day and that of the group treated with intravenous administration of the mouse parent antibody IGF11-16.

These results indicate that a single dose of hIGF13_PS can be expected to have a drug effect equivalent to that of 2-week continuous administration of human IGF-1.

### [Example 16] Elongation effect of growth plate cartilage in hypophysectomized guinea pigs by the humanized antibodies:

In order to evaluate the proliferation effect of growth plate cartilage by hIGF13_PS, the epiphyseal line thickness of the proximal tibia was evaluated using a guinea pig hypophysectomized (HPX) model. The guinea pig hypophysectomized (HPX) model is in a low IGF-1 state since the production of growth hormone is suppressed due to removal of the pituitary gland.

A single subcutaneous dose of hIGF13_PS was administered at 0.3 mg/kg or 1.0 mg/kg to hypophysectomized guinea pigs, and the right lower limbs were collected 2 weeks later. Tissue specimens of the growth plate cartilage were prepared from the proximal part of the tibia, and the thickness of the growth plate cartilage (epiphyseal thickness) was measured with toluidine blue. As a positive control, IGF-1 (mecasermin) preparation was continuously administered subcutaneously at 1 mg/kg/day using osmotic pump, and GH (somatropin) preparation was subcutaneously administered once a day at 1 mg/kg/day.

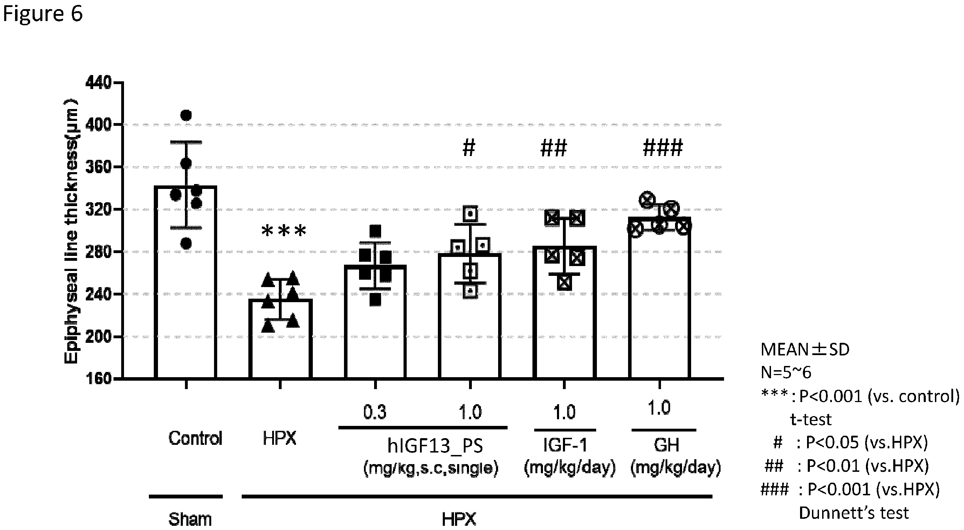

The results are shown in Fig. 6. These results indicate that an increase in the epiphyseal thickness was observed in each of the IGF-1 and GH groups, which was caused presumably since the blood IGF-1 level reduced due to HPX was supplemented, or since GH lost due to HPX was supplemented. The hIGF13_PS antibody group was shown to increase the epiphyseal thickness in a dose-dependent manner without increasing the blood IGF-1 levels in hypophysectomized (HPX) individuals.

These results indicate that the hIGF13_PS antibody is able to restore the occlusion of the epiphyseal line caused by the decrease in the IGF-1 concentration due to hypophysectomizing (HPX) treatment via activation of the IGF-1R-mediated signaling.

### [Example 17] Hypoglycemic effect in crab-eating macaques by the humanized antibodies:

In order to confirm whether the anti-IGF-1 receptor agonist antibodies have a hypoglycemic effect on crab-eating macaques, a single dose of hIGF13_PS was administered to crab-eating macaques, the blood glucose levels were measured in succession, and the hypoglycemic effect was compared with that of a single dose of IGF-1 (1 mg/kg). The hypoglycemic effect herein refers to the effect of lowering the blood glucose level to less than 50% compared to the solvent group or the effect of causing hypoglycemic symptoms.

Each humanized antibody was administered to crab-eating macaques at 10 mg/kg as a single intravenous or subcutaneous dose. Blood samples were taken before (0 hour), 5 and 30 minutes, and 1, 2, 4, 8, and 24 hours after the administration, and the blood glucose levels were measured using a Medisafe Fit (Terumo Corporation).

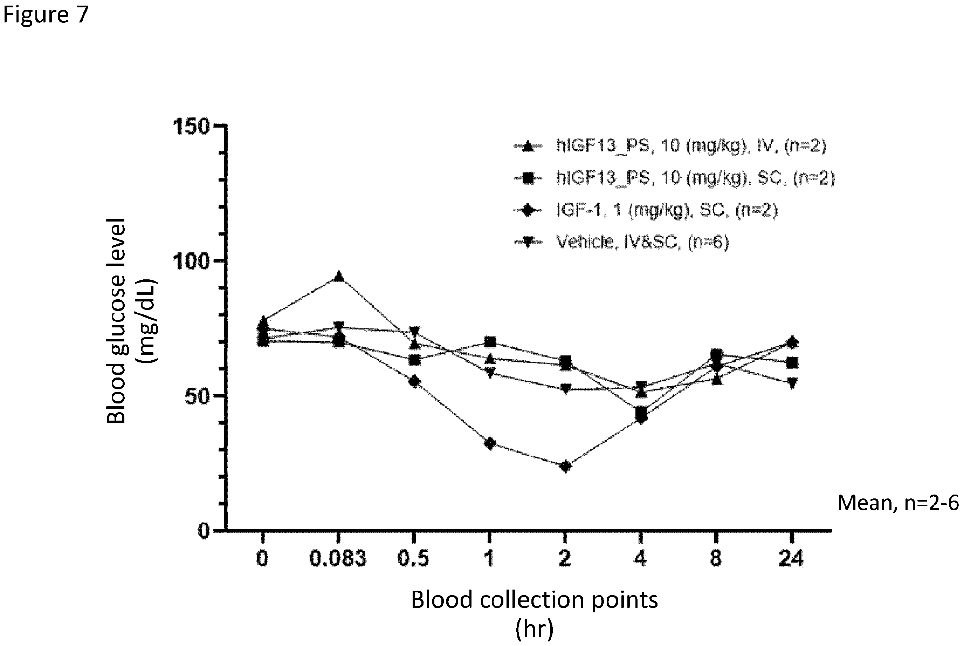

The results are shown in Figure 7. Each humanized antibody showed no difference in the blood glucose levels compared to the solvent control group, which received only solvent, and all blood glucose levels after administration were at the same level as those of the solvent control group. On the other hand, the IGF-1 group became hypoglycemic after 2 hours and showed hypoglycemic symptoms, so glucose was administered to recover.

### [Example 18] Blood kinetics of the humanized antibodies in crab-eating monkeys:

The humanized antibody hIGF13_PS was administered intravenously or subcutaneously as a single dose to crab-eating macaques at 1 or 10 mg/kg. Blood samples were collected before (0 hours), 2, 4, 8, 24, 48, 72, and 144 hours after the administration, and the concentration of humanized antibody in plasma was measured by ELISA.

Specifically, the measurement was performed by antigen ELISA using recombinant IGF-1R (305-GR-050, R&D SYSTEMS). A calibration curve of each antibody administered to macaques was prepared by diluting a known concentration of the antibody stepwise to prepare a series of standard samples. Each of the standards and the plasma samples was diluted 10- to 1000-fold for measurement.

A 0.5 µg/mL solution of the recombinant IGF-1R in PBS was added to a 96-well plate (MaxiSorp (NUNC)) and fixed at 4°C overnight. Further blocking with 3% BSA/PBS was performed to prepare a recombinant-IGF-1R fixed plate. Plasma taken from a macaque to which no antibody was administered was used for diluting the antibody stepwise to prepare a series of standard samples. Each of the plasma samples and the standard samples was diluted 10-fold and added to the recombinant-IGF-1R fixed plate at 50 µL/well. The reaction was carried out for 1 hour and 30 minutes at room temperature, followed by washing operation with PBS-T (PBS, 0.025% Tween 20). Subsequently, a solution of alkaline phosphatase-conjugated anti-human IgG (H+L) polyclonal antibody (Southern Biotechnology Associates, Cat. #2087-04) diluted 2000-fold in 3% BSA/PBS was added at 50 µL/well, and the reaction was allowed to run for 1 hour at room temperature. Subsequently, washing operation was performed with PBS-T, pNpp (Wako, Cat# 149-02342) was added as a chromogenic substrate at 100 µL/well, followed by incubation for 1 hour at room temperature. After that, the absorbance was measured at 405 nm and 550 nm with a plate reader, and the difference between the absorbances at 405 nm and at 550 nm was calculated. A calibration curve was drawn over the concentration range of the antibody using the series of standards, and the antibody concentration in each plasma sample was calculated.

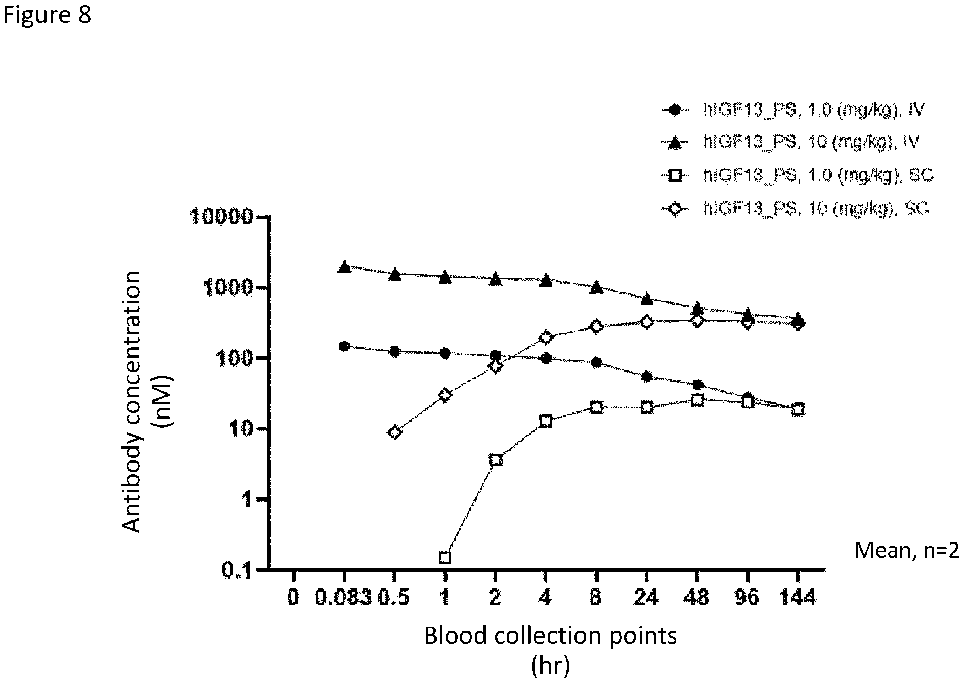

The results are shown in Figure 8. These results indicate that hIGF13_PS has excellent blood kinetics in crab-eating macaques.

### [Example 19] Effect of increasing the muscle mass in crab-eating macaques by the humanized antibodies:

Two crab-eating macaques received intravenous administration of mg/kg of hIGF13_PS. The muscle mass was measured by DXA (Dual Energy X-ray Absorptiometry) before and 3-4 weeks after the administration.

Specifically, the macaques were subjected to general anesthesia by intramuscular administration (buttocks) of ketamine hydrochloride (Arevipharma GmbH, 50 mg/mL, 0.2 mL/kg) and medetomidine hydrochloride solution (Domitor, Orion Corporation, 1 mg/mL, 0.08 mL/kg). A dual-energy X-ray absorptiometry system (Discovery-A, HOLOGIC) was used to measure the fat mass (g), lean body mass (Lean) (g), and bone mineral content (BMC) (g), and the lean body mass was analyzed as the muscle mass. BMC (bone mineral content) and Lean+BMC (g) of the right and left arms (upper limbs) were measured, and the muscle mass (g) was calculated and compared to that before administration.

As a result, both animals showed an increase in the muscle mass compared to the measurement before administration, and the muscle gain rate of the upper limbs was 7.4% and 10.9%, respectively, compared to the values before administration. These results confirm the muscle gaining effect of hIGF13_PS.

In addition, hIGF13_PS was subcutaneously administered to two crab-eating macaques at 10 mg/kg. Muscle mass was measured by DXA (Dual Energy X-ray Absorptiometry) before and 3 to 4 weeks after the administration.

Specifically, the macaques were subjected to general anesthesia by intramuscular administration (buttocks) of ketamine hydrochloride (Arevipharma GmbH, 50 mg/mL, 0.2 mL/kg) and medetomidine hydrochloride solution (Domitor, Orion Corporation, 1 mg/mL, 0.08 mL/kg). A dual-energy X-ray absorptiometry system (Discovery-A, HOLOGIC) was used to measure the fat mass (g), lean body mass (Lean) (g), and bone mineral content (BMC) (g), and the lean body mass was analyzed as the muscle mass. BMC (bone mineral content) and Lean+BMC (g) of the right and left lower limbs were measured, and the muscle mass (g) was calculated and compared to that before administration.

As a result, both animals showed an increase in the muscle mass compared to the measurement before administration, and the muscle gain rate of the lower limbs was 3.3% and 12.7%, respectively, compared to the values before administration. These results confirm the muscle gaining effect of hIGF13_PS.

### [Example 20] Effect of IGF11-16 on HepG2 cell proliferation:

The concentration-dependent effect of the mouse parent antibody IGF 11-16 on HepG2 cell proliferation was evaluated by cell survival assay.

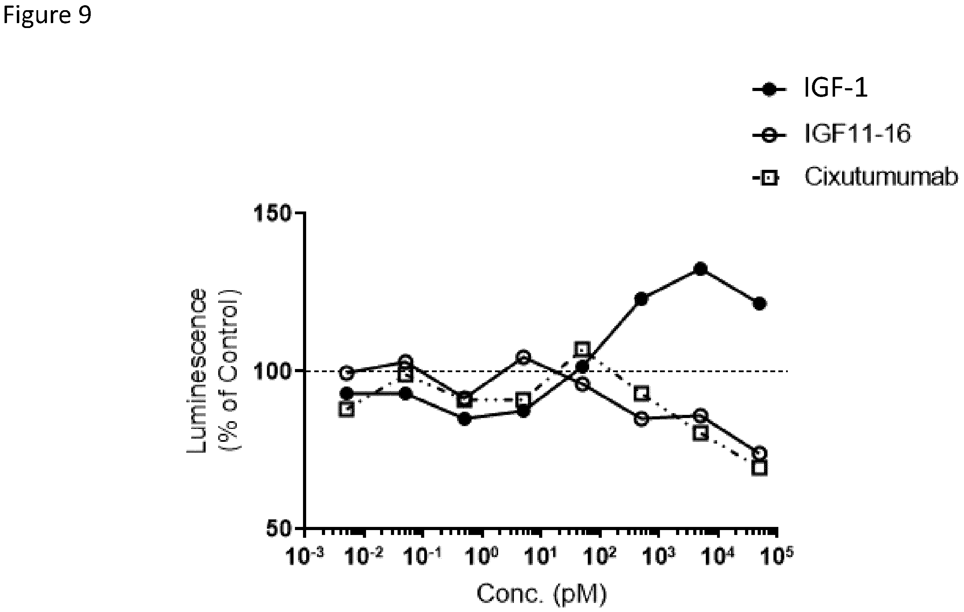

HepG2 cell line was suspended in DMEM (Gibco, 11995) with 1% FBS and seeded in a collagen-I coated 96-well plate (Corning, 356650) at 0.25 × 10⁴ cells/well. The next day, each of BSA/PBS, IGF-1 (mecasermine), control mouse IgG1 antibody (mIgG1), IGF11-16 antibody, and cixutumumab (IGF-1 receptor antagonist antibody) diluted from 50 nM at a constant ratio of 1/10 was added to the plate. After 2 days, the amount of ATP in the cells was determined as an indicator of cell proliferation by measuring the luminescence signal with a multi-detection mode microplate reader (SPARK, TECAN) by CellTiter-Glo^{®} Luminescent Cell Viability Assay (Promega, G7571). The measurement obtained for the control mouse IgG1 antibody (mIgG1) at each concentration point was set at 100%, and each measurement obtained for the other samples at each concentration point was calculated in the unit of % of Control, and plotted on a graph (Fig. 9).

The results indicated that the mouse parent antibody IGF11-16 has an inhibitory effect on HepG2 cell proliferation, suggesting that IGF11-16 has an antagonist effect on at least some types of cancer cells.

### [Example 21] Effect of IGF11-16 on the proliferative activity of human breast cancer cell line (MCF7) induced by IGF-1:

In order to evaluate the effect of IGF 11-16 on the proliferative activity of human breast cancer cell line (MCF7) induced by IGF-1, the concentration-dependent proliferative activity of hIGF-1 (Mecacermin) in the presence of 50 nM IGF11-16 was measured based on the amount of ATP in the cells 2 days after the addition.

Human breast cancer cell line (MCF7) was cultured in DMEM/F12 medium containing 10% FBS. The next day, the cells were seeded at 0.1 mL/well (2.5×10³ cells/well) in 96-well plates (Collagen-type I coated) using DMEM/F12 medium containing 10% FBS, and incubated at 37°C with 5% CO₂. The day after cell seeding, the medium was changed to DMEM/F12 medium containing 1% BSA, and the culture was incubated at 37°C and 5% CO₂ for about 8 hours. Subsequently, 50 nM of 0.1% BSA/PBS or the IGF11-16 antibody was added, and a series of IGF-1 diluted sequentially from 50 nM at a common ratio of 1/10 was added, and the culture was incubated for 2 days at 37°C with 5% CO₂. The amount of intracellular ATP was measured as an indicator of cell proliferation by using the CellTiter-Glo (registered trademark) Luminescent Cell Viability Assay (Promega, G7571), and detecting the luminescence signal with a multi-detection mode microplate reader (SPARK, TECAN). For each concentration of IGF-1, the mean value of the group with 0.1% BSA/PBS was set as 100%, and the change in the group with 50 nM IGF11-16 was expressed by calculating the % of Control. The results are shown in Table 17.

### [Table 17]

**Table 17**

| | IGF-1 alone | with 50nM IGF11-16 |
|---|---|---|
| 0.5nM IGF-1 | 100% | 67% |
| 5nM IGF-1 | 100% | 67% |
| 50nM IGF-1 | 100% | 74% |

The results indicated that the mouse parental antibody IGF11-16 had an inhibitory effect on reducing the maximum activity of IGF-1 on human breast cancer cell line (MCF7). These results suggest that IGF 11-16 has an allosteric antagonist effect.

### INDUSTRIAL APPLICABILITY

The present invention can provide anti-IGF-1 receptor humanized antibodies that specifically bind to vertebrate IGF-1 receptors and increase the muscle mass via the IGF-1 receptors without decreasing the blood glucose levels, and thus can be used in the treatment, prevention, or diagnosis of IGF-1 receptor-related disorders. The present invention can also be used in the treatment, prevention, or diagnosis of diseases related to abnormal cell proliferation or activation by suppressing excessive signaling of IGF-1 receptors. Therefore, the present invention has extremely high industrial value.

## Claims

1. An anti-IGF-1 receptor humanized antibody or its fragment or a derivative thereof comprising:
heavy-chain and light-chain complementarity determining regions (CDRs) each derived from mouse parent antibody IGF11-16; and
heavy-chain and light-chain framework regions (FRs) each derived from a human antibody,
wherein at least one of the CDRs contains a substitution of at least one amino acid residue relative to the corresponding CDR of the mouse parent antibody IGF11-16.

2. The anti-IGF-1 receptor humanized antibody or its fragment or a derivative thereof according to claim 1, wherein the amino acid residue at the 25th position in Framework Region 1 of the heavy chain variable region (FR-H1) is a proline residue.

3. The anti-IGF-1 receptor humanized antibody or its fragment or a derivative thereof according to claim 1 or 2, comprising:
as a sequence of CDR-1 of the heavy chain variable region (CDR-H1), amino acid sequence defined in SEQ ID NO: 1, or an amino acid sequence derived from SEQ ID NO: 1 via substitution of any one amino acid residue thereof,
as a sequence of CDR-2 of the heavy chain variable region (CDR-H2), SEQ ID NO:3or amino acid sequence defined in SEQ ID NO:5, or amino acid sequence derived from SEQ ID NO:3 or SEQ ID NO:5 via substitution of any one, two, or three amino acid residues thereof,
as a sequence of CDR-3 of the heavy chain variable region (CDR-H3), amino acid sequence defined in SEQ ID NO:7, or an amino acid sequence derived from SEQ ID NO:7 via substitution of any one or two amino acid residues thereof,
as a sequence of CDR-1 of the light chain variable region (CDR-L1), amino acid sequence defined in SEQ ID NO:9, or an amino acid sequence derived from SEQ ID NO:9 via substitution of any one or two amino acid residues thereof,
as a sequence of CDR-2 of the light chain variable region (CDR-L2), amino acid sequence defined in SEQ ID NO: 11, or an amino acid sequence derived from SEQ ID NO: 11 via substitution of any one amino acid residue thereof,
as a sequence of CDR-3 of the light chain variable region (CDR-L3), amino acid sequence defined in SEQ ID NO: 13, or an amino acid sequence derived from SEQ ID NO: 13 via substitution of any one or two amino acid residues thereof.

4. The anti-IGF-1 receptor humanized antibody or its fragment or a derivative thereof according to claim 1 or 2, comprising:
as a sequence of CDR-1 of the heavy chain variable region (CDR-H1), an amino acid sequence having a homology of 80% or more to SEQ ID NO: 1,
as a sequence of CDR-2 of the heavy chain variable region (CDR-H2), an amino acid sequence having a homology of 82% or more to SEQ ID NO:3 or SEQ ID NO:5,
as a sequence of CDR-3 of the heavy chain variable region (CDR-H3), an amino acid sequence having a homology of 75% or more to SEQ ID NO:7,
as a sequence of CDR-1 of the light chain variable region (CDR-L1), an amino acid sequence having a homology of 81% or more to SEQ ID NO:9,
as a sequence of CDR-2 of the light chain variable region (CDR-L2), an amino acid sequence having a homology of 85% or more to SEQ ID NO: 11, and
as a sequence of CDR-3 of the light chain variable region (CDR-L3), an amino acid sequence having a homology of 77% or more to SEQ ID NO: 13.

5. The anti-IGF-1 receptor humanized antibody or its fragment or a derivative thereof according to claim 1 or 2, comprising:
as a sequence of CDR-1 of the heavy chain variable region (CDR-H1), an amino acid sequence derived from SEQ ID NO:1 in which Trp at the 3rd position of SEQ ID NO:1 is retained or substituted with a similar amino acid residue, the amino acid sequence further including substitution of any one amino acid residue other than the amino acid residue at the 3rd position or having a homology of 80% or more to SEQ ID NO: 1,
as a sequence of CDR-2 of the heavy chain variable region (CDR-H2),
an amino acid sequence derived from SEQ ID NO:3 in which Glu at the 1st position and Asn at the 3rd position of SEQ ID NO:3 are each retained or substituted with a similar amino acid residue and Asn at the 6th position is retained or substituted with Ser or Gln, the amino acid sequence further including substitution of any one, two, or three amino acid residues other than the amino acid residues at the 1st position, the 3rd position, and the 6th position or having a homology of 82% or more to SEQ ID NO:3, or
an amino acid sequence derived from SEQ ID NO:5 in which Glu at the 1st position and Asn at the 3rd position of SEQ ID NO:5 are each retained or substituted with a similar amino acid residue and Ser at the 6th position of SEQ ID NO:5 is retained or substituted with Asn or Gln, the amino acid sequence further including substitution of any one, two, or three amino acid residues other than the amino acid residues at the 1st position, the 3rd position, and the 6th position or having a homology of 82% or more to SEQ ID NO:5,
as a sequence of CDR-3 of the heavy chain variable region (CDR-H3), an amino acid sequence derived from SEQ ID NO:7 in which Arg at the 4th position of SEQ ID NO:7 is retained or substituted with a similar amino acid residue, the amino acid sequence further including substitution of any one or two amino acid residues other than the amino acid residue at the 4th position of SEQ ID NO:7 or having a homology of 75% or more to SEQ ID NO:7,
as a sequence of CDR-1 of the light chain variable region (CDR-L1), an amino acid sequence derived from SEQ ID NO:9 in which Trp at the 9th position of SEQ ID NO:9 is retained or substituted with a similar amino acid residue, the amino acid sequence further including substitution of any one or two amino acid residues other than the amino acid residue at the 9th position of SEQ ID NO:9 or having a homology of 81% or more to SEQ ID NO:9,
as a sequence of CDR-2 of the light chain variable region (CDR-L2), an amino acid sequence derived from SEQ ID NO: 11 substitution of any one amino acid residue or having a homology of 85% or more to SEQ ID NO: 11,
as a sequence of CDR-3 of the light chain variable region (CDR-L3), an amino acid sequence derived from SEQ ID NO: 13 substitution of any one or two amino acid residues or having a homology of 77% or more to SEQ ID NO: 13.

6. The anti-IGF-1 receptor humanized antibody or its fragment or a derivative thereof according to any one of claims 1 to 5, which binds specifically to an extracellular domain of human IGF-1 receptor having the amino acid sequence defined in SEQ ID NO:71.

7. The anti-IGF-1 receptor humanized antibody or its fragment or a derivative thereof according to any one of claims 1 to 6, comprising:
as a heavy chain variable region, an amino acid sequence defined in SEQ ID NO:43, 47, 49, 53, 55, or 59, an amino acid sequence derived from SEQ ID NO:43, 47, 49, 53, 55, or 59 via substitution, deletion, or addition of one or several amino acid residues, or an amino acid sequence having a homology of 90% or more to SEQ ID NO:43, 47, 49, 53, 55, or 59, and
as a light chain variable region, an amino acid sequence defined in SEQ ID NO:65, 67, or 69, an amino acid sequence derived from SEQ ID NO:65, 67, or 69 via substitution, deletion, or addition of one or several amino acid residues, or an amino acid sequence having a homology of 90% or more to SEQ ID NO:65, 67, or 69.

8. The anti-IGF-1 receptor humanized antibody or its fragment or a derivative thereof according to any one of claims 1 to 7, comprising as a constant region of heavy and/or light chains, a constant region of heavy and/or light chains of any class of human immunoglobulin.

9. The anti-IGF-1 receptor humanized antibody or its fragment or a derivative thereof according to any one of claims 1 to 8, wherein the heavy chain constant region is the heavy chain constant region of human IgG4 or a region derived therefrom via substitution of 1 to 10 amino acids.

10. The anti-IGF-1 receptor humanized antibody or its fragment or a derivative thereof according to any one of claims 1 to 8, wherein the heavy chain constant region is the heavy chain constant region of human IgG1 or a region derived therefrom via substitution of 1 to 10 amino acids.

11. The anti-IGF-1 receptor humanized antibody or its fragment or a derivative thereof according to any one of claims 1 to 10, which binds to an IGF-1 receptor with an affinity represented by an equilibrium dissociation constant (KD) of 1 × 10⁻⁷ M or less.

12. The anti-IGF-1 receptor humanized antibody or its fragment or a derivative thereof according to any one of claims 1 to 11, which has an ability to activate IGF-1 receptor signaling.

13. The anti-IGF-1 receptor humanized antibody or its fragment or a derivative thereof according to any one of claims 1 to 12, which exhibits a proliferative activity in a myoblast proliferation assay.

14. The anti-IGF-1 receptor humanized antibody or its fragment or a derivative thereof according to any one of claims 1 to 13, which exhibits a binding affinity equivalent to or higher than that of the mouse parent antibody IGF11-16 in a BIACORE binding assay to recombinant soluble IGF-1 receptor.

15. The anti-IGF-1 receptor humanized antibody or its fragment or a derivative thereof according to any one of claims 1 to 14, which has an ability to induce muscle mass gain effect without inducing hypoglycemic symptoms in a normal mammal.

16. The anti-IGF-1 receptor humanized antibody or its fragment or a derivative thereof according to any one of claims 1 to 15, which has an ability to induce growth plate cartilage elongation effect without inducing hypoglycemic symptoms in a hypophysectomized model animal.

17. The anti-IGF-1 receptor humanized antibody or its fragment or a derivative thereof according to any one of claims 1 to 16, which, when administered to a vertebrate animal at a dose which induces an increase in muscle mass and/or body length, does not reduce the blood glucose level of the vertebrate animal.

18. The anti-IGF-1 receptor humanized antibody or its fragment or a derivative thereof according to any one of claims 1 to 16, which, even at a blood exposure level 10 times higher than an effective dose sufficient to induce an increase in muscle mass and/or body length, does not reduce the blood glucose level of a vertebrate animal.

19. The anti-IGF-1 receptor humanized antibody or its fragment or a derivative thereof according to any one of claims 1 to 18, which has an ability to inhibit the activation of IGF-1 receptor signaling.

20. The anti-IGF-1 receptor humanized antibody or its fragment or a derivative thereof according to any one of claims 1 to 18, which inhibits the proliferative activity of at least one ligand of IGF-1, IGF-2 and insulin, which ligand can activate the IGF-1 receptor in a myoblast proliferation assay.

21. The anti-IGF-1 receptor humanized antibody or its fragment or a derivative thereof according to any one of claims 1 to 20, which has an activity to inhibit cell proliferation in a cancer cell proliferation assay.

22. The anti-IGF-1 receptor humanized antibody or its fragment or a derivative thereof according to any one of claims 1 to 21, which has at least one characteristic selected from:
(1) inhibiting the proliferation of vertebrate-derived cells induced by an IGF-1 receptor activating ligand;
(2) inhibiting the proliferation of cells in a vertebrate animal induced by an IGF-1 receptor activating ligand in a cell proliferative disorder;
(3) not affecting glucose uptake by differentiated muscle cells at a dose sufficient to inhibit the proliferation of vertebrate-derived cells induced by an IGF-1 receptor activating ligand; and
(4) not affecting the blood glucose level in a vertebrate animal even at a dose sufficient to inhibit cell proliferation in a vertebrate cell proliferative disorder caused by IGF-1 receptor activating ligand.

23. The anti-IGF-1 receptor humanized antibody or its fragment or a derivative thereof according to any one of claims 1 to 22, which has an ability to induce an inhibitory effect on cancer cell proliferation without affecting the blood glucose level in a cancer-bearing model animal.

24. The anti-IGF-1 receptor humanized antibody or its fragment or a derivative thereof according to claim 23, which, even at a blood exposure level 10 times higher than an effective dose sufficient to induce an inhibitory effect on cancer cell proliferation in a cancer-bearing model animal, does not affect the blood glucose level of the model animal.

25. A nucleic acid molecule comprising a polynucleotide sequence encoding an anti-IGF-1 receptor humanized antibody or its fragment or a derivative thereof according to any one of claims 1 to 24.

26. A cloning vector or expression vector comprising at least one nucleic acid molecule according to claim 25.

27. A recombinant cell derived from a host cell via introduction of a vector according to claim 26.

28. A process of producing an anti-IGF-1 receptor humanized antibody or its fragment or a derivative thereof according to any one of claims 1 to 24, comprising:
culturing a recombinant cell according to claim 27; and
purifying the anti-IGF-1 receptor humanized antibody or its fragment or a derivative thereof produced by the recombinant cell.

29. A pharmaceutical composition comprising, as an active ingredient, an anti-IGF-1 receptor humanized antibody or its fragment or a derivative thereof according to any one of claims 1 to 24, a nucleic acid molecule according to claim 25, a vector according to claim 26, or a recombinant cell according to claim 27.

30. The pharmaceutical composition according to claim 29, for use in the treatment of muscle atrophic disease or dwarfism.

31. The pharmaceutical composition according to claim 30, wherein the muscle atrophic disease is disuse muscle atrophy, sarcopenia, or cachexia.

32. The pharmaceutical composition according to claim 30, wherein the dwarfism is Laron-type dwarfism or growth-hormone resistant dwarfism.

33. The pharmaceutical composition according to claim 29, for use in the treatment of an IGF-1 receptor associated disease.

34. The pharmaceutical composition according to claim 33, wherein the IGF-1 receptor associated disease is selected from the group consisting of: liver cancer, neuroblastoma, rhabdomyosarcoma, bone cancer, pediatric cancer, acromegalia, ovary cancer, pancreas cancer, benignant prostatic hypertrophy, breast cancer, prostate cancer, bone cancer, lung cancer, colorectal cancer, neck cancer, synoviosarcoma, urinary bladder cancer, stomach cancer, Wilms' tumor, diarrhea associated with metastatic carcinoid and vasoactive intestinal peptide secreting tumor, vipoma, Verner-Morrison syndrome, Beckwith-Wiedemann syndrome, kidney cancer, renal-cell cancer, transitional cell cancer, Ewing's sarcoma, leukemia, acute lymphoblastic leukemia, brain tumor, glioblastoma, non-glioblastomatous brain tumor, meningioma, pituitary adenoma, vestibular schwannoma, undifferentiated neuroectodermal tumor, medulloblastoma, astrocytoma, oligodendroglioma, brain room top swell, choroid plexus papilloma, gigantism, psoriasis, atherosclerosis, vascular smooth muscle restenosis, inappropriate microvascular growth, diabetic retinopathy, Graves' disease, multiple sclerosis, systemic erythematodes, myasthenia gravis, autoimmune thyroiditis, Hashimoto's thyroiditis, thyroid ophthalmopathy, hyperthyroidism and Behcet's disease.
